# EUROPEAN PATENT APPLICATION

(11) **EP 4 050 341 A1**
(43) Date of publication of application: **31.08.2022**
(21) Application number: 21159669.7
(22) Date of filing: 26.02.2021
(51) Int. Cl.: G01N 33/68, C12Q 1/6883

(54) **PROTEIN AND METABOLITE BLOOD BIOMARKERS FOR THE DIAGNOSIS OF BRUGADA SYNDROME**

(71) Applicant: Cardiomix S.r.l., 20121 Milano (IT)
(72) Inventor: PAPPONE, Carlo, 20121 Milano (IT); ANASTASIA, Luigi, 20121 Milano (IT); CICONTE, Giuseppe, 20121 Milano (IT); ESPINOSA ANGARICA, Vladimir, 20121 Milano (IT); VICEDOMINI, Gabriele, 20121 Milano (IT); PETRETTO, Enrico, 20121 Milano (IT)
(74) Representative: Calogero, Ida

(57) **Abstract**

The present invention relates to a specific set of circulating protein and metabolite biomarkers for the diagnosis of Brugada Syndrome (BrS) in a human being and relative methods of detection. The invention further relates to mutated *Prg4, Epx* and *Pon1* genes and related proteins for the prediction and diagnosis of Brugada Syndrome.

SEQUENCE LISTING

<110>  CARDIOMIX

<120>  PROTEIN AND METABOLITE BLOOD BIOMARKERS FOR THE DIAGNOSIS OF
       BRUGADA SYNDROME

<130>  P1933EP

<160>  9

<170>  PatentIn version 3.5

<210>  1
<211>  19489
<212>  DNA
<213>  Artificial sequence

<220>
<223>  Prg4 human gene

<400>  1
taaaggaagg atctaaggaa aatatcaata cattttttaa gcagattttt aaaataataa      60

aggaagctag aaaatccagt gtcaataatc tacctgtagg taactgagag catgttttct     120

gtgctcagag aaaatgaaac tactcaagaa atgtatattt gatataatgg tataagtata     180

taaaatgtat gccacaagct aacactaggt aaatttcaaa gctacctttg ctagatttaa     240

ctggaaagca gaagaaataa atgataccag tatgtgaata ttttaaagtt gctgcttcaa     300

tatactagtc aaagtctgcc agagctattt accatctcag gacaatttgt tcacttcact     360

tagaaattag aaatgtgctc tggccaatac atctaaattt attttgtggt agtcattttg     420

cttaatggaa gtgttttcca gatgttgcct aagtctagtc gtctgggccc ttttccaata     480

aatgaggaaa gtttgatttc ataggttgtc actgttgatt ttgtctaacc tttggactaa     540

ttggttcatc ccactgtatt tgcactgata tataagactc ccaggacgtg ggataaactc     600

atctatcctt tacgggtaag tgtcaaagtt aacttgcttc atagaattaa atgtgttttc     660

attagaggtg tttgaaaaaa aatgtgtaaa gatagtagtt ggaaattttt gaaaaggatt     720

atgtttatgc aatacacctg ttggaagcct ttgaatttat attgagaatt aagaaaaagt     780

tggaacagta actccatgat gcttattaaa ttacatttct gtgacacagg ttattatttt     840

ccttaagata aaattttaac cttgcactgt taagtacatg ccatactttg gctagagttt     900

taagataagt ctattctata ttggagattt cttaagaatt aaaactgtca ataacgtggc     960

tgagggtgac ttgatttgtt ctgcataaga ttaagtctaa tggccaaata ttttctataa    1020

aattaaagct gatttaaagt cttgtttcaa gaatgggatg gttttattat tgttacattt    1080

ttaaagttac tgaaatgtgt ataatgcaag cctaagttag tggtgagatg aaagagctgt    1140

tttctgatac ttttatttta ttttcagcaa gggtacctac ggtacctgaa aacaacgatg    1200

gcatggaaaa cacttcccat ttacctgttg ttgctgctgt ctgttttcgt gattcagcaa    1260

gtttcatctc aaggtagctt aaccatcgaa catactttta tttaacaact attgctaatc 1320

attcagtctt gatttttata acaacggaaa tatatttcta aaaatttata tttgcttgag 1380

tttaatatac tgtacaacct taaaataata tataacatgt agcctgtttg taagtgcttt 1440

ggaattctca tctttttctc cttttgagag ttttgagctt gtataaagaa taattttggt 1500

acttgttatt tctatggttc tctcgaaagt ttaaaaagtg cagacagttc atcataaaat 1560

tatgctggag ctatatagta tcataaaaat aactaaaaaa gaacaatatt tagataacat 1620

gtaatttttg cctatatgtt gaacaatttg ttttttaaaa tcaaataaat tattgataaa 1680

taacttattt tggcatcatt tagacataga taccatatta taataaacta tagtgtttca 1740

aatggcattt tattagtctt gaaaatgttc tctttttctt acaagaacta tttatccagg 1800

tatggacata tacagatgtg actgtcattt tgtgttataa cattaaactt ttcacagttc 1860

tcccaaaaca ggtcctggat gaatagttct tgttactcat ttttagagac tgttaaagta 1920

cattcagtga aatcacatta gtaaaagatg ctagcatgcc atctaggggg attaatagta 1980

acaatgccaa gctttggatt tttcttttct tttcttttcc ttttgttccc ctttgataag 2040

caaaatctga gagaataaaa atcaagattc atgacagtta tgatgaaatt atgtttctaa 2100

agtaaacatt tccagtaaaa tacgagattc ttatgaaacc tgaacatgag tggtaactgt 2160

ctgcataggc ataagttgca gaattgttta gataagaaaa gacaggaaaa cacatgaaag 2220

caaatgtgaa tattcaataa gaatgatgac tactccagta tctccagacc cttcggcttt 2280

ctcgtaacac tatgacaagg ttcacaacac tgggggcact ttctaaactg ccttttcctc 2340

tgtgatacaa ttggttgttc actaaaacag tgttactttc attttaattg tgattaaata 2400

aatcaaatta aaattaatgg ggctgggtgt ggtggctcac acctgtaatc ccagcattct 2460

gggaggccta gggagacaga tcacctgagg tctggagttc aagagcagcc tgggcaacat 2520

ggcaaaatct tgtctctata caaaatacaa aaaaattagc caggtgtggt agtgtgtgcc 2580

tgtaatccca gctattcgga ggctgaggca agaggatatc ttgaatccag gaggtggagg 2640

ttgcattaag ctgagcttgc atcatggcac tccagcctgg gcaacagagt gagactctgt 2700

ctcaaaaata aataaataaa taaataaaat taatggttac aattaatagc aataaaagta 2760

ggacagacac ctaatctatg aaagtaagct tttcctgtaa ggatgatttc ctcttttttt 2820

tttttctttt gagacagggt cttgctttgt ctcccagtct ggagtgcagt ggcacaatct 2880

tggctcactg caacctccac ctacggggtt caagcgattc tcctgtctca gcctcccgag 2940

tagctgggat tacaggcccc tgccaccatg cccggttaat ttttgtattt ttagtaaaga 3000

caggttttca ccatgttggc caggctggtc tcacctgacc tcaggtgatc cgcccgcctc 3060

agcctcccaa agtgctggaa ttacagatga tttcttattt cagaaatctg ccaactataa 3120

aagagcaatc tcttgatact gtcttgtctg cttctcttgc tttctcaacc tcttctcatt 3180

ctcttttttc tttatatata atatatgtat ttatatacat acactatata tacatttttg 3240

tatgcattat gcactcatgt acgcaaaaag ttctgaaagt tgtcctacaa tttactgttt 3300

tatttgcata ttcagacttt ggcattcctg gactctattc ttttaagatt tgttttcagt 3360

gtgtttcaac attcctttgt ggatttagga cagtacacct gccaatttct attccaggga 3420

tggattccat tgtcacattt ctgcagtcat ttctcaggga gggttttaag gtggtgtttt 3480

ccaaatgact tttaaaaaat atttgagaat gagtatgctt ttgttattgt ttgcttattt 3540

gttgcctgga aaacctctgt tcatgacctt tatagttatg cccagttatt ttaagggttc 3600

tcacttcagc agtggttgta agcatctgcc tcccctgaca ttttaaaaat ccagttatcc 3660

ccaccaatta aggaagaaaa agctcagtaa agtttatgct ggtgcaacca gcgtgcttta 3720

aacttatcct caggaatggg aggcaaagcg acaggtggaa acatctcagg cttaaaataa 3780

gatatatcag agttcaaatt ctggatggat tgtttactta aggtgaccat aaaatgtatt 3840

atctaaatta ggacaattat aagggtaaaa gagttcacta caaataattg ggaggtatta 3900

attaacctat gatgccaata tactggaact attaactagt cgtgtgataa tgagcaagtt 3960

gtcaaaattt ctgtgtttca gctttcttag ctctaaaaaa gggctactaa atcctggaaa 4020

cattttcata agcattagtg ataatgtatg actcagcacc tggcatagag aagctcaata 4080

aatggcaact gctaatcatc aaaaccacag atcggtagta gctgtagctg ccacctcttc 4140

cttaggagta tcaccctctt taggcaaagc cattactttg cctggttttc aaaaatgtgt 4200

ttatcatctc agtctagtga agaataaagt gacataattg agatcaccta agacatcagc 4260

caaatatcag ctgggcctat tgctgacatc attccaacac cttctcgatc aataaaattc 4320

tctctcacca agtggctttg tccccctcgt tagattgctc cctttctata aagtggtttg 4380

gccatattta cgccagtatt gtataatttt agatttatca agctgtgcag ggagatgtgg 4440

ggaagggtat tctagagatg ccacctgcaa ctgtgattat aactgtcaac actacatgga 4500

gtgctgccct gatttcaaga gagtctgcac tgcgggtaag tcctgagagc gggtgtctcc 4560

tctgtcaagc aacactgcga gtctgtgagt ccccccttgc accctcgtgc agtgctgtga 4620

gactgagcct ccccttgcac ccacttgcag tgctgttttc ccactgtatc acttcctttg 4680

cttaagtgac tttccttcac ttgcataaat gttgcagcgc attgtaccta aggatggtgt 4740

caaagcttgt gattattggg gagggtaagg ggagcctgga agtctgtcac ttgcacaagg 4800

ttattgtcta gttccaagta aatgtcttat tttaaataga gaagatactg tgagtcagga 4860

gtcattaagt aaatagtaag gagtaattat ttacaatcag agatgtcctc gactagtaat 4920

gaggactttc aatagtttca tcaaaagaat acatgagcat ggaataatct tgtctaccat 4980

tggactttct gatgttaaca cgtttgtaaa ttacctggaa aagacacttt aaagataaag 5040

tgctagtgtt taatgatgaa tttcagttaa aatctgaagg tctgaatttt aaattatgag 5100

tggtgtttca cttgaatata ttttgggcca ttttattatt aacacagaaa catttcctat 5160

tcaatttaag aaaattgtaa ttaaagttat ttgaaaggtg tatttgcttt agaatttgaa 5220

tgtgaagttg aggaatatat tgtgcaattt atgtattgga taggtaatta tttcaaataa 5280

gccttggtaa gtccctgtta actctaacaa aggcttttta aattttcatt ttttaaatga 5340

gtagtcctcc ctaacatagt ccacactgta agattaggct gaaagctttc aactatacac 5400

cttccccagt ttgtcataat aagggcctct gcattacaaa tgatttttaa gcctcaaaaa 5460

tgacccattt acgtggaata tatatacata tatatttata tggaatatat tgctgagatc 5520

tgtacattca attactgtga atctattaca aagcagtgtg tgaagaggag aaggatgaag 5580

agatttcata tgaaggctat ctcactatct agacatttcc cgatttttct ttgtccatac 5640

atgtaaataa ctcgggcagc atcaggatgt ctcttggagt ctggaagggc aagaggagtt 5700

gccctcagtc accatatttc ttttttgact tgggctgtct ccatctggga taccatctaa 5760

tttttcctgg atgatgtact ccaaatttca aataaaagac ttagaaatga acttttggaa 5820

acctagtcaa gtctaaggtg ggaaatggct gtcaaatacg tgggcctggc ttcacaatga 5880

ataatctgta acttcttgtt ttgctctggg tagagctttc ctgtaaaggc cgctgctttg 5940

agtccttcga gagagggagg gagtgtgact gcgacgccca atgtaagaag tatgacaagt 6000

gctgtcccga ttatgagagt ttctgtgcag aaggtaagca tcacagtacc aaccaatgct 6060

tctcagtaca gccagatctg tgcacctacc ttagcatcac tgataatgtc taacacgcag 6120

tccatcttag gccttgattt tactaacata taagctattt cataataatt ttgttttgtg 6180

gagatgaatt aaagaagaaa agcgccttcc aacagcagaa cccccaggct cagccaggct 6240

ccaagagtac aagatgttgc cagaggggga acatagagga tgaggctgaa tgtatatgtc 6300

ttttttttct tcagatatgg ttcaatttca ggttaaatca acagtaatca aatcacttga 6360

tgatttcttg tctcaccctt gctgacttca cgaagtcaac tatcctttga gctatctgtg 6420

cttttggcct cagtgtttga gtatatattt aaggacataa gcaacacttt cagcttccct 6480

aaaccagtaa atttatgaat tagtttaagc cattaaattt ataggcaaat ctacctaaca 6540

cgttgttaac tgtcatgttc cttcccattt ctgcagtctc ctcagcagtc tctattccaa 6600

tacatgtgca tggtaaagtc aataacaagg gatattccca tcttaaaatc aaattctcta 6660

tttataggtc ttgaaggact cctctagagt ataacaaaag gctccagatt ttacagaaaa 6720

agattttaaa aatatacttg aatactggat tactttcctt taccaaatgg ttgttcttaa 6780

attctacttt gttaaaacaa gaatatttgt ggaggtaaaa gtgaaaataa gctttgcttg 6840

cataatcaat cttatcattt tatttaaaca agcctgcgct tcctgcttct ttagaacagt 6900

gaagaaagat cggaaaactg ctaatcagca gaattgagag atgtttctta accgttcaat 6960

gttttcatgg gaaaacattc caaaaatgat gaagacaaca cccttgtaat aaaaacctta 7020

gagctcaccc ttcctgcaga ttttagcagg ccccatgatg agtgacacat atttactcat 7080

taccctaatt gttttggctg tgagacaaga aagctaagaa tttgttttgg gcagaggatg 7140

tctagggagt gaagatggtt agaaagtgat gagacattga gagaacattt tgataaagat 7200

aatactttat gttatgaaat atttggtcag attacctaaa gtttaggtag aactctgaaa 7260

aaaaaatgat gaaggcgatg ctggccaact ttctttacag aattcataag acagtcagcg 7320

tcccaactta aaataataaa accactcaga acctcaatac ttcatttttt aagtgtaata 7380

accaaactat cttgagattc actacttagt tgtttcagta tagatactac ttacaggtgt 7440

ctatatatct aaaaatcata acatttcaag aattgaagga atcccagcca tccaagtgcc 7500

tctgaatgtg aaagagaaaa cagagtccaa gaggttaacc attttggcca agaacattag 7560

ctagtacatg gcagatctgt ttctcaaatt gagatacttg atgtttttgc tgggtaggag 7620

gagaaacaag agggacagtg gccagttcac ggagactata gcagtagggt tctggggcga 7680

tgttgcgggg agcagcatga tttatcacaa cccttctgta cccagagagt ccccagtgct 7740

gttttcttat ccttcattag tccctcttct tgttcctaaa aaaaaaaaaa aaaaagaagt 7800

tccatgtttt ctcttctaga ctgagaagcg gaaaagggaa ggggactaaa aggttaaaaa 7860

gatacacacc aaaccattaa gttgtatctt tgggaagggc agggcatttt gaggaaaagc 7920

acaatgtgaa ggtgtgaact tttcacaaga atattcatgt agaacttttg taattaaaaa 7980

aagaacaaaa ggcaaattgg catcatttaa tttaaatgaa tattaaaaca gaaaaaaatg 8040

aatactaaaa cagaaaaaaa ttctgaaggc aaagaccttc agaaatcatc ttgaataaac 8100

atcttagttt gtagacaaga agatgaagtt caagataatt atttgcctaa ggtcacaatg 8160

gtagaggctc gcaggcctag gactggaatt taggtcttct gattcctgag actttgctcc 8220

ctgtcataac ttacaacaat ttgagaacca gtattttata gcaatgcatc cttagactta 8280

aatggagtca ttcgtgttga gctggagcct gcctgcactg gctgtcacca gcatctactc 8340

ttgaagctag atgcatctgt gcttttcaca gttagagctg ctgatgaaca taaacaagat 8400

gttaactgac ttgtcttact tggcctcagt gcataatccc acatcaccac catcttcaaa 8460

gaaagcacct ccaccttcag gagcatctca aaccatcaaa tcaacaacca aacgttcacc 8520

caaaccacca aacaagaaga agactaagaa agttatagaa tcagaggaaa taacagaagg 8580

taggaagatg acagatataa tcaaaggagc tttcttagat gaagtaactt gtaggtgact 8640

gcttatctaa gcccattctc agagaacagg gtaatcttag gaatcatgag cctcattaca 8700

ctcgaaggtt ttagactttg cttttaagta aagtttaaga caaagtataa actctcagct 8760

ctttctgtat ttaccaaacc cagcatgagt ctgggttaaa acaaatcaga agggacaaat 8820

cttactataa aaaacaaaaa ccaccccatg attttctttg tagaataatt tgattctgtg 8880

ttttggagga tatgggaaag ttgagagata ctagtaatac tgctagtatc tgtgataagc 8940

ccaggtgcct tgcttttaac tgacagatta aaaggcagtt ggtcatatta ctaataaaag 9000

caaaatccag atacttgtag actagtaaat agcacttctt gctgtgttta gactggtggt 9060

tcttttttgt tttaaatcac agttggtgtg atcaaacttt ctatttgatt tatagaacat 9120

tctgtttctg aaaatcaaga gtcctcctcc tcctcctcct cttcctcttc ttcttcaaca 9180

attcggaaaa tcaagtcttc caaaaattca gctgctaata gagaattaca gaagaaactc 9240

aaaggtttga gcattgataa agatcaaaga ctgtatcaat gccatattaa caatgattat 9300

atttaaaagt aatgcgtgtt ggcagaatga ggacatctta atatgggggg gaatataact 9360

ttatgaatat tatcttagaa agtaccaaat gaatacttcc tacaggaaat tagtgccaag 9420

cggctaagaa gccactctaa aagcaaagga taagaagatg ctgtagcagg actgtggaac 9480

cacaagatgg caccagagat gattattgat ggctttctac tgtcattctt gtttacttga 9540

catcattttg cttcttctcc ttcatcttcc ttgcattttc attttccctt ccccagcttt 9600

attttaacaa acatttatat actattgttc taagtgtttt acaagtatta actcattaaa 9660

ttcttacaaa cctctatgga aaaaatatta tccatatttt acaaatcctc tttattttcc 9720

tagtaactta tatattatat ctggctgact tttcctctag tagagactgt gctacagaat 9780

aggctattat tgcctgaaaa gagatttcct ttgctgttta ttatttcctg aagttctggt 9840

tctaaatata tattctcttt ctccaggaaa tactggattt ttggataagg gagttttaaa 9900

gaactctaag aggttgtgat ttcctccgaa gcattcgctc taaataaata gccaggcatt 9960

taatctttta gtcacccaga aaatcgtgca ggaagccacc cttgggcttt gtagtccaca 10020

tgtggggcag ctgctatggg aaagccttga taaatactgt ctttttgctg cccatcacac 10080

cctaagctcc tccttatgtc ccctgggacc tgcaaggtta gctgcgcaga agaaaaggag 10140

tggctaatag ccctatgtat gggaacaagg ctcagcaggg actgtggggg tgactggggc 10200

agattgctca gaggtagctg tagaggcctc tgagataatt ggtgcagcaa gatggtagta 10260

ctaggtttcc aggaggaagg aagggacact gggcactgaa gaataagagt acaaaaagta 10320

gggggtaaag aacattaata taaacagatg aattagtgtt atttatcgtg ttggtaattt 10380

tgaacttgcc actgacttcc atgtattaat tgtatagagt tttatttgtg ctttttcatc 10440

cctcatctca tttattccct aaaacagccc aataagatag gttgagtatt tgccatttta 10500

caacaaatta aaggacatag tagagattag caatttgctt agaaacacac aactagtcaa 10560

tgataaagat gggatcttta tgtcactatt aaagaaaaaa aaatattcta aaataacaag 10620

atgtatattt tttctccagt aaaagataac aagaagaaca gaactaaaaa gaaacctacc 10680

cccaaaccac cagttgtaga tgaagctgga agtggattgg acaatggtga cttcaaggtc 10740

acaactcctg acacgtctac cacccaacac aataaagtca gcacatctcc caagatcaca 10800

acagcaaaac caataaatcc cagacccagt cttccaccta attctgatac atctaaagag 10860

acgtctttga cagtgaataa agagacaaca gttgaaacta aagaaactac tacaacaaat 10920

aaacagactt caactgatgg aaaagagaag actacttccg ctaaagagac acaaagtata 10980

gagaaaacat ctgctaaaga tttagcaccc acatctaaag tgctggctaa acctacaccc 11040

aaagctgaaa ctacaaccaa aggccctgct ctcaccactc ccaaggagcc cacgcccacc 11100

actcccaagg agcctgcatc taccacaccc aaagagccca cacctaccac catcaagtct 11160

gcacccacca cccccaagga gcctgcaccc accaccacca agtctgcacc caccactccc 11220

aaggagcctg cacccaccac caccaaggag cctgcaccca ccactcccaa ggagcctgca 11280

cccaccacca ccaaggagcc tgcacccacc accaccaagt ctgcacccac cactcccaag 11340

gagcctgcac ccaccacccc caagaagcct gccccaacta cccccaagga gcctgcaccc 11400

accactccca aggagcctac acccaccact cccaaggagc ctgcacccac caccaaggag 11460

cctgcaccca ccactcccaa agagcctgca cccactgccc ccaagaagcc tgccccaact 11520

acccccaagg agcctgcacc caccactccc aaggagcctg cacccaccac caccaaggag 11580

ccttcaccca ccactcccaa ggagcctgca cccaccacca ccaagtctgc acccaccact 11640

accaaggagc ctgcacccac cactaccaag tctgcaccca ccactcccaa ggagccttca 11700

cccaccacca ccaaggagcc tgcacccacc actcccaagg agcctgcacc caccaccccc 11760

aagaagcctg ccccaactac ccccaaggag cctgcaccca ccactcccaa ggaacctgca 11820

cccaccacca ccaagaagcc tgcacccacc actcccaaag agcctgcccc aactaccccc 11880

aaggagactg cacccaccac ccccaagaag ctcacgccca ccacccccga gaagctcgca 11940

cccaccaccc ctgagaagcc cgcacccacc acccctgagg agctcgcacc caccacccct 12000

gaggagccca cacccaccac ccctgaggag cctgctccca ccactcccaa ggcagcggct 12060

cccaacaccc ctaaggagcc tgctccaact acccctaagg agcctgctcc aactacccct 12120

aaggagcctg ctccaactac ccctaaggag actgctccaa ctacccctaa agggactgct 12180

ccaactaccc tcaaggaacc tgcacccact actcccaaga agcctgcccc caaggagctt 12240

gcacccacca ccaccaagga gcccacatcc accacctctg acaagcccgc tccaactacc 12300

cctaagggga ctgctccaac tacccctaag gagcctgctc caactacccc taaggagcct 12360

gctccaacta cccctaaggg gactgctcca actaccctca aggaacctgc acccactact 12420

cccaagaagc ctgcccccaa ggagcttgca cccaccacca ccaaggggcc cacatccacc 12480

acctctgaca agcctgctcc aactacacct aaggagactg ctccaactac ccccaaggag 12540

cctgcaccca ctacccccaa gaagcctgct ccaactactc ctgagacacc tcctccaacc 12600

acttcagagg tctctactcc aactaccacc aaggagccta ccactatcca caaaagccct 12660
gatgaatcaa ctcctgagct ttctgcagaa cccacaccaa aagctcttga aaacagtccc 12720
aaggaacctg gtgtacctac aactaagact cctgcagcga ctaaacctga aatgactaca 12780
acagctaaag acaagacaac agaaagagac ttacgtacta cacctgaaac tacaactgct 12840
gcacctaaga tgacaaaaga gacagcaact acaacagaaa aaactaccga atccaaaata 12900
acagctacaa ccacacaagt aacatctacc acaactcaag ataccacacc attcaaaatt 12960
actactctta aaacaactac tcttgcaccc aaagtaacta caacaaaaaa gacaattact 13020
accactgaga ttatgaacaa acctgaagaa acagctaaac caaaagacag agctactaat 13080
tctaaagcga caactcctaa acctcaaaag ccaaccaaag cacccaaaaa acccacttct 13140
accaaaaagc caaaaacaat gcctagagtg agaaaaccaa agacgacacc aactccccgc 13200
aagatgacat caacaatgcc agaattgaac cctacctcaa gaatagcaga agccatgctc 13260
caaaccacca ccagacctaa ccaaactcca aactccaaac tagttgaagt aaatccaaag 13320
agtgaagatg caggtggtgc tgaaggagaa acacctcata tgcttctcag gccccatgtg 13380
ttcatgcctg aagttactcc cgacatggat tacttaccga gagtacccaa tcaaggcatt 13440
atcatcaatc ccatgctttc cggtattaag aatcagttat tttcattttt aaagctggta 13500
atgttagttt acatctatct gaaccagaat gccttagttt agtatcactc aactgagata 13560
tattacctga ccttgttagc ttgtgaagtt ttacagcttc cctgtaggta agcagaggtg 13620
taattacagt aagctttttc attctctaga aaattaagcc tgggaattta gcatatgtgt 13680
gtctgtgagc agcagagatg gtatatagaa agtcaaaaaa atttagttcg gagtctattc 13740
ttaaaagtac aatgcaaagc aaaaatctgt agtatcattt tccaaactaa cccaaaggga 13800
gggcaatcag aactgaaaga tgcaatttca cattgagttc atacatccaa aaaatgtcta 13860
gtttacatga aaatcaaggt aacctggtcc attccaattc ataataattt atttcatata 13920
atacttttga agagtatcat ctttaagtaa aatggaatta tcaagtatat aactatgcaa 13980
acaggtcaaa ctgtgtcctt caagatctta gaaaaggtaa acaggaaata gaaccatgtg 14040
gaaagacttg aaaagaacat acagactttt ctcattctgt tctatatttg ttttgttttt 14100
gttaatttgt ttagatgaga ccaatatatg caatggtaag ccagtagatg gactgactac 14160
tttgcgcaat gggacattag ttgcattccg aggtgagcta tgtacacatt tatttttctt 14220
ctcatcattc tgttttggca tttatgagaa ccaaagccgt ggaagagtgc tagtttgggt 14280
tgttttctgg aggggaatct gattgataag cacacctcac aggagaatcc gagagtaata 14340
acagaaatat tggattaaga aaattagaca aagtaacaaa acaaaaccaa gaccctgaac 14400
tgatactact gaggtttttt cctactgtta agatttaaaa agtagttcat tttttttggg 14460

gggggggggt agttaaaatt taaaaaataa aataaaattt taatttcata ccaaatgaaa 14520

tgacaccagc atgcatattc actgctttat ttgaaaataa catctcaatt gtgatgtgtt 14580

caagtgttac actctctgtc ggagagtaac agaagaaaag ttatttcatg ttaattctct 14640

gctgtactct gctagtccag tcttgaaaca tatttagatt aaatctgaaa gcaattcctt 14700

ttcgtacagt agaggctaaa gagatgttag tcattaaatt ataattcctt agttctaaga 14760

gataaagaaa ttgagatcta aagagatagt gactgtaagt cacaaagcca aaggcttttt 14820

ttgagaccgg atctccctct gtcacccagg ctggagtaga gtggtgcaat tacggctcaa 14880

tgcaggctcg atctcctggg cccaagtgat cctcccatct cagccacctg agtagctggg 14940

actacaggtg tgcaccacca tgcctggcta atttttgtat ttttttttgt attttttttt 15000

ttttgtagag acagggtttt gtcatggtgt ccacgctggt ctcaaactcc tgggcccaag 15060

ccatctaccc gccttaacct cccaaagtgc tggattacag gtgtgagcca ctgcacctgg 15120

cccaaccaat aggctctttc taaagaaact agaaaactaa agtatctcaa aggttatgct 15180

aaatttcgga cttgggaaat acctgctctc agaaatgttc aaatactacc ttttgtatct 15240

gactaaactt ccagtcatac aatgcataag aaaactacat tttttttcat tttggtttct 15300

tttgtgatag gtttagcatt cagcttgtag gctgatgtct ttccttaaat tttaggtcat 15360

tatttctgga tgctaagtcc attcagtcca ccatctccag ctcgcagaat tactgaagtt 15420

tggggtattc cttcccccat tgatactgtt tttactaggt gcaactgtga aggaaaaact 15480

ttcttcttta aggtaacaca aatatctttg tgagagaaat ttaataataa tttgtaactg 15540

cattatttat tacaacatat attgccttaa cctccaatat gtgtcctttt aaatactctg 15600

tcatcaaaat ttaatcataa ttcaacagtt tgataacagt aatggtcaaa attcaaccgt 15660

ttaaccaagt taacacgttc tctctatact taaatctaga aaggagttcc aaatcttttt 15720

tcccaaagct gataacataa ttttctcttt taaattatca ggattctcag tactggcgtt 15780

ttaccaatga tataaaagat gcaggtaccc ccaaaccaat tttcaaagga tttggaggac 15840

taactggaca aatagtggca gcgctttcaa cagctaaata taagaactgg cctgaatctg 15900

tgtatttttt caagagaggt atgtgttaca taattgacaa gattacaaaa cttttaaaga 15960

attacactca gcattttcat ttattattga ctttactgtc aaaagatatc tttaatggct 16020

gaaatcaaat attttcagtg aaaaaaatca atattgaggg tagtattctt attgccctca 16080

atttttattt tcatttcttc acaggcaagt cacaattcat ttgagttttc aggtcactga 16140

tagaatgtca tttactttcg cttcacaaat gtgcatgtca tccttgcaca agggccatgc 16200

taatctctgt atcattccag ttttagtata tgtgctgcca aactgagcac ttgtcacttt 16260

caattgaaat taaatatata actatgtaat ttgctgcatc tattcattca acaagtattt 16320

cagtttaata attattttta taataccctt gactaatagc cattattaat atcaatatat 16380

tatatgaaaa atgagaacaa aactgtttcc tatacattgt aaaagttgtt ttccaccttt 16440

tctgagggta ttaaaattaa ttttcatttt ccatgtgata ttctaataca taacaggtgg 16500

cagcattcag cagtatattt ataaacagga acctgtacag aagtgccctg gaagaaggcc 16560

tgctctaaat tatccagtgt atggagaaac gacacaggtt aggagacgtc gctttgaacg 16620

tgctatagga ccttctcaaa cacacaccat cagaattcaa tattcacctg ccagactggc 16680

ttatcaagac aaaggtaaca tttttattgt gttaaaaaaa tccttaaaca taagtagatg 16740

taatacagtt tcttatacag taaggcttat gaaatatgga tactgatcaa acagccctaa 16800

taatttaagc ttactgatga aaaactcatc cacttgcctt agtgaataac caaagaccaa 16860

tactttcttt ttccttgtgg gtaagtaaag cagtttgttc aggtttgtta gttataacca 16920

atactattta tcaagtactt cttaccaaga acattatata ccagtctata accctcaata 16980

gttctacatc agagggctaa aactgagatt aaaactcaga tgtctggctc tttccaagat 17040

agtacattgc cttttaatct ggtatctttt attaaacatg ccacttacag gtgtccttca 17100

taatgaagtt aaagtgagta tactgtggag aggacttcca aatgtggtta cctcagctat 17160

atcactgccc aacatcagaa aacctgacgg ctatgattac tatgcctttt ctaaaggtaa 17220

ggtattaact aacagtttcc caaggaggtg atatcatttg tgaaaacatg aagataaagt 17280

aaaaatgctg gctgcaatga tgactgaatg tgagaagtta cactacggta cttattctaa 17340

ttgctgtgga aaagagttaa gacttttgct ttaatttcaa ttaaaatgat ggcactgcaa 17400

ttcaattctg ctctaacctt catgagatta cgataaaaca tccagttact agagtaaact 17460

tgctactgac aatagtattt tacttctatc atttgtggca tttaacagat accttgtttg 17520

gcacaaggtt ataatgattt gcatcactgc agagtctgta agatcagaaa gaagttcctg 17580

agaaactaat tttattaatt aaattatatt actcaggatg cttttctttg aaatgcccag 17640

aaatctaagt tattctgcct gtaatttcat aatatgaatg acattggcat gtatttttta 17700

aaaggaataa ccccaagtaa attatttttc aaacttggtt actctttaat gtttacttca 17760

taaaggagag ctgaacctga ttttacaaaa agatggtgaa atgtcaatct tttgaaacat 17820

caaaaaagct gaaaagtcta ggcaattgtt tttctttttg ttataaagtt caaattaatt 17880

agtaaaaaca tctctattaa aatgataaac attgtctttg agcataatag tcaacataag 17940

ttatttttta gtttgggcat tgttttcttt ttaactaaaa aaatgttttc tcttccattt 18000

agatcaatac tataacattg atgtgcctag tagaacagca agagcaatta ctactcgttc 18060

tgggcagacc ttatccaaag tctggtacaa ctgtccttag actgatgagc aaaggaggag 18120

tcaactaatg aagaaatgaa taataaattt tgacactgaa aaacatttta ttaataaaga 18180

atattgacat gagtatacca gtttatatat aaaaatgttt ttaaacttga caatcattac 18240

actaaaacag atttgataat cttattcaca gttgttattg tttacagacc atttaattaa 18300

tatttcctct gtttattcct cctctccctc ccattgcatg gctcacacct gtaaaagaaa 18360

aaagaatcaa attgaatata tcttttaaga attcaaaact agtgtattca cttaccctag 18420

ttcattataa aaaatatcta ggcattgtgg atataaaact gttgggtatt ctacaacttc 18480

aatggaaatt attacaagca gattaatccc tctttttgtg acacaagtac aatctaaaag 18540

ttatattgga aaacatggaa atattaaaat tttacacttt tactagctaa aacataatca 18600

caaagcttta tcgtgttgta taaaaaaatt aacaatataa tggcaatagg tagagataca 18660

acaaatgaat ataacactat aacacttcat attttccaaa tcttaatttg gatttaagga 18720

agaaatcaat aaatataaaa tataagcaca tatttattat atatctaagg tatacaaatc 18780

tgtctacatg aagtttacag attggtaaat atcacctgct caacatgtaa ttatttaata 18840

aaactttgga acattaaaaa aataaattgg aggcttaagg attagaaaac ttgttccatt 18900

tattactatt ccacattcta tcatgtaatc cagttatgtc agaaattcac ctctctgtct 18960

gttaaactga cgaccacgga caccttgtct caatgttgtc tgaagtctta ctcgtctgaa 19020

aggctttggt tgactactac tagtatctaa gaaaaacatt aagataaaag aaaagcaagt 19080

gaaaaaatga gaaagcattt ataatgatac aaactaatta ctaaatgaat gaatgaataa 19140

gatgtagact tgttcatttg atttcaattc tttttttcaa acaatgagga acatttaaca 19200

aaccgaaaaa gggtaaatgg ttaaacaaca aaaaaagcag cactttggga ggctaaggtg 19260

ggaggatcac ttgagctcag gagttcgaga ccagcctggg caacacagcg agaccctgtt 19320

tctacaaaat tataaaacaa ttagctgggc atggtggaac atgcctacag tcccagttac 19380

tcaggaggct gaggctagag gatcacttga gcccgggtga ggttgaggct gcagtgagcc 19440

atgatcaagc taacagtact ccagggagac cggtgagatc ctgccttaa 19489

<210> 2
<211> 1404
<212> PRT
<213> Artificial sequence

<220>
<223> Mutated PRG4 protein (aminoacid change R180->W)

<400> 2

Met Ala Trp Lys Thr Leu Pro Ile Tyr Leu Leu Leu Leu Leu Ser Val
1               5                   10                  15

Phe Val Ile Gln Gln Val Ser Ser Gln Asp Leu Ser Ser Cys Ala Gly
            20                  25                  30

Arg Cys Gly Glu Gly Tyr Ser Arg Asp Ala Thr Cys Asn Cys Asp Tyr
        35                  40                  45

Asn Cys Gln His Tyr Met Glu Cys Cys Pro Asp Phe Lys Arg Val Cys
    50                  55                  60

Thr Ala Glu Leu Ser Cys Lys Gly Arg Cys Phe Glu Ser Phe Glu Arg
65                  70                  75                  80

Gly Arg Glu Cys Asp Cys Asp Ala Gln Cys Lys Lys Tyr Asp Lys Cys
                85                  90                  95

Cys Pro Asp Tyr Glu Ser Phe Cys Ala Glu Val His Asn Pro Thr Ser
            100                 105                 110

Pro Pro Ser Ser Lys Lys Ala Pro Pro Pro Ser Gly Ala Ser Gln Thr
        115                 120                 125

Ile Lys Ser Thr Thr Lys Arg Ser Pro Lys Pro Pro Asn Lys Lys Lys
    130                 135                 140

Thr Lys Lys Val Ile Glu Ser Glu Glu Ile Thr Glu Glu His Ser Val
145                 150                 155                 160

Ser Glu Asn Gln Glu Ser Ser Ser Ser Ser Ser Ser Ser Ser Ser Ser
                165                 170                 175

Ser Thr Ile Trp Lys Ile Lys Ser Ser Lys Asn Ser Ala Ala Asn Arg
            180                 185                 190

Glu Leu Gln Lys Lys Leu Lys Val Lys Asp Asn Lys Lys Asn Arg Thr
        195                 200                 205

Lys Lys Lys Pro Thr Pro Lys Pro Pro Val Val Asp Glu Ala Gly Ser
    210                 215                 220

Gly Leu Asp Asn Gly Asp Phe Lys Val Thr Thr Pro Asp Thr Ser Thr
225                 230                 235                 240

Thr Gln His Asn Lys Val Ser Thr Ser Pro Lys Ile Thr Thr Ala Lys
                245                 250                 255

Pro Ile Asn Pro Arg Pro Ser Leu Pro Pro Asn Ser Asp Thr Ser Lys
            260                 265                 270

Glu Thr Ser Leu Thr Val Asn Lys Glu Thr Thr Val Glu Thr Lys Glu
        275                 280                 285

Thr Thr Thr Thr Asn Lys Gln Thr Ser Thr Asp Gly Lys Glu Lys Thr

290                 295                 300

Thr Ser Ala Lys Glu Thr Gln Ser Ile Glu Lys Thr Ser Ala Lys Asp
305                 310                 315                 320

Leu Ala Pro Thr Ser Lys Val Leu Ala Lys Pro Thr Pro Lys Ala Glu
                325                 330                 335

Thr Thr Thr Lys Gly Pro Ala Leu Thr Thr Pro Lys Glu Pro Thr Pro
            340                 345                 350

Thr Thr Pro Lys Glu Pro Ala Ser Thr Thr Pro Lys Glu Pro Thr Pro
        355                 360                 365

Thr Thr Ile Lys Ser Ala Pro Thr Thr Pro Lys Glu Pro Ala Pro Thr
    370                 375                 380

Thr Thr Lys Ser Ala Pro Thr Thr Pro Lys Glu Pro Ala Pro Thr Thr
385                 390                 395                 400

Thr Lys Glu Pro Ala Pro Thr Thr Pro Lys Glu Pro Ala Pro Thr Thr
                405                 410                 415

Thr Lys Glu Pro Ala Pro Thr Thr Thr Lys Ser Ala Pro Thr Thr Pro
            420                 425                 430

Lys Glu Pro Ala Pro Thr Thr Pro Lys Lys Pro Ala Pro Thr Thr Pro
        435                 440                 445

Lys Glu Pro Ala Pro Thr Thr Pro Lys Glu Pro Thr Pro Thr Thr Pro
    450                 455                 460

Lys Glu Pro Ala Pro Thr Thr Lys Glu Pro Ala Pro Thr Thr Pro Lys
465                 470                 475                 480

Glu Pro Ala Pro Thr Ala Pro Lys Lys Pro Ala Pro Thr Thr Pro Lys
                485                 490                 495

Glu Pro Ala Pro Thr Thr Pro Lys Glu Pro Ala Pro Thr Thr Thr Lys
            500                 505                 510

Glu Pro Ser Pro Thr Thr Pro Lys Glu Pro Ala Pro Thr Thr Thr Lys
        515                 520                 525

Ser Ala Pro Thr Thr Thr Lys Glu Pro Ala Pro Thr Thr Thr Lys Ser
    530                 535                 540

Ala Pro Thr Thr Pro Lys Glu Pro Ser Pro Thr Thr Thr Lys Glu Pro
545                 550                 555                 560

Ala Pro Thr Thr Pro Lys Glu Pro Ala Pro Thr Thr Pro Lys Lys Pro
                565                 570                 575

Ala Pro Thr Thr Pro Lys Glu Pro Ala Pro Thr Thr Pro Lys Glu Pro
            580                 585                 590

Ala Pro Thr Thr Thr Lys Lys Pro Ala Pro Thr Thr Pro Lys Glu Pro
        595                 600                 605

Ala Pro Thr Thr Pro Lys Glu Thr Ala Pro Thr Thr Pro Lys Lys Leu
    610                 615                 620

Thr Pro Thr Thr Pro Glu Lys Leu Ala Pro Thr Thr Pro Glu Lys Pro
625                 630                 635                 640

Ala Pro Thr Thr Pro Glu Glu Leu Ala Pro Thr Thr Pro Glu Glu Pro
                645                 650                 655

Thr Pro Thr Thr Pro Glu Glu Pro Ala Pro Thr Thr Pro Lys Ala Ala
            660                 665                 670

Ala Pro Asn Thr Pro Lys Glu Pro Ala Pro Thr Thr Pro Lys Glu Pro
        675                 680                 685

Ala Pro Thr Thr Pro Lys Glu Pro Ala Pro Thr Thr Pro Lys Glu Thr
    690                 695                 700

Ala Pro Thr Thr Pro Lys Gly Thr Ala Pro Thr Thr Leu Lys Glu Pro
705                 710                 715                 720

Ala Pro Thr Thr Pro Lys Lys Pro Ala Pro Lys Glu Leu Ala Pro Thr
                725                 730                 735

Thr Thr Lys Glu Pro Thr Ser Thr Thr Ser Asp Lys Pro Ala Pro Thr
            740                 745                 750

Thr Pro Lys Gly Thr Ala Pro Thr Thr Pro Lys Glu Pro Ala Pro Thr
        755                 760                 765

Thr Pro Lys Glu Pro Ala Pro Thr Thr Pro Lys Gly Thr Ala Pro Thr
    770                 775                 780

Thr Leu Lys Glu Pro Ala Pro Thr Thr Pro Lys Lys Pro Ala Pro Lys
785                 790                 795                 800

Glu Leu Ala Pro Thr Thr Thr Lys Gly Pro Thr Ser Thr Thr Ser Asp
                805                 810                 815

Lys Pro Ala Pro Thr Thr Pro Lys Glu Thr Ala Pro Thr Thr Pro Lys
            820                 825                 830

Glu Pro Ala Pro Thr Thr Pro Lys Lys Pro Ala Pro Thr Thr Pro Glu
        835                 840                 845

Thr Pro Pro Pro Thr Thr Ser Glu Val Ser Thr Pro Thr Thr Thr Lys
    850                 855                 860

Glu Pro Thr Thr Ile His Lys Ser Pro Asp Glu Ser Thr Pro Glu Leu
865                 870                 875                 880

Ser Ala Glu Pro Thr Pro Lys Ala Leu Glu Asn Ser Pro Lys Glu Pro
                885                 890                 895

Gly Val Pro Thr Thr Lys Thr Pro Ala Ala Thr Lys Pro Glu Met Thr
            900                 905                 910

Thr Thr Ala Lys Asp Lys Thr Thr Glu Arg Asp Leu Arg Thr Thr Pro
        915                 920                 925

Glu Thr Thr Thr Ala Ala Pro Lys Met Thr Lys Glu Thr Ala Thr Thr
    930                 935                 940

Thr Glu Lys Thr Thr Glu Ser Lys Ile Thr Ala Thr Thr Thr Gln Val
945                 950                 955                 960

Thr Ser Thr Thr Thr Gln Asp Thr Thr Pro Phe Lys Ile Thr Thr Leu
                965                 970                 975

Lys Thr Thr Thr Leu Ala Pro Lys Val Thr Thr Thr Lys Lys Thr Ile
            980                 985                 990

Thr Thr Thr Glu Ile Met Asn Lys Pro Glu Glu Thr Ala Lys Pro Lys
        995                 1000                1005

Asp Arg Ala Thr Asn Ser Lys Ala Thr Thr Pro Lys Pro Gln Lys
    1010                1015                1020

Pro Thr Lys Ala Pro Lys Lys Pro Thr Ser Thr Lys Lys Pro Lys
    1025                1030                1035

Thr Met Pro Arg Val Arg Lys Pro Lys Thr Thr Pro Thr Pro Arg
    1040                1045                1050

Lys Met Thr Ser Thr Met Pro Glu Leu Asn Pro Thr Ser Arg Ile
    1055                1060                1065

Ala Glu Ala Met Leu Gln Thr Thr Thr Arg Pro Asn Gln Thr Pro
    1070                1075                1080

Asn Ser Lys Leu Val Glu Val Asn Pro Lys Ser Glu Asp Ala Gly
    1085                1090                1095

Gly Ala Glu Gly Glu Thr Pro His Met Leu Leu Arg Pro His Val
    1100                1105                1110

Phe Met Pro Glu Val Thr Pro Asp Met Asp Tyr Leu Pro Arg Val
    1115                1120                1125

Pro Asn Gln Gly Ile Ile Ile Asn Pro Met Leu Ser Asp Glu Thr
    1130                1135                1140

Asn Ile Cys Asn Gly Lys Pro Val Asp Gly Leu Thr Thr Leu Arg
    1145                1150                1155

Asn Gly Thr Leu Val Ala Phe Arg Gly His Tyr Phe Trp Met Leu
    1160                1165                1170

Ser Pro Phe Ser Pro Pro Ser Pro Ala Arg Arg Ile Thr Glu Val
    1175                1180                1185

Trp Gly Ile Pro Ser Pro Ile Asp Thr Val Phe Thr Arg Cys Asn
    1190                1195                1200

Cys Glu Gly Lys Thr Phe Phe Phe Lys Asp Ser Gln Tyr Trp Arg
    1205                1210                1215

Phe Thr Asn Asp Ile Lys Asp Ala Gly Tyr Pro Lys Pro Ile Phe
    1220                1225                1230

Lys Gly Phe Gly Gly Leu Thr Gly Gln Ile Val Ala Ala Leu Ser
    1235                1240                1245

Thr Ala Lys Tyr Lys Asn Trp Pro Glu Ser Val Tyr Phe Phe Lys
    1250                1255                1260

Arg Gly Gly Ser Ile Gln Gln Tyr Ile Tyr Lys Gln Glu Pro Val
    1265                1270                1275

Gln Lys Cys Pro Gly Arg Arg Pro Ala Leu Asn Tyr Pro Val Tyr

1280                1285                1290

Gly Glu Thr Thr Gln Val Arg Arg Arg Arg Phe Glu Arg Ala Ile
    1295                1300                1305

Gly Pro Ser Gln Thr His Thr Ile Arg Ile Gln Tyr Ser Pro Ala
    1310                1315                1320

Arg Leu Ala Tyr Gln Asp Lys Gly Val Leu His Asn Glu Val Lys
    1325                1330                1335

Val Ser Ile Leu Trp Arg Gly Leu Pro Asn Val Val Thr Ser Ala
    1340                1345                1350

Ile Ser Leu Pro Asn Ile Arg Lys Pro Asp Gly Tyr Asp Tyr Tyr
    1355                1360                1365

Ala Phe Ser Lys Asp Gln Tyr Tyr Asn Ile Asp Val Pro Ser Arg
    1370                1375                1380

Thr Ala Arg Ala Ile Thr Thr Arg Ser Gly Gln Thr Leu Ser Lys
    1385                1390                1395

Val Trp Tyr Asn Cys Pro
    1400

<210>  3
<211>  13649
<212>  DNA
<213>  Artificial sequence

<220>
<223>  Epx human gene

<400>  3
gcaccttgga gggagtgagt agggctaggg gataaggaga ggggccaaat cattcatcac      60

agatcattca ttcactcaac aaacacctgc taagcacata ctaaatgctg ggctttgagc     120

tgggtatggg gggtgtgtat aacagtaagg cagattcagt ttctgccctg ttgcaggagg     180

agaggcaccc ttccaatcat ctggcccaac tctcattcta gggatgtgaa aactgaggcc     240

tggagaggga aagtggcttg cccaaggcca tgcagccagg cggtggcgac tgaggtgcac     300

cgctcctgtc agccaacaaa tatccattga gcgacacctg tgtcccaggt gctgctctgg     360

gccctgggag aagtgcatca gtgggcttgg tagtagaggg tagggatgga gtgaagggta     420

ggcaggaaga atgtccccag gctggtagga ggtggggtgg ggggtttcag tctcaaaact     480

cccatgaaaa ccagagagaa gtttcagaac tccacccaag aggctgggtt tctagggccc     540

agagctgccc tcccccaccc tagaatgggc tataaaagtc ccttcccagc tacgtccaga     600

gaagagctgg aggaagtgag aggtcggctg ggggtcctca aagtgagagg ggagcagagg 660

atcctcccgt gcaggctgtg gatgtcactc acttcccagc tggtgaagcc tcgctgcaga 720

gatgcatctg ctcccagccc tggcaggggt cctggccaca ctcgtcctcg cccagccctg 780

tgagggcact gacccaggta atagtcccct agacaggcaa ggaggaggga ggggaaatgg 840

aaggggaagc acttgggtct tggagggggt cttgtggctt gctgaaccct gagtccccat 900

ctctttgaac agcctcccct ggggcagtgg agacctcggt cctgcgagac tgcatagcag 960

aggccaagtt gctggtggat gctgcctaca attggaccca gaagaggtgg acttgggtct 1020

gggggctgca tgggcctggg aggatcagtg aggggcatgg gccccagcca agttcatctc 1080

actcatcagc cacctctgga ccccatgaac atttcctgtt ggtagagcct cccttccatc 1140

catccttctg tccgcttgcc ctgtcctgac tgtgccccag gactgggtct ctgctgggtg 1200

ggtctgcacc ctctctccag ccctcactcc tcctctcctg ggcagcatca agcagcggct 1260

tcgcagcggt tcagccagcc ccatggacct cctgtcctac ttcaaacaac cggtagcagc 1320

caccaggaca gttgttcggg ccgcagatta tatgcatgtg gctttggggc tgcttgaaga 1380

gaagttacaa ccccagcggt ccggaccctt caatgtcact ggtactctga tccccactga 1440

gcccgctggg cctaccctgg cctggagtag aaggaatcca ggagagggag gcagggtgca 1500

caggtttggg gtgctgggag gagagagggt aaagggatgg gaggtacaga gcaggccagc 1560

tcaggtctgc ccatttgcct tcccacagat gtgctaacag aaccacagct gcggctgctg 1620

tcccaggcca gtggctgtgc tctccgggac caggccgagc gctgcagcga caagtaccgc 1680

accatcactg gacggtgcaa caacaagtgc gtgcggggcg gcaggagggg ctgcccctgc 1740

ctgggggacc tctcccttcc tgcacccacc ctctccctcc atgctgagcc atctccaggc 1800

cctgccccct gctaacctat cccacccatg gctgcaggag gagacccttg ctaggggcct 1860

ccaaccaggc tctggctcgc tggctgcccg ccgagtatga ggatgggctg tcgctcccct 1920

tcggctggac ccccagcagg aggcgcaatg gcttccttct ccctcttgtg agttggggct 1980

gagggtttgg gaggttgctt gatctcttaa atgcggggag taaaacacag cccagagtca 2040

cgcaggcagg gcttgaaccc aggcccttcc actgaccagt gcagtgactt gaggcaaata 2100

acacagcttt ctgaggctta gtttcctcat ctgtaaaatg gccctaaaac ctacctcgta 2160

gagcctgtgt ggatattgaa gtccttggta taaatagcta tagaggacat gatctattca 2220

tgctaaccat tacggtgttg tacggttttt tctgagaaaa ttagtgtcaa agtcttgcta 2280

aatataaaca gctctatacc ttaatctggc tttatctatt gatctatttc acatttatca 2340

actcatcagt tttctattga tatatcgata gagcagtcta tcatctaccc atctatcaac 2400

atctattaat taattttctt ctatccaccc ttccatctgc tgaccccgta ctccctacgt 2460

gtattatcca tcacctggca accagcccat gcacctgtga atccgtgcta tcatctggtt 2520

agctatcagt gtacaaattg atctatctat ttgatctccc ttcccctctg ggagagctgg 2580

tgaggtctga gccagtcaac ctagcccctc tccttctctt taccaccgga atcctcagga 2640

gcccagccag aaaccatcct tctaggaatg agagcaggag gtggctacgc ctccagggac 2700

aaaaggggca tggagggcag aagaggagag gctgtcaatt ccagcagggg agctgctgct 2760

ccctgagtcc tgggttggct ctaatacctt gtggggtcag ggagcccatg tcccgtgctg 2820

atgttatttc cccaccaggt ccgggctgtc tccaaccaga ttgtgcgctt ccccaatgag 2880

agactgacct ccgaccgtgg ccgagccctc atgttcatgc agtggggcca gttcattgac 2940

catgacctgg acttctcccc ggagtccccg gccagagtgg ccttcactgc aggcgttgac 3000

tgtgagagga cctgcgccca gctgcccccc tgctttccca tcaaggtacc taccctcagc 3060

caatctccca tgcccttgtg tggcctcccc caaaggcaag gtgctggggg tggggatctg 3120

gaagactgga gcaccatcct taaggagctg cctgtggagc tagggtatga gacagagaca 3180

caagaaacac agctgagcag agaccccgcg ccgtgtgtgt ttgagaggtg ggggtagggc 3240

aatctgccag gaggctcagg tcaggcttca tggagtggga agcccttgac acatgccttg 3300

acccatgggt ctgaatccaa caggggaagc ctctgggccc ctgcttttgg caacctaagg 3360

gcctcttagc tcttgccctt ctctccttcc cagagtagtc caagggtcct gcggctcttg 3420

ccagcttctt gggcttgggc tgtaagtagg gtcctaacct tcaggctaga agccaaggac 3480

agtgtggggc actaggggag gaaagataga atccagggag cagagtctct gctaggaaca 3540

tagggttgac acacgtgcac acacacactc tctcacacac acacacgtgc acacacacgc 3600

tccctctcac acacacatgt acacacacag tcacccttaa taggtggggc cacattcatg 3660

agggatgaca tgtgagggcc tctggatggc ctctccctgt tgcaacctta tctattcctc 3720

aatccctggc ttaggggcac ctcctctggg gagccttctc agatcttcct attacacttt 3780

taacatatgt cctcccctcc cctctcctct ctctccttcc ttccttcttc cttcctcttt 3840

ctttctttct tctttctttc ctttccttcc ttccttccct tccttccttc atttccttcc 3900

ttcctttctt tctttctttt tctttctttt tctttcttcc gtcctttctt tctttatctt 3960

tccttctttc tttcttcttt attattatta ttattgtttt gagacagaat ctcactctgt 4020

tgtccagact ggagtacaga ggggcaatct cagctcactg caacctctgc ctcctgggtt 4080

caagcagttc tcatgcctca gcctcccgag tagctgggat tacaggcatg tgccaacaag 4140

cctggctaat ttttgtattt ttagtagaga ttgggtttca ccatgttgac aaggctgatc 4200

tcaaacttct ggcctcatgt aatccacctg cctcggcctc ccaaagtgct aggattatag 4260

gcgtgagcca ccacacctgg cccacatatg gcttttctat caagcacagc agatgccact 4320

tcaacttagt ttcttaactc tatgtgtgtg tgtctttttc cactgaactg ccagcttcct 4380

gcaacctttc ctgtgttttt ttaaccttca taatctccaa agcagcaagc aagaaagggg 4440

cttagaaaag gtctgtcgaa ttgaacagaa ttaaagaagc tcaggcctaa gagtcaagag 4500

aactaagttc taatcccacc tttcctgcta actggttgtc acttcccctc tctgaggctg 4560

ttttccttgc tgtaaaatga ggatcgagat tgtttcaaaa gcctcattca ttcctaacct 4620

tttgagactc tatgaaacaa aatgttaagg acactcagac tgacggggtg agcagcatga 4680

gcctgggtga gtcaaggagg gcttcctgga ggaagggagt tttaagcaag gttttgagga 4740

ggtggaagat gacggagagg agtgagtctg ctattgaggg ggccccatgt cactgtctcc 4800

tcttccatct cagatcccac ccaatgaccc ccgcatcaag aaccagcgtg actgcatccc 4860

tttcttccgc tcggcaccct catgccccca aaacaagaac agagtccgca accagatcaa 4920

cgcgctcacc tcctttgtgg acgccagcat ggtgtatggc agtgaggtct ccctctcgct 4980

gcggctccgc aaccggacca actacctggg gctgctggcc atcaaccagc gctttcaaga 5040

caacggccgg gccctgctgc ccttcgacaa cctgcacgat gacccctgtc tcctcaccaa 5100

ccgctcggcg cgcatcccct gcttcctggc aggtcagaca gggaggaagg tggtgtcttc 5160

ccaggaaaca gccatccctg gggtcccaac tgggaagcaa tggtgggatg tggtgaaggt 5220

acatggtttg ggacctcagt attaggcaca ccataagcat ggatctgtgc acagccatca 5280

tagaatcaga acgttgagtc cctttgcaag ctccctgtgt gtggccagag ataactgctt 5340

aaccgtctcc agtgacaagg ctctcaccgc ctctatggca ccccagctca gccacaggca 5400

gctccgactg ttagaaagtc tccttatttt gatcacatat tcacctattc cccttaatca 5460

tgcttctgga acctcagaaa agacactgtc cctcttacac gcaggaatac ctcaaagtag 5520

ctgttaactc catctccatt ttttttctct tctctgggat aaatttccgt agttcctccc 5580

tgtccccagc tgttgctaga cagttaatac cctcttctgg aagcataatt tgaactttcc 5640

tcagggtctt ttgtcccatg tacatggtta caataataat ggtagtcatt tctcattaag 5700

atatttatct tattattatt attattttaa gatggagtct cactctgtcg cccaggctgg 5760

agtgcagtgg cgtgatctca gctcactgcc acctctgcct cctgggttca agtgattccc 5820

ctgcctcagc ctcccgagta gctggaatta caggtgcctg ccaccacgtt tggctaattt 5880

ttgtatattt agtggagata gagtttcacc atgttggcca ggctggtctc gaactcctga 5940

cctcaggtga ttcacctgcc ttggcctccc aaagtgttgg gattacaggt gtgagccacc 6000

acgcccaacc ttatcaagat atttaaatgt gccatctcta aaccttgcaa cagccctgaa 6060

aggagggaat tattattgtt ctcattttac gataagaaac tgagactcag ggaaagtgaa 6120

tcatgcgccc aagagcacag agttagtttc aaatgacaga gtccacttga gaacacagtc 6180

agtgtagtat caaggtctct gcccagtcca ctgtattcca ctgttttcct cacagagagt 6240

cggccccaac ccctcttttg aaggattgta catttgtgtg tcctggaaag agcagcttgt 6300

gccttcggga gaggtcaact gaactaagac cttgtgcaaa agggtttagt taaaaccaag 6360

caaatgatgg aattaacttt ctttccctgc ctgtagtagg tctgttcgta aggacagtct 6420

ctgcccaacc ctccctctct tcctctttcc ctctggttca cgggggagag cagtattata 6480

ccaagcccaa agataggaag aggtacacca gcccttggca gagaacacgc aggcagatgc 6540

agctcatgga caggtgggct ctcagtccca aagtcaaggt gcccttgccc atagctggtc 6600

caggctatgg atctggttgt cttgtagagt agaatcctta ggcaaagatg cccctgcctg 6660

agaccagcag ctgccctgtg ctttcccacc tcacccaagc ctgctaggaa cccccggtcc 6720

ttccgctcct ggcctgtggt gttagggagg agggtggcga tgaggagcct cagcacagct 6780

gaagagatgg aggtccagtg agggccagga gtttggccca ccccgtctct cccatcccca 6840

gccctgggtc taccctggta gaaagacatt tctctgggaa aggctgcagt aaatctgagc 6900

ttggggtttt caaggtgaca cccgatcaac ggaaaccccc aaactggcag ccatgcacac 6960

cctctttatg cgagagcaca accggctggc caccgagctg agacgcctga atccccggtg 7020

gaatggagac aaactgtaca atgaggctcg gaagatcatg ggggccatgg tccaggtaag 7080

gagctctgca tcccagcatc ccccagatga caagcttggc atgagaagca gtccttaaca 7140

catccttgcg gatgtgccta aaaccagctg ggtctgggca actggcggag cacctggacc 7200

tgtcctctgg ccccgattct gcctctaact cccgtgtgac cctgtctgtg tcactcaccc 7260

tctctgggct ttgtatctcc acccaccaat agtaaattaa tgttgtcaca tttgacgtga 7320

tgacaataaa gaatatgtct gagccaccct ttgaaaaggc aagggtatgg gtgagtagcc 7380

tctggggaat gttcctcctg tcttcccttc cagatcatca cctaccgaga ctttctgccc 7440

ctggttctgg gcaaggcccg ggccaggaga accctggggc actacagggg gtactgctcc 7500

aatgtggacc cacgggtggc caatgtcttc accctggcct tccgctttgg ccacacaatg 7560

ctccagccct tcatgttccg cttggacagt cagtaccggg cctccgcacc caactcgcat 7620

gtcccactta gctctgcctt ctttgccagc tggcggatcg tgtatgaagg tgaccaggtt 7680

ttccaggggg caaatggggg tgagggtggg gagcatgccc tcccctaggt gggccaagct 7740

tactgccagg aagccaggct gctgcagagg ccactgctaa tatctcccca ggacagtgga 7800

aacaaggcag gtgccagcaa gaccctcagt cacgggcctc ccatcctgtg tggaatgaga 7860

ggatttttta aaggggtgga gactaatgtc agattgatgg ggagctcacc ttccattcct 7920

taagaagtac ctcccagctc cagctgcttc atgtctctcc agaactctgt ttcctgacaa 7980

acgttactaa catacccgac tggcttgtcc agctctgggc tagcttggca tcatgtgata 8040

acccaagtag cttcccagag gctggtccaa tctgtgctgc tcacattccc tgccaccagg 8100

gggcatcgac cccatcctcc ggggcctcat ggccacccct gccaagctga accgtcagga 8160

tgccatgtta gtggatgagc tccgggaccg gctgtttcgg caagtgagga ggattgggct 8220

ggacctggca gctctcaaca tgcaacgaag ccgggaccac ggccttccag gtgagggggc 8280

tgtccacctc ttctcccagc tttgctcggg ccaggctgct caaggggttc tgggaagacc 8340

ctggtacctc ctttctgact gggactgtct acaggatgtg caggagtgca gaggcatgca 8400

aggccaaggt cgatgtccca aagcactcct ggaacaccgc ttgctgtcct gccatggctc 8460

ccccatccac tgtaggggct ccagctttag gttcaccaca ggaccagcac ttaggctcag 8520

gagctgaaaa agggaggctc gaactttcca agtagaaaaa aagcaatagg cttaaaaggc 8580

agggaagaac tgctccattc ccaacacgct ggcaagttcc aaggaggaag gctaaaattc 8640

cagagctaca gaaacgctcc ttgctctaag tggagaaagg caaagagcct tagaggcagg 8700

gaataggtgg aataaaacta aggctgcagt ggctgtttgg tgagggcaca aatgacttta 8760

tcccaggaaa gaagaaaata taggcacttt gttagtcact ttcatgtata ttaatttatt 8820

ttgttctccc aatcaccttg agaagcaaat gattacagcc atttatagaa tgagaaacta 8880

tgttcagaga cagctagtaa tttgcccaag atcacataaa taagaagtag taaaatctga 8940

atttaaattt acaatttctc ctttcttccc cctgttcaac cccagtaggg gaaggttttg 9000

gccactgcct tgctcctctc tccttcccac ctcctacctc cctgtctaga aagaacctga 9060

aggcccatct gcccatgtat gaagcagtgg tggcacctcc tactgcccac cccacttcac 9120

acagcgcctg gtagagcaca gcaggcactc cggaaacctg agcttctttc ctttcttaaa 9180

cttgggcgct cctggcagct aaggagctgt gggcaccccc tgccggagag ctgagatcgc 9240

tagccaccca agtttctatt aggttttgaa gggaaggcaa gggttaagga aagacacaaa 9300

gagagagggc ggctgaacag ccaatgcagg tatttatttc cagcataaaa cctacagagg 9360

tgggggacca gcttattgcc agaacctgcc gccgcctaca gctggggtac tgggtatggg 9420

tatgggtatg ggcaggaggg gtctgggtcg tatggcttgc tacccagcag gatattgata 9480

agatgtacct atgatcaggt ccttttttca gtgggatgtg gtaagatgtt ccttggacct 9540

ttgcccagca ggatatgata gggatgttcc ttcagttggg cctttgccca ttagggtatg 9600

ttatgttttt catggcccga cctcctgtgg aacgtttcac tttgaccaag gtctgggaaa 9660

tggtgggggg cttagaaaat ggtgcagttt ggactaacat tcttgccttc tactttggta 9720

taaaaggaag agctggagag ctgagatcgc tagccaccca agtttccaca ttgcgagctt 9780

ctgctccccg caatacctgt gccctccaga aatattatcc tgacactagc tcttgcaatc 9840

tcctctaatc ccttcccaag tccaacatca aactgggaac tggacaacct ggtttgagct 9900

ccacttaaca gtaaagtgac cctgaagcct atggttgctc aactttagta aacataccag 9960

atatagttag catgccataa aagcacctca aaaaggtgct gtgagggtcc tgagatattc 10020

acccaatagt gctcagtaat ggtaaaatac tgtgtggttt tcatgggttc cgctggggga 10080

tggtgtgtgt gagctgtagt tcaatggatg acgttgggct ggtgtccctt cgggacatgg 10140

caaagctatc tcctaccttg attgtcacat cttatgatgt ggcttggccc tgcgtgggat 10200

cctgatggct gacaaattgg tatggcttgt ctttggaagt ttctgaaaac tttttaaaaa 10260

cttctttatt gagatatacc ataaaattca cccatatgaa atagacaatt caatgatttt 10320

agtgtattta gagttatgca cccatcacca tgatctaata tcacaccatt ttcactcccc 10380

aaagaaacct tgtaagaaca gtcaatcccc atccccaccc cagccccaat cacctctaat 10440

atactttgtg tctctatatt gacctatgca ggacctctca tatcaacaga atcatagtgt 10500

gccgtccttt tctgagtaga tgaattttat cattgctctc catttgtttg cttgttcatt 10560

catttattca tttgctcact cattcattct acaaacctga tggagagtct tttatattct 10620

gggtactgtc ttacttgtgt ctaatgaatg cagtcgagcc cagtaatatc actaagtgtc 10680

ggatttgtgg ttatgagttt tgggttaaaa cagcttttta ggttaggaca cactcctcag 10740

tctgatgcac actgagcaca gttgtccact cacattttca atcagaggac agaaacctag 10800

tctttggaag ctctagggac tcttctgcca gcaggatctt ctggttctgc ccaatattga 10860

ctggccacag cttcccccca gaggactggt ggagaaaaac agaagctaat gggagatcag 10920

caagactgaa gctgcttctc cccgttcccc tgcagggtac aatgcttgga ggcgcttctg 10980

tgggctctcc cagccccgga atttggcaca gcttagccgg gtgctgaaaa accaggactt 11040

ggcaaggaag ttcctgaatt tgtatggaac acctgacaac attgacatct ggattggggc 11100

catcgctgag cctcttttgc cgggggctcg agtggggcct cttctggctt gtctgttcga 11160

gaaccagttc agaagagccc gagacggaga caggtaagtg accctatcat aaaagacatc 11220

agcaccagag gcagagcaga aaaacactag catttcaaga ctaaacattg aagaacactg 11280

ctctttttag tatcatttct tccaagttca caggatctga aatcaggagg ctcctctctg 11340

aaaagctggg tcaaaggaag agagacactg acccaggcaa ggcccatatt gcctgagctg 11400

gagtcatctt aaacccagag gttgcctggc agcctcacta ttctgggttg gtggatccca 11460

gaaaacatgg gcagaaaggg ctaaatctgg tttccctcca atacatttgt gatttaagac 11520

ctgacggccg ggcgcggtgg ctcacgcctg taatcccagc actttgggag gccgaggcgg 11580

gcggatcacg aggtcaggag atcgagacca tcccggctaa aacggtgaaa ccccgtctct 11640

actaaaaata caaaaaatta gccgggcgta gtggcgggcg cctgtagtcc cggctacttg 11700

ggaggctgag gcaggagaat ggcgtgaacc cgggaggcgg agcttgcagt gagccgagat 11760

cctgccactg cactccagcc tgggcgacag agcgagactc cgtctcaaaa aaaaaaaaaa 11820

aaaaaaaaaa aaaaaaaaaa aaaaagacct gtctccttat ttggagttgg tattgcccga 11880

gttcaaatcc agccctggcc acttaaatta ctgtgcactt aatactgggc aatttatttg 11940

gccactgaat ttgtttcatt tctgagattc caatcttgca ggaattttgt gagaattgaa 12000

tggaataata tatgtaaagt acctggcaca caacaggtgc tcattataag gtaattcctc 12060

cccagccttc acccacatct ctcgactgcc tggtaggttc tggtggcaga aacgaggtgt 12120

tttcaccaaa agacagcgca aggccctgag cagaatttcc ttgtctcgaa ttatatgtga 12180

caataccggt atcaccacgg tttcaaggga catcttcaga gccaacatct accctcgggg 12240

ctttgtgaac tgcagccgta tccccaggtt gaacctatca gcctggcgag ggacatgagg 12300

cttctgcagg taaggggagg ccacctccag caccctgggc tgggttaagc cctcacatcc 12360

ttccctggat ggatggctga gtcctcttag gtctctaagc agagaaaaca gaacttgtca 12420

ctaggtactc tttccaagtg gcttcccaat gtgctagttt ctgggctgac agtcaattcc 12480

aggccctagg actttggggg gaaattagga gcatccaact atcctcagct tggtcactaa 12540

tacctgaaaa cagctccatt tctaaacatc cagtaaattc ctgacttatc cagttctgtt 12600

acttccacag gagtctatcc caagtctcca acttttggag acaaggggaa ggggaggacc 12660

atgaggctgc cttgtctccc tggagcaagt gcaggctgct gacgcttctg ctggctacag 12720

ctcagagctg ggttccccag ccaggagtga aggctggggg ctcctatcag caatggacct 12780

tcccgccttg ggagcctctt aggtattagg ctatgaatca gcgccacgtg caaaggcttg 12840

ggagccaagc catgtggtct tgcaccccag gcaagaaaag tcagctggag ggtttacagc 12900

actttctact gtttcccagc cctccctccc ctccctcacc atgactaaga gaccactcgg 12960

tcctagcctc cagacacccc acaatactcc tctgagcctg aggccaggca gcatgctctg 13020

cttctaccaa taaagcactg ctaagggcag gggctgctgt ttctgtgttg ggctagggaa 13080

gacgtggcct cagttccatg ctttgctcca gatgtttaag agaagtgctg ttaacacgca 13140

agctggcaca accatctaag cctctcctgg gggctgaatt aggagagccc aaagaaacac 13200

tgtggagtag ggaaaggcag aaaaggggaa gggcagacat ggtaggggcc ccgacattaa 13260

gaacacccta gccataccag caacctgagc agacaaacag ccaacagaac atcagtcaaa 13320

ataaaagggg tttatgtaac aaaaaacaaa aaaacaaaca aacaaaaaaa cacagtaaaa 13380

gataccaccc agctagcaga aaggactcag cactgccttc agccttcact tactcagaaa 13440

taactcatat ctcaacctca gcaagcccca agcagtagaa tgaggcttcc ctggaaagag 13500

gctgagactc acaggctggg gatttaagac cctctcaccg tccaccttcc ttccttcttt 13560

ggtcttggaa gatgaaaggc tccattttta aagggtggag aacagcagat cccctgctac 13620

tgtgagacaa gccagaaaag tgagtcaag 13649

<210> 4
<211> 715
<212> PRT

<213>  Artificial sequence

<220>
<223>  Mutated EPX protein (amino acid change Q122->H)

<400>  4

Met His Leu Leu Pro Ala Leu Ala Gly Val Leu Ala Thr Leu Val Leu
1               5                   10                  15

Ala Gln Pro Cys Glu Gly Thr Asp Pro Ala Ser Pro Gly Ala Val Glu
            20                  25                  30

Thr Ser Val Leu Arg Asp Cys Ile Ala Glu Ala Lys Leu Leu Val Asp
        35                  40                  45

Ala Ala Tyr Asn Trp Thr Gln Lys Ser Ile Lys Gln Arg Leu Arg Ser
    50                  55                  60

Gly Ser Ala Ser Pro Met Asp Leu Leu Ser Tyr Phe Lys Gln Pro Val
65                  70                  75                  80

Ala Ala Thr Arg Thr Val Val Arg Ala Ala Asp Tyr Met His Val Ala
                85                  90                  95

Leu Gly Leu Leu Glu Glu Lys Leu Gln Pro Gln Arg Ser Gly Pro Phe
            100                 105                 110

Asn Val Thr Asp Val Leu Thr Glu Pro His Leu Arg Leu Leu Ser Gln
        115                 120                 125

Ala Ser Gly Cys Ala Leu Arg Asp Gln Ala Glu Arg Cys Ser Asp Lys
    130                 135                 140

Tyr Arg Thr Ile Thr Gly Arg Cys Asn Asn Lys Arg Arg Pro Leu Leu
145                 150                 155                 160

Gly Ala Ser Asn Gln Ala Leu Ala Arg Trp Leu Pro Ala Glu Tyr Glu
                165                 170                 175

Asp Gly Leu Ser Leu Pro Phe Gly Trp Thr Pro Ser Arg Arg Arg Asn
            180                 185                 190

Gly Phe Leu Leu Pro Leu Val Arg Ala Val Ser Asn Gln Ile Val Arg
        195                 200                 205

Phe Pro Asn Glu Arg Leu Thr Ser Asp Arg Gly Arg Ala Leu Met Phe
    210                 215                 220

Met Gln Trp Gly Gln Phe Ile Asp His Asp Leu Asp Phe Ser Pro Glu
225                 230                 235                 240

Ser Pro Ala Arg Val Ala Phe Thr Ala Gly Val Asp Cys Glu Arg Thr
                245                 250                 255

Cys Ala Gln Leu Pro Pro Cys Phe Pro Ile Lys Ile Pro Pro Asn Asp
            260                 265                 270

Pro Arg Ile Lys Asn Gln Arg Asp Cys Ile Pro Phe Phe Arg Ser Ala
        275                 280                 285

Pro Ser Cys Pro Gln Asn Lys Asn Arg Val Arg Asn Gln Ile Asn Ala
    290                 295                 300

Leu Thr Ser Phe Val Asp Ala Ser Met Val Tyr Gly Ser Glu Val Ser
305                 310                 315                 320

Leu Ser Leu Arg Leu Arg Asn Arg Thr Asn Tyr Leu Gly Leu Leu Ala
                325                 330                 335

Ile Asn Gln Arg Phe Gln Asp Asn Gly Arg Ala Leu Leu Pro Phe Asp
            340                 345                 350

Asn Leu His Asp Asp Pro Cys Leu Leu Thr Asn Arg Ser Ala Arg Ile
        355                 360                 365

Pro Cys Phe Leu Ala Gly Asp Thr Arg Ser Thr Glu Thr Pro Lys Leu
    370                 375                 380

Ala Ala Met His Thr Leu Phe Met Arg Glu His Asn Arg Leu Ala Thr
385                 390                 395                 400

Glu Leu Arg Arg Leu Asn Pro Arg Trp Asn Gly Asp Lys Leu Tyr Asn
                405                 410                 415

Glu Ala Arg Lys Ile Met Gly Ala Met Val Gln Ile Ile Thr Tyr Arg
            420                 425                 430

Asp Phe Leu Pro Leu Val Leu Gly Lys Ala Arg Ala Arg Arg Thr Leu
        435                 440                 445

Gly His Tyr Arg Gly Tyr Cys Ser Asn Val Asp Pro Arg Val Ala Asn
    450                 455                 460

Val Phe Thr Leu Ala Phe Arg Phe Gly His Thr Met Leu Gln Pro Phe
465                 470                 475                 480

Met Phe Arg Leu Asp Ser Gln Tyr Arg Ala Ser Ala Pro Asn Ser His
                485                 490                 495

Val Pro Leu Ser Ser Ala Phe Phe Ala Ser Trp Arg Ile Val Tyr Glu
            500                 505                 510

Gly Gly Ile Asp Pro Ile Leu Arg Gly Leu Met Ala Thr Pro Ala Lys
        515                 520                 525

Leu Asn Arg Gln Asp Ala Met Leu Val Asp Glu Leu Arg Asp Arg Leu
    530                 535                 540

Phe Arg Gln Val Arg Arg Ile Gly Leu Asp Leu Ala Ala Leu Asn Met
545                 550                 555                 560

Gln Arg Ser Arg Asp His Gly Leu Pro Gly Tyr Asn Ala Trp Arg Arg
                565                 570                 575

Phe Cys Gly Leu Ser Gln Pro Arg Asn Leu Ala Gln Leu Ser Arg Val
            580                 585                 590

Leu Lys Asn Gln Asp Leu Ala Arg Lys Phe Leu Asn Leu Tyr Gly Thr
        595                 600                 605

Pro Asp Asn Ile Asp Ile Trp Ile Gly Ala Ile Ala Glu Pro Leu Leu
    610                 615                 620

Pro Gly Ala Arg Val Gly Pro Leu Leu Ala Cys Leu Phe Glu Asn Gln
625                 630                 635                 640

Phe Arg Arg Ala Arg Asp Gly Asp Arg Phe Trp Trp Gln Lys Arg Gly
                645                 650                 655

Val Phe Thr Lys Arg Gln Arg Lys Ala Leu Ser Arg Ile Ser Leu Ser
            660                 665                 670

Arg Ile Ile Cys Asp Asn Thr Gly Ile Thr Thr Val Ser Arg Asp Ile
        675                 680                 685

Phe Arg Ala Asn Ile Tyr Pro Arg Gly Phe Val Asn Cys Ser Arg Ile
    690                 695                 700

Pro Arg Leu Asn Leu Ser Ala Trp Arg Gly Thr
705                 710                 715

<210>  5
<211>  28057

<212>  DNA
<213>  Artificial sequence

<220>
<223>  PON1 human gene

<400>  5
gaagacgttt tgtcctgttc tgtagcatct agaacagcgc ctggcacata gtaggtactc      60

aataaatgcc agctgcatga ggaaatgaat gagctgtgtg ggggatgtac ttgagtgaac     120

tctaaagtca gagtggtgtt gagagaaaaa tgcttgaaat ccagatgttg gaaggtgaca     180

cagagtagta gcctggtgag aacagttaga tcttaggggt tcctactaca gccctccctt     240

ccgcaccttt ttggctgtca ccatgatcaa gctactgaat ctctctgaga cgcaaggacc     300

gggatggcac aaagtgagtg ctcaccaaag cttgactgtc ctttcccatg gcaatttact     360

tcagcttgtt tgatttcccc tccccgactg gactaggcac ctattctctg tcttctctct     420

ttacagttgg aaggagcaaa atggactttt tggctgaaag tgctgagctc ctgcggtggg     480

ggctgaccgc aagccacgcc ttctgtgcac ctggtcggcc cagctagctg ccgacccggc     540

ggggaggggc ggggcgggcc aatcggcgct gccccagcag ggctgcggct gcaggcaggc     600

agagcctcct agcccgtcgg tgtctgcgcc catcgatccc tttgtctatc cccgaccatg     660

gcgaagctga ttgcgctcac cctcttgggg atgggactgg cactcttcag gaaccaccag     720

tcttcttacc agtaagttgg gttgtggtga gggctgcagc cttcgtcctc ctcactccct     780

gccagctcca cgagccccca gaaagttggg tccaggctgt taacagaaca tttacaagca     840

ggtgctagat gcacactctg cgggggtttc ataaatgttt gtccagccgt catgtgtcac     900

ttgaaaacaa actcttgctc atggcctgag acatgggata aggaggcaat tgccaggggt     960

gtgagtgccc aggttgtcag catacacgaa ttaatttttc atgaagattt gtgtgccctg    1020

gctacagatt cacttctcaa cctggaaaga aacaaactaa cctggctggg ggttctcctg    1080

atattttgtg tattagagaa aggagcccct gtcgcttctg cgccttgact gcctggccat    1140

ggcaaacctt ccaggcttga agattctcaa attgaggcta ctgagggcca cagcaaaact    1200

gcgtgtggac ccgagcaggt ggagctaacg tgtgctgtga tggttcgctt cagtgggggc    1260

aggcggggaa gagggaaccc tggagccagc atctgatgtc ctggggttta gctctgcccc    1320

atgagtatgc gatttgggca aatcacttta catctctaac cttagctgct tcagtaaact    1380

ggaaatagta atacacactg cctgcgacag agtagctatt caataaatgt tagttccctg    1440

cttcccttta aagtatttaa taaaggttta agtgctatac aagggggtgg gggatatttt    1500

aagaatgtat caaaagtttc ataactttta acaggagttc aaagtctaca gatcatccta    1560

gtctctaagg cttagtcaac aatgccttat ttactttaaa gtaaaaccta ataaaaatta    1620

aatataaaaa aattatatga gcttatggac taattagaag cttacattca gaatgggttt    1680

ttactattta ttgtggatgt ttttctaatt tttttaagtt acagagtgca gaaaaaaagt 1740

aagaagcagt tggtattctt ggcttttaaa tcatgtaaaa attacagttt gtgagttttt 1800

attaatcaca gtgctggtgg ctgccaaagg agattatagg tatagataaa aatatataga 1860

taaaggatgg gttgctgagc ctcctggagt gacccagaca gaggaaggaa gggaaatttt 1920

acccactgtt ggaaacccct aaaacctagc tactcaactg ggtctgggga ccagatgcct 1980

gagcatcacc taggggctcc ctgtacaggc agaatctcaa gccctacccc acactattga 2040

atcagaacca gcgtcatgga acaaactccc tgggggatta gagatcatga atctgcccca 2100

gggagagaag gcaagagatg atatcatgag gcctgcatca ggagcaagcc aaatccccag 2160

gatagatgca tccttttttc ttcagacttg gagtgtatgt gtgtgcggct cacaggaagc 2220

tatatatgcc tgggctggag ggaacttaag gaagcacaca agggatgcct caagagattt 2280

ctgagtttcc caggtgggag cagtacagag aaaagaactg agagagttcc cctagaccac 2340

gagtctccca cccacgagtc tcccaccttt gtagtcggct gtatgtgccg cacaagtcca 2400

gggaatgcct tggatgatgg tagaaacagc tcccctcccc taccccaact tgggctatgg 2460

aatgttgcag catctgtatg gcaacttcct tgtccctaca ttgctaattg caatacgtga 2520

gtccactgag tttcccttct cagaaggaaa ccacaaagct aacgttgggc ctctcgatgc 2580

atttctgcca ttccatgctt actgtaggca acacacagtt gtgggaagag agtagttgtg 2640

gattcctgat gacctgggtt agatatcatt tctgcaactt tcaagttatt ttaccacttt 2700

attccagaaa agttccagcc agtttctcca cccgaaaatt aggggataat aatacatatt 2760

acagcatata tatatatata tacacacaca cacacacaca catacatata tttatattta 2820

tataaaacat ataaataaaa ctaagtatat atatatatat aaaaatcagg catatagcag 2880

atgtcctgta aacgttagct tttatagctg tcatatgtga tctcaaaaat atagagtgat 2940

acttgaatat gtattataat catacttcat tgaaagtctg agataaggac aaataatcct 3000

ccaaccctcc agtctcccct atgaatgcaa ccaacaccat cttctcagtc tgatgtactt 3060

gcctagtatc tccacaaaac acttcagtgg acatctgcag agtaccttct ctttgcaaac 3120

gttgtgtttc tgctggaggt tataggaatg aataggttgc agtattcact caccaatttt 3180

aaagtctact gggagcaaaa gaacaaaaac tgacagctgt gaaactgaca actatgacaa 3240

ctatggtaag aaagttgaca gatgtatatg tgaaggttcc tgagggtaaa ctaaggagtt 3300

attaatccca atgcagaaga ctggagaagc tacaaagcat catgagtagg ggtctttcca 3360

gtgggtaatg ggctaaagag cattctaggt gaagggaaaa gtgtgagcaa ataaaaagag 3420

gtatgaaaga aatacaggga agagtcctta gtatcatgac acttgatttc aaatcccaac 3480

tctaccctct taaataagca accacatgca atttactcag cctctcagtg cctccatttc 3540

cttacctaca aaacagggta aatattcatc tttttctcaa aggtggctaa aaagattaaa 3600

tgaggaagta tgtgtgagag cccttattgg agcttggcac atagtaggcc atgaaaagca 3660

atttgaatga atgaggcgtg tgtagtagca gaaaatagac tgattagagt gaatttggcc 3720

ccaagaagag tttgagaatc gatacggatt tcttaatgcc taggcaaaga tctctgcttt 3780

tttctgcgag cagccagcat gtattttcac cgaatacttc tgccttggtt tggatgttcg 3840

tcctctccaa acctcgtatt tgatctctaa tgttggaggt ggggcccaat gggaggtgtt 3900

tgggtcatga agatggatcc ctcatgaata gattaatgac ctccttggga gtgagtgaat 3960

tctcactcta ttcatttcga gagagctggt tgattgaaaa gagcctggta cctctcccct 4020

ctcttacatt ctctctcatc ccatgatctc tgtgagtact ggcttctctt catcttctgc 4080

aatgagtgga agcagccaga gcccctcacc agatgcagat acccaatcct gaactttcta 4140

accatccaga atcataagcc aaataaacct tttttcttta tcagttgctt agcctcagat 4200

gttcctttag agtgacacaa agcagactca gacaagttca cagcaaaggc tgtatggttt 4260

ctgcgcagag ttcagagtct agtgaatcag agatatatct cagacattga cccttatctc 4320

ttaggcccag aacatttgga cttaagcctt tgacaagata tttcctaata aattgaaaat 4380

gaggtagaaa aaatatgagt tggatgataa taacacaggt gggtcaattt tcaggtgcat 4440

cttccagctt ttaagcagtg ttctctgtag attttgcctg gtcagagagc taaatgtgag 4500

ctttgtgttc atacttgaat tccaatccct gctcagccac tttataactc tgtggcctta 4560

gaaagccact taatcttggt caactataac ttagggaaaa taacacctca cggaggtgtt 4620

tgaggattaa acgcgggcat atacggtacc tgaactatag tagtaatgat tatcctgttg 4680

agatatgaca tgtttgggcc acaaattgga acccaaatca acaagtgctg acttaatgcc 4740

cccttgccca ccctgggctc cctttaactg tgattattct atgttttcca ggctagagat 4800

catcttctat gagagagaag cctgaattag aataggagct gaccagttct gcctttctcc 4860

atcttttgtg aacccatatg tttagtgacc cttgtgagtc aggcagcatg agtattcttc 4920

ccattggttt tgagtctaag aaaagtgaat ttgactatcc ttggtgtttt tcacaagtct 4980

tggcccattc agtgatttta tgtccttgaa tctctccttc catgccagtc tttgtgttgt 5040

cctcggttat gaccatcacc cttaacatct tgtacctgcc ctttggttcc ttgagggcat 5100

gctacaaata ttcactacac tttccctaga ccagagtttt ttcaaatggt gggtaacaaa 5160

gtcaatttag taaataatta ccaacattta aaaataaata aaacagaatt gaacagaaaa 5220

gttatgaatg tatgacacag attaggatga gtattgtttt gtgaagcttg attcagatgt 5280

gtatgtgtat gtgtgtgcat gtgctgggtg ttcgcaccat ataaaatact tttcctactg 5340

tgagtctctg tctgaaaagt ttgtaagcca ctgctttatc acttcccctt ttatttcctt 5400

attgtcaata tttgcaatta taatgtcaga ttttcatttt gaaaatacct ttccctttct 5460

taggttatgg gacagattcc aactttcctt tgaagcctgc tttctaagtt cacatctatc 5520

aatcaggaaa ccacagtctc ctcacagtcc aagccttcat catctattca ttcatctctg 5580

tatttctagt acctagactg gtggctcttt actctggtta tacgttggga caaactgtgg 5640

cacttttaca aagcagatta ttaagaggca ctccttgcct cttacgtcag atttttttag 5700

acagcgaaaa tgatagccag tggcctgatg gtaggaatgt actcattttt ttctgaatga 5760

ataaatgaaa gatcaatcac ccccaaattc ctatcatttt gtaccacgtc ggtaagaatt 5820

ttatctagcg tgtctgttcc gcctgtagcc catccgcctc ctgtgagttc aagtcatcag 5880

gaggacaagt aaatttctta gcctatctga agccaaataa gatttctagg gatatttgga 5940

taattgtcac tacttgctat gacaatactg tatcactagt tctctaaaaa aaaaaaaaaa 6000

agtttattgt ttaaatctct tggttgtgct aacgtcgtct gtttctttca aatgcagcat 6060

cagtagcttc gctctgctca ggcagccggc tggcacttcc ttctcacatc tgttcatttt 6120

tcctttgtcc tttactcaga cattttctat ggtgtttatg cactcatatc taataagtcg 6180

ctcctgtgta tgtgtgtgag tgcacctgtg tgtgtgaaat gcctttttac atgcaggcct 6240

tgtgtatgat tttctgaagt tacaaatcct ttcaacgtac ctgtattttt caagcactac 6300

atcacaaagc agatgcccag tccaggctgc ggagtcagat tgggcagatg agtaggaggc 6360

tgcccatctg tcagcaactc taaaacatca atggaataaa ctggaaccat gttgccattt 6420

acccggactt tccacctagg agtccttaca cactgatgaa attgctcctg ttcctgccaa 6480

agtacacaga gccctagaga ggatattatg tatctcttat gtgtacctat ccacctctgc 6540

taaataaaaa ctaaatattg tacccagagt tccccacttg tcttgccctg gccttgcctg 6600

gaaaaattgc aggaaaaact ctggagttga aactcaggca aagtacagtg aaggagaact 6660

tttgtggact gcctaccttt gcaacttatt ataatttctg aatagttttg tgtgtgtttt 6720

atttctttct cccccacaga acacgactta atgctctccg agaggtacaa cccgtagaac 6780

ttcctaactg taatttagtt aaaggaatcg gtatgtatgt gagaggaaga agggtctcat 6840

ttgaacttcc ggtatttttt tgctctttgt gtgattaatg tgcctgttca attctgtcca 6900

aaaataaaat tttgaatcag gagagtcagt ggtagttaat gataaatacc atgttgtttg 6960

acttttgact tcaccaacat actgccaagg tgttgggagt tttggttgtt tgtttttgtt 7020

tgatcaatag agcaaaaaaa aaaaaaagaa aaaaagaaaa aagaaaaata gaataatgtt 7080

tgattccatg gcaatctaac ctgaggagat ggcagaagag gggagggaga gccagataaa 7140

gcattgaata aaggtaactt ttttttagaa aggctctgac aggactttgc aattaccaca 7200

aggcttctcc ttatgcatct ctcttttgat attggacagg aaaatttttt ccagaagctc 7260

tcagcagatt tccccttaga tcttatttgt cagagctgga ggacaggctg gtgccctagt 7320

tgcaaaggat gctgggaaag taattatcaa gcctccaatg tgggaggcag gccctgtcag 7380

caaagataaa acggtaggag aatagcagct gggaggcaac caacaaggag agtccccggg 7440

ctcaactctc cagctggttc caaccaacca gctttgttat ttgaagacaa ttggtttgag 7500

atcatgcttt tgtgtgcttt gggtgtttct ttttctttct tttttttttt tttttttttg 7560

ttcttttaga attattcatc tcagttgatt aaggctcagg aggctagatc agatgactta 7620

gtaccactaa atttcactag atatcttatg gtgcgattgt tttctagaaa aatatgatat 7680

tgatatcaaa tatcactggg tttttgaaaa gcaaactttc cactaagtgt agaaatacct 7740

atcaaatagt tcatttttct ctagataaat tgaaatgtta aaaatttaca tgtacttaaa 7800

gtataactaa cttgaagaca gtaatattta accgatgttt ataattagta gtaaaaattc 7860

atattcttga tcacctaaaa tgtatcccca ttttaactca actatttgga atttgcttat 7920

atcttcaata tggctttcca agggtttaat tgatttggaa gtattaagta tcaacatata 7980

tttgtaattt accttgtatc tttagaaaac aagttctttt caaataatca tggcttttgt 8040

acgttttgtg aatttccatg aactttaaga ggattcagtc tttgaggaaa aagctctagt 8100

ccatcaattt aaaacaaata tactaagtgt aaattttaat taaacttgtt aatgaattaa 8160

tatattttta atttccatat aatcgcattc atcaatttga ataaatgaaa gaatgaatta 8220

ttctgaacct attaaagaag agtgatgtat agccccagtt tcaagtgagg tgtgataaag 8280

aaatggatcc acatcctgca ataatatgaa acaacctgta ctttctgttc tcttttctgg 8340

cagaaactgg ctctgaagac ttggagatac tgcctaatgg actggctttc attagctctg 8400

tgagtgtttt ctttcacttt tctgtgttct agaacgtttt ctaggactgg cagtttaagt 8460

ctttcactta ggctttctgt atacccatgc ccactttcaa ataaaatgaa cagtcatggt 8520

ttaaaagaca aattgtgtat gcatttacac ttgtgctata attatgatca tgcgtgttct 8580

atgcatattt gtgttcttcg gtctacattg aaccatatta cgttagcttc tattgcagca 8640

atttttttgt gagttcagtg tattcaagaa ttgttttcct ctcaattgtc tattccctaa 8700

gcacttaaaa ttgccatatt caaatatttg ctaaattaaa tatacattta atgtatacta 8760

cttcacaggt catttcaact ttcacatttt taaacaaatt tcagaataag ccctttaatt 8820

taaaattgtt gataggtccc aattcccaat tcccaagctt tgaagacaga atccaccata 8880

tttgctgaaa ctgtcctgca tatagatcgc tatggtagct gatatagtgc aaaacaaaag 8940

aagtccacca tctggcctag ggatcataac ataacagagt tgataggaaa cgacagacaa 9000

tcatccagcc atttaaaaac ctttgctacc ccttgtaatt tacttagtcc agttttctct 9060

tacaaagatg atagactagt catcctgcaa agcaatgtct tgcaaacttt cttgagcaac 9120

ataaggtgta tttcaagcaa aatcttatat gggaacacaa tatataaaac aacagcttta 9180

ttgttttgat tgaaggctaa gaaaggagta gttcctctgg cccaccaaaa ctctgtcttt 9240

gagatgcttc caaggaccta ccagggaaca gtttaaaagc cataagcaat attctctctg 9300
tattccttgc ttatatcatt gaagactatt attaattgcc tttttggtct gctcttccat 9360
ttttgatctt tctcatgggc tggaagctgt agtgtcgtgc acatatgact ttgctgcagg 9420
ggatgaaagg gtttgtgtgg ggtggcctag tggagcatgt ggcttccaga agacttctcc 9480
tggggaccca ttacagaagc taactaagag gcttcctatg tggccatctg gctagccctg 9540
tggtttggag cagtcaacat gaacccatgg gccatgcagc gcctctatta gcatttgaag 9600
tactggtatt gtgcttgatt tttctcctcc atggtttata agggattaaa gtatcctgga 9660
ataaagagct tcaaccccaa cagtcctgga aaaatacttc tgatggacct gaatgaagaa 9720
gatccaacag tgttggaatt ggggatcact ggaagtaaat ttgatgtatc ttcatttaac 9780
cctcatggga ttagcacatt cacagatgaa ggtaacaata tttactattt attaaaacca 9840
aaaccaaact taaaacaaaa acagaaaaac atgccaataa atgaattatt ttttctcatt 9900
caaacaaagc atagtcatga ataatgttct tactctgggt tttaaactta ccctctatgt 9960
taattgaact ttatagtaaa accacttaaa tacttgagaa tgctaaaata tttaagattt 10020
gtaaagcatc taattttgta aaatactaac attcacatta tccctataat taccataata 10080
ataatatcca gccctgatca ttatagcttt tccattttaa acgattttca agactcgggc 10140
atttacagga acagattttt ctaacccaag gcatctgtaa gctataggtc cagtgacaga 10200
aagtcaccat ggtcagcttt ctgccggtcc cattatcttc aatgcctcct gtccgctggt 10260
tcctctgttc tgtgttcaaa atgctcctct gcatgttggt ccctttcaac ataacttggt 10320
gtcatccttt atgtctttac tccctttctc cctttccatt tgctgtcact tccttgtctc 10380
tgtattaatt ttcagcccca gttgcacaat ggtagcacct gaagagttta aaaagactcg 10440
atgcccagcc cccagcttga cagattccaa ttccgttagt ctgagggagg cctggatcac 10500
agtagttgtt aaaagttttt cagttgataa aaatgtcagg ctagggttaa gaaccacgtt 10560
tatacccatt acctccattt catcatcaga cgatccctca ttcagttcct cttctggact 10620
ttccccactg tgggtggcta aaattgctac cttgagaatc atcagtgatg ttcctactaa 10680
caaatctcct ctcttccctt cctccctctc cttccttccc tcttcccttc cttccttcct 10740
tcacctcctt ttgttttttg ttttgttttg tttttgagat ggagtcttgc tctctcccag 10800
gctggagtgc agtggcgcaa tcttggctca ctgaaaactt cacctcccgg gttcaagcaa 10860
ttctcctgcc tcagcctcct gagtagcggg gaccacaggt gcatgccgcc atgcctggct 10920
gatttttcgt attttagtag agacgggggt ttcaccatgt tgcccaggct ggtctcgaac 10980
tcctgagctc aggcaatcct cccatctcgg cctcccaaag tgctgggatt acagttgtga 11040
gccaccgcgc ccagcctact tcacctgctt ttatgctgtt taccattttc tcattcacaa 11100
aacttgctct tccctgatac tgcagtgcac ctagctgttc cagttctcat cctactacag 11160

gttttctccc caattcattg ttttgtcact cattgctact tccctgagca ctaccccaga 11220

ggtttgatct tttacttcct ctatattttc tctattttag aactttcaaa ccagtgtcac 11280

actgcctgct ttctaggagg acatccccaa ataggggtat tattattctt cttcttttca 11340

ttagcccttc acttttaatg ccgcaggtgc ttactggaca cttctatttg gattcctttc 11400

ctatagacac tttaaactca aaatgttgaa agtgattttc catcctttta tctttccttc 11460

tcttccccat gtgagacaca cacacacaca cacacacaca cacacacaca cacatataca 11520

tatatatccc tttcaatgga ctttccactc atccacccat tcatcaccat tatgttttat 11580

tttaaaaata tttttaagta caaagaaata tagtgtttga tgtgtggata ctttttaaaa 11640

taaaaatgag gtaatattac acaacccctt ctataaattt cttctgcatt aaacactaca 11700

tcatggactc ctttatatat agcacatttg cagttgcttt gccaaggcag ttcgggattc 11760

agttgtgtgg gagcactgta atatagctca tctgctaaca aatatttagg ttgttcccac 11820

ctttatgctc attgcaaaca atgccaaaat aaactttctc gcacatgtat tttctgagac 11880

tatatcctta ggatacattt ctagaagtag aatccactgc acaaaatgtg aattcaggtt 11940

ttaatcctca cttgaatttg atttccaaca ccctccttcc tcagctgtat tcagggcctt 12000

tggattcttc ctgtcaaatg cctctcatga cctttcctgc cttttattgc cagtggcatc 12060

ctcttggtcc agtcctttct ctccacattg ctggattact gctgtagcct cagctcatgt 12120

tactccttgg ttcaaatacc ccagtgagca aagcttggat cttcatctta agccctaaca 12180

agactgattc tggctccctg tggtttcagc cctctcactc tcccactctc tcttgccttc 12240

actctgctcc cagcacaacc tcacaattaa gtgaatatgt ccaggaagtt ctgccccggg 12300

gcctttgcac actcccagca caacctcact attaagtgaa tatgtcccgg aagttctgcc 12360

ccggggcctt tgcacactcc cagcacaacc tcactattaa gtgaatatgt ccaggaagtt 12420

ctgccccagg gcctttgcac actttctccc ctcaacctgg aatgctcttt ccctagatat 12480

ccacattatt cgttctctta tttcacttag gtttttgctc taacatcccc tcatcagaga 12540

gaacatgcct gactcccatg aagataatac ttcgttactc tctattcttt tatcctgttg 12600

tatttttcct ttgtagcatt taacgccaac taaaataaat taaacacttg tttgctgaat 12660

ggggtaatga aaatcatttt agcagctttc tctgttcaaa tcttctcaat tcaacctgcc 12720

aaattttgca tcttaacatg gtgaaacccc atctctacta aaaacacaaa aattagccag 12780

gcatggtggt gggcacctgt aatcccagct acttgggagg ctgaggcagg agaatcgctt 12840

gaacctggga ggcggaggtt gcagtgagcc gagatggcgc cattgcactc cagcctgggc 12900

aacaggagtg aaactccgtc tcaaaaacaa aacaaaacaa aacaaaaacg ccaatttgat 12960

catgtcagag gtttcaaaag acctactctt tcttaaggca aagaccataa tctaactttt 13020

acttgccagc tctgatcaca cattcttgac cgcttagact ggtcaccttt gtgttcttaa 13080

agtcccactt atccaaggaa gctgcttctg tttgtttgct actatttctc agatgttctt 13140

ctatggaatg tatcatctca cactgtgtaa attacacctg atcacacctt ctcatattgg 13200

gagttcccat ggtttttatc cctttcatcg caactgtgca tttcgtggag accatttgaa 13260

ctggaaaact taaaggattc tttggagttt tagtgtcagt gatactcatt cttgagaatt 13320

tttttttctc ctgtggagtt ttgagttttc agagactgtc actggttctt ccttgagcca 13380

cttttgtttg gaacaggagc aatcaccaat gcttctctct acaaagagat gaaaatattc 13440

catctgcctg acatcctaac tactagctgg aggtgggttg aaattggttt ttggagagag 13500

ggctgacagt gagagcttag ttaatgtttc atttttgttt ttaatctcct ctcagataat 13560

gccatgtacc tcctggtggt gaaccatcca gatgccaagt ccacagtgga gttgtttaaa 13620

tttcaagaag aagaaaaatc gcttttgcat ctaaaaacca tcagacataa acttctgcct 13680

aagtactgag atatatcaca tttctatttt cctttagctg tagcggatag ttaatccact 13740

tttagttttt ccttttggcc agagtaggta tggctgttca actctcattt ctaatgatca 13800

caaactcctc cacatgctct tctgaagaac cttctgagga ctcaagcccc tcagagacgg 13860

cccattcttt tcctctcttg tttctcgagc tgattggatg ctggctcctg gcctcaggat 13920

ctccattcaa gttattacca ggaaaaaaat cctcttcttc agaaccagac ttagtatttc 13980

aaacccaaac cggcttcata gataaatttg acatcatgag cttatttatt tatattaaaa 14040

agtagactct tagatgtttc catgaattag aaacacagac ccaagaggag accctgaatc 14100

aagtgcatct aagacctcct tatactgcct ccgctgctgt ttgattgcac aaaatttatg 14160

caatagcaga tctgtggtaa cactcaattc cccctaggca ccttgttcat gtaacatatg 14220

gtgaaataaa acactcaaga atctccaatc tcagtcaact tcctcacatg ccctgtgaag 14280

atttggcttg taaggccagt gatggattta ggagaacaat ccgactctat cttctgaaac 14340

atttacctcc aactgattac gattttctag aatcccaacc tatttttata taatttatgt 14400

acagcatttg actacctcca tatctgtaaa tctagatttg tactctttgc aggtacctag 14460

ctaagggcct ggtatgtagt gtgcactctg tgttaccagg accccttcct cttctttctt 14520

atctggtggt ttgaggatga ggaaaatgag attggagaga aattgagtta ctggatgaag 14580

gccaatcaac tcactagtgg ccaagtagga accccagctc aggtcatctt ctgccccatc 14640

aagatgaata aaagttcaga gaacagcttt tggtggaaga gtttattcgt gcatttattt 14700

aacaaatata ttttggagag agaataaggt cactgagtgg agaatgtgag gaacaccaag 14760

atttaaagta gagaacagta tgtagaatgg agaagctggg caaatgataa cactaataaa 14820

tgagctttct ctgtaagtca ggtgctacac taactaattc aaacagatta ttttattgat 14880

gttttacaaa taccttctga attaggtgtt attattatat ccattttgga tttttaaaaa 14940

gttgaggcct ggactaattg ttaagtttcc ccagatgaca tcatcagtaa atggtgcagc 15000

tgtgattcta atcctggtaa catgagttca gagctggtgc tttaaacctc tacaaaatag 15060

tgaaaaaaat tatgtgtttt aacatttaat cttggaaatc tcagattggt ctacgttacc 15120

ggattggagt atttttttct tgagccagtt gcaaaaatat tggtcttagg cagcattgga 15180

gactgaaaat aaccacggtg ccgtaattaa ctagagttct gctctctcaa tccaaggatt 15240

agctgtgtgt attttcttct ttgtatttta tggtttttta taacattaat attccaataa 15300

ctaaatctat tataatttaa acttgttatt attcatatat cacaactgtt attattggtc 15360

atttactgta tgccaggcat ggtgtgaagg cctttgcatt cattatttca ttgtattctt 15420

actctagcct ttcaaggtat acagagaaaa cagaggttca gaatagtggc tcaaaattta 15480

gtgacaatgc tgtggcccca cattataagg atagcaagca gtgtcaggat gcaaatgaag 15540

tccatcagat ttcaaagcta ttactctatt accctttaaa aatttgttat ttatttactt 15600

attttactac acctgggggt agtaaatttt cagcaataaa aagctattgc tctaaaccat 15660

tccactctgc ccctttctgt gaaaaacaga caaatgatag tccagcatac taatttaaga 15720

aatttacttt taaaaaagag agagtaaaat cagattgtag atttatgtct ggagcccatc 15780

cagactaatc ctaaattccg agaccttttt ttttttttca tgttgtaata gtggctagca 15840

ttgattgagg gtttattgaa tgtcatgctc cctgcaaagc acacaacagg cattgtctca 15900

ttgtaattcc cactgcaact ccacaaatta agatcctgac ccttagtttt ctactaatga 15960

gctgtatgat tttgagaata tcatttaacc tctctttaca gatgagaaaa ctgaggctct 16020

gcatgcctgt ctaacttgcc tgagagctca gagctgcgtc agagctgatg cgctggccat 16080

gacgcctctg caatagagca gagataattt tctcccagat catgccctgg ggaacttcca 16140

tcacctcaat cagcaaagaa tgactcactt atgtgttccc tattgtatcc attaaagcag 16200

aaaaatagac tcatatatct tacattttca gtaacagaga atcctaatca gaaaggaaaa 16260

aaaaaaagcc atagcattct atttctgagc attcttgttc atggacattt cattcagttt 16320

agaatcacga aatcttaagg ctggaagatc aaccagtcta tcatcctgct ttcacagata 16380

agaaaactga ggtcctgaca tgtgaaaaga ttgatttttc ttagatcaca cagctaatag 16440

tagcagagct taaactaaaa tgcaagtatc caggctgctg gttcagcatt ttttcaattt 16500

cttatgggag ttagttctat gtgagcccag atatgtgagc acatttttgc cctgacaatg 16560

atttctggct taatttaatt tctactgaat ttagtgtatt tacacacaga cacacacaga 16620

tacacacaca cacacacaca cacacacaca cacacacgac gtaacactat tttaagatac 16680

atataatagg atttttatgt tacagtgcta taatcacctc ctcagatttc ttagaccaat 16740

gattgtctaa ggattgtatc ggcaggacta atttcatatt aattgtgttc cattatagct 16800

agcacgaagg ctccatccca catcttgatt ttaggaatag acagtgagga atgccagtta 16860

ataatcctgt aatgttcaat accttcacct tatatattat gtgtgtatgt tttaattgca 16920

gtttgaatga tattgttgct gtgggacctg agcactttta tggcacaaat gatcactatt 16980

ttcttgaccc ctacttacaa tcctgggaga tgtatttggg tttagcgtgg tcgtatgttg 17040

tctactatag tccaagtgaa gttcgagtgg tggcagaagg atttgatttt gctaatggaa 17100

tcaacatttc acccgatggc aagtatgtga actctctgaa atgtagtgga tttactcaga 17160

ttctcaggag atatcttgga atatattctt tttttaagta atatgataca ttttaacatg 17220

ttaccctagt gagataaaat taggaaaaat gtaaaatgtc ttaatttttc agggggtgaa 17280

atttatcaaa tcaaaaattt cattgtgaag gcatactggt tttatttcct ttttggaaag 17340

aaagtacatt tttaagaggg gaactgtgat gaagatttga tatgtctgaa tagctttgtt 17400

ttgagtaaag atgtgagtgt ataatgagtt gaatagtatg caggtataga tacgtcagta 17460

gtgcaggttt atgacaagat gaggaatttg taccaaaatg taaatcaagg caatacttcc 17520

ttcatcaaga aagtattact gtgaaaaaca agtcagttta actaagcagt gtgcttagtg 17580

atgtatttga tttataatgt tatctcatgg tgagctataa caaacaattt gcaaaccaac 17640

attggtctgg agactgcatt ttgggtagca ttggtatata catgaaatat ctatatccat 17700

ccacatatat gccctttctc atcttgtgtc atcatcaagg attttagagt ccgtaacata 17760

tgcttagaga aagcaacagg ggccaggtgt ggtggttcat gcctgtaatc ctagcacttc 17820

gggaggccaa ggtgggtgga tcacctgagg tcatagacca gcctggccat catggcgaaa 17880

ccccatctct actaaaaata caaaaattag ccaggcgtgg tggcgcactc ctgtaatccc 17940

agctaccagg gaggccaggc aggagaattg cttgaacctg ggaggtggag gttgcagtga 18000

gctgagattg caccactgca ctccagcgtg ggtgacagag caagactctg ttttttttta 18060

aaaaaaaaaa aaagaaagaa agaaagaaaa gaaaaagcaa gcaagcaagc aagtgagagc 18120

aacagggaca gggacagctt ttaattattc atatcataga ccagttttca aaaaatagaa 18180

gataattgag cagtaatttc ttttttttgt tggggggatg cacagtgctt agactaaccc 18240

cagatctttg tggggtatga ctaaagccac agggtcagga ccacactcag ggcagaagag 18300

gaggaagact tctcttgatc caagccaagc agcttttctt ctctcatatt ttctctggat 18360

taaaacaagg ggctgttgtc caagggcttt cctgcaactt tcccgagaga gaactcacta 18420

gcccctgaaa tgcagaacct gggccttgac actaaattgt caaaccaaaa gagaaacgct 18480

gtaatgcatt ttactcccag atactaaaag ttagtgttag ggctaagttc ctcccatact 18540

gataaggaca actatgtccc ctccttcaga aacaactttt cagagattaa acccctctgt 18600

cactgtaaac tcactgagtt ctttcagaat ttggtgtgtt tgaggtgggt tccattttcc 18660

attccttaag caaagttctt ccctatgaat aggtattaca acttcatctt aatctctcaa 18720

gttgtgttac ttctagtact ttgatgtagc tctttcttct ttgaaggtat gtctatatag 18780

ctgagttgct ggctcataag attcatgtgt atgaaaagca tgctaattgg actttaactc 18840

cattgaaggt aagacgtatt aacactgtaa gtctgcagag tgataattag attctaattg 18900

atttgtgaaa ttaaatgtga agaaaaaagg actgcttaat tggggtgggt gagaaccaat 18960

ttttcactcc tttattaaga gtcattagct tggttatgtg tttgttgttc atttttactc 19020

aactgcagag tggcaaacag caaaccccac ctcttacccg tgaaccttgc attctttctg 19080

ctctagcaaa ttttaagctt ggcattgtgt tcccagatcc tccttccatc cctcccttcc 19140

tgcctttctt ctgatctccc tctcctccac cttccttccc tctcctccct tttctttctc 19200

ttttttcctc tatgttgatg gaacagccaa gaaatagcct caaattgtac tttatgcagg 19260

gctgctctcc acaatgttta tgaagtccac gtgctcacga tggctgctag gtgacctggc 19320

tgcccctcac ctctctgcct ttacctccaa ccattgtgtc cctgccccac gtctcctgcc 19380

actgtctcag aacggggcac cattccctct gctaggagga gaaaccttcc tccagatctc 19440

catgaccccc tccttcatct cctttcattt ttactaaaac gtcacctcga tcttcaaaac 19500

ctggaactgc tccctcccgc tacgtatttt ccctcgcctt tcctgcttta tttttccatt 19560

cagcacgtat tgctaacata ctatatattt tattcatacc ctttattacc tgattcccca 19620

actagggtgt aagctccaca aggacaggct gtttttctat ttgcttctgg tacaattcac 19680

attgactagt tggcacttaa caaatacttg ttaaattact gcggtggggc tgggcatgct 19740

tcagggattc acctgctgcc cccaccaatt ctggtcatct gaaggttgga gtcaaaatgg 19800

gtgaaaacac aaaggagagg aaaaagagac caagagcaaa ctagagccct aagaaagcag 19860

ccctctacct ccgaaaaaca gcaagacgtt gctttcctgc cataaacgcc cactgcagga 19920

agcactgtca gcgccaattg tatgactgtt cctgtcagca catctgagag actgtcatga 19980

ggggcctcac tcagagaaaa ttaccttaaa gcaacatcaa atgtaacatc gtgtttgaca 20040

ttaaaaatca ctggcagggc tagtttggta ttagctcaag taaaatggaa tttcacacgc 20100

tcatactggc taagggaata aaaaacaagc tgcggtttta acaacaggta tttctgtaaa 20160

acaaaagtta aagaatttta tgagaataga acccaatgta acagacattc accacagaat 20220

gtcaaggctc tgctctcacc agggtgagac tgagggcaag gccagtttca gcaacagacc 20280

aggcctgtct cccatcatga aaaccccaaa aaggttctcc atcaattgtc acattattct 20340

ttcttttgaa agaccgtatt aattaatatc agttaattaa tgcccgagag tgccaagtaa 20400

tttgataaac tagctgaggc tattcttaaa tttgtgatga ggtgtggatg ggtttatgtc 20460

caattggata ttgttcagcc ttaaaaagga atgaagttct tggccgggtg cggtggctca 20520

cacctgtaat cccagcactg tgggaggctg aggtgggtgg atcacgaggt caagagttca 20580

agaccagcct ggccaatatg gtgaaacccc gtctctacta aaggtacaaa aaattagcca 20640

gacatggtgg cacatgccta taatcccagc tacctgggag actgaggcag gagaatcact 20700

tgaacctggg aggcggaggt tgcagtgagc tgagatcaca ccattgtact ccagtctggg 20760

caacaagagc aaaacgccat ctaaaaaaaa aaaaaaaaaa aaaaaggaat gaagttctga 20820

tacctgctac aacatggata agccttgaaa aacatgatgc taaatgacat aagccagcaa 20880

caaaaggata aatattgtac gatttactaa taggaagtac ctagaacagg caaattcatg 20940

gagacagaaa atagaataga atggtgattg ctaggaactt gggggagaga agaataggga 21000

gctattgttt aatgggtaca ggatttctat ttggggctga ttaaaagttc tggagaggga 21060

tggtgatgat ggttgcacaa cattgtgaat atacttaatg ccactgagtt ttacacttaa 21120

aaatggttaa aatgtcaaat ttttatatat tttaccacga tagaaataat tctctcctct 21180

ctccaaaaaa agattccagg acttagtcaa tgaattagtc acacttcaat gaacctcaat 21240

gcgcaaaaac ctggaatatt tgttaacaga gaatactctg tttttcccaa agagaataat 21300

ttattatgtt ttcttgacca aggaatgcga atttttcgag ttgcctttgt gggagtgtga 21360

gatgtgatac gcaggggtct tgcatgagtt agcatggaga ggaattattt atttgcaagg 21420

caaagagttc attgctggac tcagaacagg ctccctgcta gactgtcagc acttccatgg 21480

catgagctgt gtctgctggg tacctggtgc tctccaggtg ttgcgttcct tgcagagctc 21540

atgtaggagc tcagtaattg tgtcttgaat gaatgggacc ccaggtttct gtgtccttct 21600

cccactccca ctctcatctc aggtttcatc tgccgtggaa ttcaccaatt ggctgtactg 21660

cttccaaaga taagagcaaa gccctgaaac aaacattttc ctcattcaaa gacattatgg 21720

tatcagagtg gtgtgctgac actgggatgg acaggggttt agggatggag aaagcaagca 21780

tggagccgcc tgtcttgcct gtctctgggg taagaaagac tgatggctct gcatggtggc 21840

gagactgagg ctctgccatg atgcttgagc cctctaatcc ctccttgggc atgtgaggaa 21900

cagctctcat tttgagtagg aggcacaggg aaacgttggt tttctgcagc tgtctgctta 21960

tgccctggct ttttcaatga gtcatgacca gttatttaca ggttctctgg gcactgaggt 22020

atgctggcct ctgaggtaca tggtagatat tatttctcca actgagaatt agaggtaaac 22080

aaatagatgg gatttctccc cagtagaagg cagccaagat gatgaatggt ggaattaaga 22140

atcattggta ttatttatga tccttgtaca aagcctcgac agcaatctgt gcagtcatca 22200

aatgcttgca acttatattt agagaataaa cagcagaaga acggggagtt gaacaacatc 22260

aggaaactaa agaacaaact tttcctcaac tacaaggcaa aaatgagacc ctgggtgatc 22320

atttcttctc tggctgcaca gcctatgatt agccattaaa gagtccacat ttcattgata 22380

ttcttctgag aaagaatcta catttcctgg ttgttttctg aatgcttata aaaatatcat 22440

taccacatgc cattttagaa ggagatgttt tatgatgcac cgaaaacttc tttttttttt 22500

cagttttagg ttcatgcaca gatgttggta caaatgattt tgtctaactt ttaacagtct 22560

ttcttggctt tctgtatctt caccctaccc aattctgccc tggaaactga caaaatacta 22620

gcatagatga aaggtcagag aaattttgtg agtcttattg aaatggggca gaattgtatc 22680

cttggaagca gcagcgtggt gaccaaggac aagaggcaac ttttctctga tatattttgc 22740

tccatcacaa ttgtgtttta ctttatgtct tgttcttttc ttgtttttaa tcttttaagt 22800

cccttgactt taataccctc gtggataaca tatctgtgga tcctgagaca ggagaccttt 22860

gggttggatg ccatcccaat ggcatgaaaa tcttcttcta tgactcagag aatcctcctg 22920

catcagaggt aagttttaaa aatgaaccag ataagtgact ttattcccag ctactgtttt 22980

ttcagttgac aacataattc tcaattcttg gttttttttt tttttttttt ttttttgtga 23040

cagagtatcg ctctgtcgcc caggctggag tgcagtggcg caatcttggc tcactgcaag 23100

ctccgcctcc cgggttcaca ccattctcct gcctcagcct cccaagtagt tgggaccaca 23160

ggcgcatgcc accatgcctg gctaattttt tgtatttttt ttttttttta gtagagacag 23220

ggtttcatcc tgttggccag gatggtcttg atctgctgac cttgtgatcc acccgcctcg 23280

gcctcccaaa gtgccgggat tacaggcgtg agccaccgtg cccggccaat tctcaattct 23340

atgtgcaaat aaaagggggg acatggatag atgatccaaa tgatcagaaa tgaaggcttt 23400

tggaggtgct ttagtcagag tctaggcaag aaacacatgg cacacttaaa ttggataatt 23460

tgaggggagt ttaatcaacg gactatacac aaaatgtgtg gacacagtgt agggtaatca 23520

acagagttat aatgcagtac cccagggcta gcaagggagg gaggaaatga ataccagaag 23580

ccagaacagg tagacggtgg tgggtggaga gggttgcctg acaggaactt ggcctctggt 23640

taagggatcc tcaaagaaca taatgatgcc atactgctat ggtttaatca ttcattacct 23700

gccttcctat tcaactcccc cttcctgtgt ggcggtatac gcacaggtcc atgcaatgga 23760

ccagtcctgg agcacactgg ccagcctctc tagatcttcc tgcaggtggc aagataaata 23820

gctggaatta ttctggctta gaagatctca tcttccacct tagttacagt actcaaaggt 23880

cccatttttt gtccacggtc tttattcagt gaactatttt gggtacaagt aagaatttgc 23940

aacctacttc aatataggta tttgcacatt tactgagttt tcagtatcta ctaaggatac 24000

ttattactta catcatatat atgatatcat cttgacatat ttgtctactg ctgtgtattg 24060

ttaaagctca gttttaagag tccatttcac ctgcccaaca ggaaaaaaaa aagtgttaaa 24120

gcccgtatct cttgggttaa aaaaaaaaga aacaaatgtg tatttccatt tacattatgg 24180

ctctcttacc ttttatctct aggtttgcag gacaattgtg atcatgttta atggcaaccc 24240

tttgtgcctt ttgctagttt ttagctatta gggttgctga atggtatgtt taatagaatt 24300

gaagaaatgt atgctgtgga gtgttcagtg ctcagaactg agcagaatgt tagattctaa 24360

tttgttcttg cttgttagct catttagtaa ccttgggtca tttgctatag catggacttg 24420

gtttgttgtt ctggagaaaa tacttcattt ctaatgattg atctgcaaag gagtagcaaa 24480

aagtagtaac aattccttat ccaatgcact gcccctagat gagtgattta gttttattaa 24540

caagaagctt agctaagcca aggtgtacca tcaactattt ccctgtaact actacatgtt 24600

aatgctatgg caacttccta agacatctgt ctttggtgcc agctgttttt aagaggtcat 24660

tttgaacgtt cccacagacc cttttatatc cacgcagtgg ccttcaccgt ctgccacatt 24720

tgtgatgtag atatcctttc ttcaagtcat tatatattat cataaaggct taataataga 24780

aagctagagc tgaaggggaa tttagagata atctagtccc acccatttca ttctacagat 24840

gagaaacaaa tgtgagtgga agatccaaga catgacccag gtctcccacc tcagtgtagt 24900

gcttttcata ctaagttgaa tcacacgcta ttgctatttt ttacagtcag aatgatcaga 24960

tattggctat ttcatatggt tggcttaaca tgatactaca ctgttattat tgatacaatc 25020

acatttctcc tcaattaagt gaaggaaact gaacttacaa aatcttatac atttagagac 25080

aggaggttaa gttcagaaca catgagtatg gtacacagat atggtttttt gagggccaca 25140

ttttttttct tgtctgcatt ttaaaaacca ggaattaagt gtaaacatcc agttagttcc 25200

ctggcttacc tgaaaataca tgaaaacgga ccttaattac tgcatggcaa cagtttggct 25260

attgctgagt gtattagtca aggttcccta gagggacaga actaatagga tatatatata 25320

tgtaaagggg gtttattaag tattaactta cctgatcaca agatcccaca gtaggcggtc 25380

tgcaagcctg aggagcaagg agagccagtc tgagtctcaa aactgaagaa tttggagtct 25440

gatgtttgag ggcaggaagc atcagcatgg gagaaagatg taggctggga gggtaggcca 25500

gtctctcctc ttcatgtttt tctgcctgct ttatattcgc tggcagctga ttagattgtg 25560

cccacccaga ttaagggtgg gtctgccttt tgcagcccac tgactcaaat gttaatctcc 25620

tttggcaaca cgctcacaga cacccaggat caatactttg catccttcaa tccaatcaag 25680

ttgactctca gtattaacta tcatactgag taacagcttc ctccctccac atttactgaa 25740

atccccactg ctccctaatg tcccacatcc aggatgcgct gctcatttgc atttcctgcc 25800

tcatccctgt aggtctagat actctccacc tctgctaaag atcacttgag atcagtaccc 25860

acctgttccc aaatgaagaa acaggtgtgt taagtgtaat tttggaacaa aataggattt 25920

atctcattgt ataagattat atggcttctt atggaggatg accctattat gcataaggtt 25980

gttttaagtg acttaacaaa aatattaacc caatgttatt tcttccacag gtgcttcgaa 26040

tccagaacat tctaacagaa gaacctaaag tgacacaggt ttatgcagaa aatggcacag 26100

tgttgcaagg cagtacagtt gcctctgtgt acaaaggaaa actgctgatt ggcacagtgt 26160

ttcacaaagc tctttactgt gagctctaac agaccgattt gcacccatgc catagaaact 26220

gaggccatta tttcaaccgc ttgccatatt ccgaggaccc agtgttctta gctgaacaat 26280

gaatgctgac cctaaatgtg gacatcatga agcatcaaag cactgtttaa ctgggagtga 26340

tatgatgtgt agggcttttt tttgagaata cactatcaaa tcagtcttgg aatacttgaa 26400

aacctcattt accataaaaa tccttctcac taaaatggat aaatcagtta tgtcaattgt 26460

cagatattaa ataacagtgt gtgaccccaa aagtacttac cctaaaacat gtgttgcctg 26520

gaagcacatg tgtgtatcgc tgccttgcca tgtcttgttc agaagacaca ggggagcagg 26580

gttagctcac gtgtctttag aactccagta ctcacccagg gactccagtt cacaggccag 26640

aaaacatatg cattatgaag ttcccctcta ctccatgcac atagtaagtc tgactatggc 26700

agtcagactt acttactccc attttccctt cgatatatga ctttttctca gtaaatatta 26760

acctgaatta ttccaactcc ccttgtactc ttgctttttc aattctcctg ttgcaatgac 26820

acataggaaa atcttaaaat tcttgggagt gttgtcacac ctgaaaatta tgagtctcta 26880

tgatcttggc acaaattgta catttgagtg tctttgactt ggttaaagga agtttgttca 26940

cttcgatgac tggatacaga atgaatccca taattgacat gggcgaagct aaaagtgtcc 27000

ccaaagacta cactgttgtt gaggtggtgg tagtgctggt gggtttttgt ttaatattta 27060

aacttcttgt tgtggaggct gaaaagaaaa aaaataatag aaaggtaaac aaacaaataa 27120

atagaaaaga tcaacaaccc ctttggctat ctactgagac atgactagga agaaaacatg 27180

actttatcat tttgttatag aaactgatat ataaaggtta cacattttca tttatttgtt 27240

tttctgattt gaaggtataa ccttcatgat gaattacttc ttcagggtgt taaggcagtg 27300

actttagaaa caaatttttt tcttgttttt gttttgtttt tgagaccgaa tctcactctg 27360

ttgcccaggc tggagtgcag tggtgcgatc ttggctcact gcaacttcta cctccgaggt 27420

tcaagagatt cttgtgcctc agcctcccgg atagctggga ctacaggcac acaccaccat 27480

gcccggctaa tttttgtatt tttagtagag acggggtttc accatgttgg ccagcctggt 27540

ctcgaacacc tgacctcagg tgatccaccc gccttggcct tccaaagtgc tgggattaca 27600

ggtgcgagcc accacaccca gccagaaacg aagtttttct aaggcacttg ttatgatgat 27660

agtctggcat ttccgggaaa atgttttgcc aataaattga gaaccaccaa cagttgttta 27720

agaatcactg acttgaagta ataaacagga acattttgtt tgggtcaaaa aaccacttta 27780

ttcacagcaa gattggagtt actcatgatt ccagtgacat aatatcccgt gccctacatg 27840

ggctcaattt ttctgagatg ctgctcacag cccaggatgc tgggttctct gaaagactgt 27900

cttgaaatta tcaatgagtg tgatgcttag ggaacacccc caacatattt cattatttag 27960

aaagaaagaa tttcctgagt agttaggctc cctaacatct tcaataaaat gaccacattc 28020

tgttctttct tgttcttgat gatagataag caaattc 28057

<210> 6

<211>  355
<212>  PRT
<213>  Artificial sequence

<220>
<223>  Mutated PON1 protein (amino acid change L55->M)

<400>  6

Met Ala Lys Leu Ile Ala Leu Thr Leu Leu Gly Met Gly Leu Ala Leu
1               5                   10                  15

Phe Arg Asn His Gln Ser Ser Tyr Gln Thr Arg Leu Asn Ala Leu Arg
            20                  25                  30

Glu Val Gln Pro Val Glu Leu Pro Asn Cys Asn Leu Val Lys Gly Ile
        35                  40                  45

Glu Thr Gly Ser Glu Asp Met Glu Ile Leu Pro Asn Gly Leu Ala Phe
    50                  55                  60

Ile Ser Ser Gly Leu Lys Tyr Pro Gly Ile Lys Ser Phe Asn Pro Asn
65                  70                  75                  80

Ser Pro Gly Lys Ile Leu Leu Met Asp Leu Asn Glu Glu Asp Pro Thr
                85                  90                  95

Val Leu Glu Leu Gly Ile Thr Gly Ser Lys Phe Asp Val Ser Ser Phe
            100                 105                 110

Asn Pro His Gly Ile Ser Thr Phe Thr Asp Glu Asp Asn Ala Met Tyr
        115                 120                 125

Leu Leu Val Val Asn His Pro Asp Ala Lys Ser Thr Val Glu Leu Phe
    130                 135                 140

Lys Phe Gln Glu Glu Glu Lys Ser Leu Leu His Leu Lys Thr Ile Arg
145                 150                 155                 160

His Lys Leu Leu Pro Asn Leu Asn Asp Ile Val Ala Val Gly Pro Glu
                165                 170                 175

His Phe Tyr Gly Thr Asn Asp His Tyr Phe Leu Asp Pro Tyr Leu Gln
            180                 185                 190

Ser Trp Glu Met Tyr Leu Gly Leu Ala Trp Ser Tyr Val Val Tyr Tyr
        195                 200                 205

Ser Pro Ser Glu Val Arg Val Val Ala Glu Gly Phe Asp Phe Ala Asn
    210                 215                 220

Gly Ile Asn Ile Ser Pro Asp Gly Lys Tyr Val Tyr Ile Ala Glu Leu
225                 230                 235                 240

Leu Ala His Lys Ile His Val Tyr Glu Lys His Ala Asn Trp Thr Leu
                245                 250                 255

Thr Pro Leu Lys Ser Leu Asp Phe Asn Thr Leu Val Asp Asn Ile Ser
            260                 265                 270

Val Asp Pro Glu Thr Gly Asp Leu Trp Val Gly Cys His Pro Asn Gly
        275                 280                 285

Met Lys Ile Phe Phe Tyr Asp Ser Glu Asn Pro Pro Ala Ser Glu Val
    290                 295                 300

Leu Arg Ile Gln Asn Ile Leu Thr Glu Glu Pro Lys Val Thr Gln Val
305                 310                 315                 320

Tyr Ala Glu Asn Gly Thr Val Leu Gln Gly Ser Thr Val Ala Ser Val
                325                 330                 335

Tyr Lys Gly Lys Leu Leu Ile Gly Thr Val Phe His Lys Ala Leu Tyr
            340                 345                 350

Cys Glu Leu
        355

<210>  7
<211>  19489
<212>  DNA
<213>  Artificial sequence

<220>
<223>  Mutated prg4 sequence; mutated base: chr1:186304862 (C->T);
       mutated codon: Cgg->Tgg

<400>  7
taaaggaagg atctaaggaa aatatcaata cattttttaa gcagattttt aaaataataa       60

aggaagctag aaaatccagt gtcaataatc tacctgtagg taactgagag catgttttct      120

gtgctcagag aaaatgaaac tactcaagaa atgtatattt gatataatgg tataagtata      180

taaaatgtat gccacaagct aacactaggt aaatttcaaa gctacctttg ctagatttaa      240

ctggaaagca gaagaaataa atgataccag tatgtgaata ttttaaagtt gctgcttcaa      300

tatactagtc aaagtctgcc agagctattt accatctcag gacaatttgt tcacttcact      360

tagaaattag aaatgtgctc tggccaatac atctaaattt attttgtggt agtcattttg      420

cttaatggaa gtgttttcca gatgttgcct aagtctagtc gtctgggccc ttttccaata      480

aatgaggaaa gtttgatttc ataggttgtc actgttgatt ttgtctaacc tttggactaa 540

ttggttcatc ccactgtatt tgcactgata tataagactc ccaggacgtg ggataaactc 600

atctatcctt tacgggtaag tgtcaaagtt aacttgcttc atagaattaa atgtgttttc 660

attagaggtg tttgaaaaaa aatgtgtaaa gatagtagtt ggaaattttt gaaaaggatt 720

atgtttatgc aatacacctg ttggaagcct ttgaatttat attgagaatt aagaaaaagt 780

tggaacagta actccatgat gcttattaaa ttacatttct gtgacacagg ttattatttt 840

ccttaagata aaattttaac cttgcactgt taagtacatg ccatactttg gctagagttt 900

taagataagt ctattctata ttggagattt cttaagaatt aaaactgtca ataacgtggc 960

tgagggtgac ttgatttgtt ctgcataaga ttaagtctaa tggccaaata ttttctataa 1020

aattaaagct gatttaaagt cttgtttcaa gaatgggatg gttttattat tgttacattt 1080

ttaaagttac tgaaatgtgt ataatgcaag cctaagttag tggtgagatg aaagagctgt 1140

tttctgatac ttttatttta ttttcagcaa gggtacctac ggtacctgaa aacaacgatg 1200

gcatggaaaa cacttcccat ttacctgttg ttgctgctgt ctgttttcgt gattcagcaa 1260

gtttcatctc aaggtagctt aaccatcgaa catactttta tttaacaact attgctaatc 1320

attcagtctt gatttttata acaacggaaa tatatttcta aaaatttata tttgcttgag 1380

tttaatatac tgtacaacct taaaataata tataacatgt agcctgtttg taagtgcttt 1440

ggaattctca tctttttctc cttttgagag ttttgagctt gtataaagaa taattttggt 1500

acttgttatt tctatggttc tctcgaaagt ttaaaaagtg cagacagttc atcataaaat 1560

tatgctggag ctatatagta tcataaaaat aactaaaaaa gaacaatatt tagataacat 1620

gtaatttttg cctatatgtt gaacaatttg ttttttaaaa tcaaataaat tattgataaa 1680

taacttattt tggcatcatt tagacataga taccatatta taataaacta tagtgtttca 1740

aatggcattt tattagtctt gaaaatgttc tctttttctt acaagaacta tttatccagg 1800

tatggacata tacagatgtg actgtcattt tgtgttataa cattaaactt ttcacagttc 1860

tcccaaaaca ggtcctggat gaatagttct tgttactcat ttttagagac tgttaaagta 1920

cattcagtga aatcacatta gtaaaagatg ctagcatgcc atctaggggg attaatagta 1980

acaatgccaa gctttggatt tttcttttct tttcttttcc ttttgttccc ctttgataag 2040

caaaatctga gagaataaaa atcaagattc atgacagtta tgatgaaatt atgtttctaa 2100

agtaaacatt tccagtaaaa tacgagattc ttatgaaacc tgaacatgag tggtaactgt 2160

ctgcataggc ataagttgca gaattgttta gataagaaaa gacaggaaaa cacatgaaag 2220

caaatgtgaa tattcaataa gaatgatgac tactccagta tctccagacc cttcggcttt 2280

ctcgtaacac tatgacaagg ttcacaacac tgggggcact ttctaaactg ccttttcctc 2340

tgtgatacaa ttggttgttc actaaaacag tgttactttc attttaattg tgattaaata 2400

aatcaaatta aaattaatgg ggctgggtgt ggtggctcac acctgtaatc ccagcattct 2460

gggaggccta gggagacaga tcacctgagg tctggagttc aagagcagcc tgggcaacat 2520

ggcaaaatct tgtctctata caaaatacaa aaaaattagc caggtgtggt agtgtgtgcc 2580

tgtaatccca gctattcgga ggctgaggca agaggatatc ttgaatccag gaggtggagg 2640

ttgcattaag ctgagcttgc atcatggcac tccagcctgg gcaacagagt gagactctgt 2700

ctcaaaaata aataaataaa taaataaaat taatggttac aattaatagc aataaaagta 2760

ggacagacac ctaatctatg aaagtaagct tttcctgtaa ggatgatttc ctcttttttt 2820

tttttctttt gagacagggt cttgctttgt ctcccagtct ggagtgcagt ggcacaatct 2880

tggctcactg caacctccac ctacggggtt caagcgattc tcctgtctca gcctcccgag 2940

tagctgggat tacaggcccc tgccaccatg cccggttaat ttttgtattt ttagtaaaga 3000

caggttttca ccatgttggc caggctggtc tcacctgacc tcaggtgatc cgcccgcctc 3060

agcctcccaa agtgctggaa ttacagatga tttcttattt cagaaatctg ccaactataa 3120

aagagcaatc tcttgatact gtcttgtctg cttctcttgc tttctcaacc tcttctcatt 3180

ctcttttttc tttatatata atatatgtat ttatatacat acactatata tacatttttg 3240

tatgcattat gcactcatgt acgcaaaaag ttctgaaagt tgtcctacaa tttactgttt 3300

tatttgcata ttcagacttt ggcattcctg gactctattc ttttaagatt tgttttcagt 3360

gtgtttcaac attcctttgt ggatttagga cagtacacct gccaatttct attccaggga 3420

tggattccat tgtcacattt ctgcagtcat ttctcaggga gggttttaag gtggtgtttt 3480

ccaaatgact tttaaaaaat atttgagaat gagtatgctt ttgttattgt ttgcttattt 3540

gttgcctgga aaacctctgt tcatgacctt tatagttatg cccagttatt ttaagggttc 3600

tcacttcagc agtggttgta agcatctgcc tcccctgaca ttttaaaaat ccagttatcc 3660

ccaccaatta aggaagaaaa agctcagtaa agtttatgct ggtgcaacca gcgtgcttta 3720

aacttatcct caggaatggg aggcaaagcg acaggtggaa acatctcagg cttaaaataa 3780

gatatatcag agttcaaatt ctggatggat tgtttactta aggtgaccat aaaatgtatt 3840

atctaaatta ggacaattat aagggtaaaa gagttcacta caaataattg ggaggtatta 3900

attaacctat gatgccaata tactggaact attaactagt cgtgtgataa tgagcaagtt 3960

gtcaaaattt ctgtgtttca gctttcttag ctctaaaaaa gggctactaa atcctggaaa 4020

cattttcata agcattagtg ataatgtatg actcagcacc tggcatagag aagctcaata 4080

aatggcaact gctaatcatc aaaaccacag atcggtagta gctgtagctg ccacctcttc 4140

cttaggagta tcaccctctt taggcaaagc cattactttg cctggttttc aaaaatgtgt 4200

ttatcatctc agtctagtga agaataaagt gacataattg agatcaccta agacatcagc 4260

caaatatcag ctgggcctat tgctgacatc attccaacac cttctcgatc aataaaattc 4320

tctctcacca agtggctttg tccccctcgt tagattgctc cctttctata aagtggtttg 4380

gccatattta cgccagtatt gtataatttt agatttatca agctgtgcag ggagatgtgg 4440

ggaagggtat tctagagatg ccacctgcaa ctgtgattat aactgtcaac actacatgga 4500

gtgctgccct gatttcaaga gagtctgcac tgcgggtaag tcctgagagc gggtgtctcc 4560

tctgtcaagc aacactgcga gtctgtgagt ccccccttgc accctcgtgc agtgctgtga 4620

gactgagcct ccccttgcac ccacttgcag tgctgttttc ccactgtatc acttcctttg 4680

cttaagtgac tttccttcac ttgcataaat gttgcagcgc attgtaccta aggatggtgt 4740

caaagcttgt gattattggg gagggtaagg ggagcctgga agtctgtcac ttgcacaagg 4800

ttattgtcta gttccaagta aatgtcttat tttaaataga gaagatactg tgagtcagga 4860

gtcattaagt aaatagtaag gagtaattat ttacaatcag agatgtcctc gactagtaat 4920

gaggactttc aatagtttca tcaaaagaat acatgagcat ggaataatct tgtctaccat 4980

tggactttct gatgttaaca cgtttgtaaa ttacctggaa aagacacttt aaagataaag 5040

tgctagtgtt taatgatgaa tttcagttaa aatctgaagg tctgaatttt aaattatgag 5100

tggtgtttca cttgaatata ttttgggcca ttttattatt aacacagaaa catttcctat 5160

tcaatttaag aaaattgtaa ttaaagttat ttgaaaggtg tatttgcttt agaatttgaa 5220

tgtgaagttg aggaatatat tgtgcaattt atgtattgga taggtaatta tttcaaataa 5280

gccttggtaa gtccctgtta actctaacaa aggcttttta aattttcatt ttttaaatga 5340

gtagtcctcc ctaacatagt ccacactgta agattaggct gaaagctttc aactatacac 5400

cttccccagt ttgtcataat aagggcctct gcattacaaa tgatttttaa gcctcaaaaa 5460

tgacccattt acgtggaata tatatacata tatatttata tggaatatat tgctgagatc 5520

tgtacattca attactgtga atctattaca aagcagtgtg tgaagaggag aaggatgaag 5580

agatttcata tgaaggctat ctcactatct agacatttcc cgatttttct ttgtccatac 5640

atgtaaataa ctcgggcagc atcaggatgt ctcttggagt ctggaagggc aagaggagtt 5700

gccctcagtc accatatttc ttttttgact tgggctgtct ccatctggga taccatctaa 5760

tttttcctgg atgatgtact ccaaatttca aataaaagac ttagaaatga acttttggaa 5820

acctagtcaa gtctaaggtg ggaaatggct gtcaaatacg tgggcctggc ttcacaatga 5880

ataatctgta acttcttgtt ttgctctggg tagagctttc ctgtaaaggc cgctgctttg 5940

agtccttcga gagagggagg gagtgtgact gcgacgccca atgtaagaag tatgacaagt 6000

gctgtcccga ttatgagagt ttctgtgcag aaggtaagca tcacagtacc aaccaatgct 6060

tctcagtaca gccagatctg tgcacctacc ttagcatcac tgataatgtc taacacgcag 6120

tccatcttag gccttgattt tactaacata taagctattt cataataatt ttgttttgtg 6180

gagatgaatt aaagaagaaa agcgccttcc aacagcagaa cccccaggct cagccaggct 6240

ccaagagtac aagatgttgc cagaggggga acatagagga tgaggctgaa tgtatatgtc 6300

ttttttttct tcagatatgg ttcaatttca ggttaaatca acagtaatca aatcacttga 6360

tgatttcttg tctcaccctt gctgacttca cgaagtcaac tatcctttga gctatctgtg 6420

cttttggcct cagtgtttga gtatatattt aaggacataa gcaacacttt cagcttccct 6480

aaaccagtaa atttatgaat tagtttaagc cattaaattt ataggcaaat ctacctaaca 6540

cgttgttaac tgtcatgttc cttcccattt ctgcagtctc ctcagcagtc tctattccaa 6600

tacatgtgca tggtaaagtc aataacaagg gatattccca tcttaaaatc aaattctcta 6660

tttataggtc ttgaaggact cctctagagt ataacaaaag gctccagatt ttacagaaaa 6720

agattttaaa aatatacttg aatactggat tactttcctt taccaaatgg ttgttcttaa 6780

attctacttt gttaaaacaa gaatatttgt ggaggtaaaa gtgaaaataa gctttgcttg 6840

cataatcaat cttatcattt tatttaaaca agcctgcgct tcctgcttct ttagaacagt 6900

gaagaaagat cggaaaactg ctaatcagca gaattgagag atgtttctta accgttcaat 6960

gttttcatgg gaaaacattc caaaaatgat gaagacaaca cccttgtaat aaaaacctta 7020

gagctcaccc ttcctgcaga ttttagcagg ccccatgatg agtgacacat atttactcat 7080

taccctaatt gttttggctg tgagacaaga aagctaagaa tttgttttgg gcagaggatg 7140

tctagggagt gaagatggtt agaaagtgat gagacattga gagaacattt tgataaagat 7200

aatactttat gttatgaaat atttggtcag attacctaaa gtttaggtag aactctgaaa 7260

aaaaaatgat gaaggcgatg ctggccaact ttctttacag aattcataag acagtcagcg 7320

tcccaactta aaataataaa accactcaga acctcaatac ttcatttttt aagtgtaata 7380

accaaactat cttgagattc actacttagt tgtttcagta tagatactac ttacaggtgt 7440

ctatatatct aaaaatcata acatttcaag aattgaagga atcccagcca tccaagtgcc 7500

tctgaatgtg aaagagaaaa cagagtccaa gaggttaacc attttggcca agaacattag 7560

ctagtacatg gcagatctgt ttctcaaatt gagatacttg atgtttttgc tgggtaggag 7620

gagaaacaag agggacagtg gccagttcac ggagactata gcagtagggt tctggggcga 7680

tgttgcgggg agcagcatga tttatcacaa cccttctgta cccagagagt ccccagtgct 7740

gttttcttat ccttcattag tccctcttct tgttcctaaa aaaaaaaaaa aaaaagaagt 7800

tccatgtttt ctcttctaga ctgagaagcg gaaaagggaa ggggactaaa aggttaaaaa 7860

gatacacacc aaaccattaa gttgtatctt tgggaagggc agggcatttt gaggaaaagc 7920

acaatgtgaa ggtgtgaact tttcacaaga atattcatgt agaacttttg taattaaaaa 7980

aagaacaaaa ggcaaattgg catcatttaa tttaaatgaa tattaaaaca gaaaaaaatg 8040

aatactaaaa cagaaaaaaa ttctgaaggc aaagaccttc agaaatcatc ttgaataaac 8100

atcttagttt gtagacaaga agatgaagtt caagataatt atttgcctaa ggtcacaatg 8160

gtagaggctc gcaggcctag gactggaatt taggtcttct gattcctgag actttgctcc 8220

ctgtcataac ttacaacaat ttgagaacca gtattttata gcaatgcatc cttagactta 8280

aatggagtca ttcgtgttga gctggagcct gcctgcactg gctgtcacca gcatctactc 8340

ttgaagctag atgcatctgt gcttttcaca gttagagctg ctgatgaaca taaacaagat 8400

gttaactgac ttgtcttact tggcctcagt gcataatccc acatcaccac catcttcaaa 8460

gaaagcacct ccaccttcag gagcatctca aaccatcaaa tcaacaacca aacgttcacc 8520

caaaccacca aacaagaaga agactaagaa agttatagaa tcagaggaaa taacagaagg 8580

taggaagatg acagatataa tcaaaggagc tttcttagat gaagtaactt gtaggtgact 8640

gcttatctaa gcccattctc agagaacagg gtaatcttag gaatcatgag cctcattaca 8700

ctcgaaggtt ttagactttg cttttaagta aagtttaaga caaagtataa actctcagct 8760

ctttctgtat ttaccaaacc cagcatgagt ctgggttaaa acaaatcaga agggacaaat 8820

cttactataa aaaacaaaaa ccaccccatg attttctttg tagaataatt tgattctgtg 8880

ttttggagga tatgggaaag ttgagagata ctagtaatac tgctagtatc tgtgataagc 8940

ccaggtgcct tgcttttaac tgacagatta aaaggcagtt ggtcatatta ctaataaaag 9000

caaaatccag atacttgtag actagtaaat agcacttctt gctgtgttta gactggtggt 9060

tcttttttgt tttaaatcac agttggtgtg atcaaacttt ctatttgatt tatagaacat 9120

tctgtttctg aaaatcaaga gtcctcctcc tcctcctcct cttcctcttc ttcttcaaca 9180

atttggaaaa tcaagtcttc caaaaattca gctgctaata gagaattaca gaagaaactc 9240

aaaggtttga gcattgataa agatcaaaga ctgtatcaat gccatattaa caatgattat 9300

atttaaaagt aatgcgtgtt ggcagaatga ggacatctta atatgggggg gaatataact 9360

ttatgaatat tatcttagaa agtaccaaat gaatacttcc tacaggaaat tagtgccaag 9420

cggctaagaa gccactctaa aagcaaagga taagaagatg ctgtagcagg actgtggaac 9480

cacaagatgg caccagagat gattattgat ggctttctac tgtcattctt gtttacttga 9540

catcattttg cttcttctcc ttcatcttcc ttgcattttc attttccctt ccccagcttt 9600

attttaacaa acatttatat actattgttc taagtgtttt acaagtatta actcattaaa 9660

ttcttacaaa cctctatgga aaaaatatta tccatatttt acaaatcctc tttattttcc 9720

tagtaactta tatattatat ctggctgact tttcctctag tagagactgt gctacagaat 9780

aggctattat tgcctgaaaa gagatttcct ttgctgttta ttatttcctg aagttctggt 9840

tctaaatata tattctcttt ctccaggaaa tactggattt ttggataagg gagttttaaa 9900

gaactctaag aggttgtgat ttcctccgaa gcattcgctc taaataaata gccaggcatt 9960

taatctttta gtcacccaga aaatcgtgca ggaagccacc cttgggcttt gtagtccaca 10020

tgtggggcag ctgctatggg aaagccttga taaatactgt ctttttgctg cccatcacac 10080

cctaagctcc tccttatgtc ccctgggacc tgcaaggtta gctgcgcaga agaaaaggag 10140

tggctaatag ccctatgtat gggaacaagg ctcagcaggg actgtggggg tgactggggc 10200

agattgctca gaggtagctg tagaggcctc tgagataatt ggtgcagcaa gatggtagta 10260

ctaggtttcc aggaggaagg aagggacact gggcactgaa gaataagagt acaaaaagta 10320

gggggtaaag aacattaata taaacagatg aattagtgtt atttatcgtg ttggtaattt 10380

tgaacttgcc actgacttcc atgtattaat tgtatagagt tttatttgtg ctttttcatc 10440

cctcatctca tttattccct aaaacagccc aataagatag gttgagtatt tgccatttta 10500

caacaaatta aaggacatag tagagattag caatttgctt agaaacacac aactagtcaa 10560

tgataaagat gggatcttta tgtcactatt aaagaaaaaa aaatattcta aaataacaag 10620

atgtatattt tttctccagt aaaagataac aagaagaaca gaactaaaaa gaaacctacc 10680

cccaaaccac cagttgtaga tgaagctgga agtggattgg acaatggtga cttcaaggtc 10740

acaactcctg acacgtctac cacccaacac aataaagtca gcacatctcc caagatcaca 10800

acagcaaaac caataaatcc cagacccagt cttccaccta attctgatac atctaaagag 10860

acgtctttga cagtgaataa agagacaaca gttgaaacta aagaaactac tacaacaaat 10920

aaacagactt caactgatgg aaaagagaag actacttccg ctaaagagac acaaagtata 10980

gagaaaacat ctgctaaaga tttagcaccc acatctaaag tgctggctaa acctacaccc 11040

aaagctgaaa ctacaaccaa aggccctgct ctcaccactc ccaaggagcc cacgcccacc 11100

actcccaagg agcctgcatc taccacaccc aaagagccca cacctaccac catcaagtct 11160

gcacccacca cccccaagga gcctgcaccc accaccacca agtctgcacc caccactccc 11220

aaggagcctg cacccaccac caccaaggag cctgcaccca ccactcccaa ggagcctgca 11280

cccaccacca ccaaggagcc tgcacccacc accaccaagt ctgcacccac cactcccaag 11340

gagcctgcac ccaccacccc caagaagcct gccccaacta cccccaagga gcctgcaccc 11400

accactccca aggagcctac acccaccact cccaaggagc ctgcacccac caccaaggag 11460

cctgcaccca ccactcccaa agagcctgca cccactgccc ccaagaagcc tgccccaact 11520

acccccaagg agcctgcacc caccactccc aaggagcctg cacccaccac caccaaggag 11580

ccttcaccca ccactcccaa ggagcctgca cccaccacca ccaagtctgc acccaccact 11640

accaaggagc ctgcacccac cactaccaag tctgcaccca ccactcccaa ggagccttca 11700

cccaccacca ccaaggagcc tgcacccacc actcccaagg agcctgcacc caccaccccc 11760

aagaagcctg ccccaactac ccccaaggag cctgcaccca ccactcccaa ggaacctgca 11820

cccaccacca ccaagaagcc tgcacccacc actcccaaag agcctgcccc aactaccccc 11880

aaggagactg cacccaccac ccccaagaag ctcacgccca ccacccccga gaagctcgca 11940

cccaccaccc ctgagaagcc cgcacccacc acccctgagg agctcgcacc caccacccct 12000

gaggagccca cacccaccac ccctgaggag cctgctccca ccactcccaa ggcagcggct 12060

cccaacaccc ctaaggagcc tgctccaact acccctaagg agcctgctcc aactacccct 12120

aaggagcctg ctccaactac ccctaaggag actgctccaa ctacccctaa agggactgct 12180

ccaactaccc tcaaggaacc tgcacccact actcccaaga agcctgcccc caaggagctt 12240

gcacccacca ccaccaagga gcccacatcc accacctctg acaagcccgc tccaactacc 12300

cctaagggga ctgctccaac tacccctaag gagcctgctc caactacccc taaggagcct 12360

gctccaacta cccctaaggg gactgctcca actaccctca aggaacctgc acccactact 12420

cccaagaagc ctgcccccaa ggagcttgca cccaccacca ccaaggggcc cacatccacc 12480

acctctgaca agcctgctcc aactacacct aaggagactg ctccaactac ccccaaggag 12540

cctgcaccca ctacccccaa gaagcctgct ccaactactc ctgagacacc tcctccaacc 12600

acttcagagg tctctactcc aactaccacc aaggagccta ccactatcca caaaagccct 12660

gatgaatcaa ctcctgagct ttctgcagaa cccacaccaa aagctcttga aaacagtccc 12720

aaggaacctg gtgtacctac aactaagact cctgcagcga ctaaacctga aatgactaca 12780

acagctaaag acaagacaac agaaagagac ttacgtacta cacctgaaac tacaactgct 12840

gcacctaaga tgacaaaaga gacagcaact acaacagaaa aaactaccga atccaaaata 12900

acagctacaa ccacacaagt aacatctacc acaactcaag ataccacacc attcaaaatt 12960

actactctta aaacaactac tcttgcaccc aaagtaacta caacaaaaaa gacaattact 13020

accactgaga ttatgaacaa acctgaagaa acagctaaac caaaagacag agctactaat 13080

tctaaagcga caactcctaa acctcaaaag ccaaccaaag cacccaaaaa acccacttct 13140

accaaaaagc caaaaacaat gcctagagtg agaaaaccaa agacgacacc aactccccgc 13200

aagatgacat caacaatgcc agaattgaac cctacctcaa gaatagcaga agccatgctc 13260

caaaccacca ccagacctaa ccaaactcca aactccaaac tagttgaagt aaatccaaag 13320

agtgaagatg caggtggtgc tgaaggagaa acacctcata tgcttctcag gccccatgtg 13380

ttcatgcctg aagttactcc cgacatggat tacttaccga gagtacccaa tcaaggcatt 13440

atcatcaatc ccatgctttc cggtattaag aatcagttat tttcattttt aaagctggta 13500

atgttagttt acatctatct gaaccagaat gccttagttt agtatcactc aactgagata 13560

tattacctga ccttgttagc ttgtgaagtt ttacagcttc cctgtaggta agcagaggtg 13620

taattacagt aagctttttc attctctaga aaattaagcc tgggaattta gcatatgtgt 13680

gtctgtgagc agcagagatg gtatatagaa agtcaaaaaa atttagttcg gagtctattc 13740

ttaaaagtac aatgcaaagc aaaaatctgt agtatcattt tccaaactaa cccaaaggga 13800

gggcaatcag aactgaaaga tgcaatttca cattgagttc atacatccaa aaaatgtcta 13860

gtttacatga aaatcaaggt aacctggtcc attccaattc ataataattt atttcatata 13920

atacttttga agagtatcat ctttaagtaa aatggaatta tcaagtatat aactatgcaa 13980

acaggtcaaa ctgtgtcctt caagatctta gaaaaggtaa acaggaaata gaaccatgtg 14040

gaaagacttg aaaagaacat acagactttt ctcattctgt tctatatttg ttttgttttt 14100

gttaatttgt ttagatgaga ccaatatatg caatggtaag ccagtagatg gactgactac 14160

tttgcgcaat gggacattag ttgcattccg aggtgagcta tgtacacatt tatttttctt 14220

ctcatcattc tgttttggca tttatgagaa ccaaagccgt ggaagagtgc tagtttgggt 14280

tgttttctgg aggggaatct gattgataag cacacctcac aggagaatcc gagagtaata 14340

acagaaatat tggattaaga aaattagaca aagtaacaaa acaaaaccaa gaccctgaac 14400

tgatactact gaggtttttt cctactgtta agatttaaaa agtagttcat ttttttgggg 14460

gggggggggt agttaaaatt taaaaaataa aataaaattt taatttcata ccaaatgaaa 14520

tgacaccagc atgcatattc actgctttat ttgaaaataa catctcaatt gtgatgtgtt 14580

caagtgttac actctctgtc ggagagtaac agaagaaaag ttatttcatg ttaattctct 14640

gctgtactct gctagtccag tcttgaaaca tatttagatt aaatctgaaa gcaattcctt 14700

ttcgtacagt agaggctaaa gagatgttag tcattaaatt ataattcctt agttctaaga 14760

gataaagaaa ttgagatcta aagagatagt gactgtaagt cacaaagcca aaggcttttt 14820

ttgagaccgg atctccctct gtcacccagg ctggagtaga gtggtgcaat tacggctcaa 14880

tgcaggctcg atctcctggg cccaagtgat cctcccatct cagccacctg agtagctggg 14940

actacaggtg tgcaccacca tgcctggcta atttttgtat ttttttttgt attttttttt 15000

ttttgtagag acagggtttt gtcatggtgt ccacgctggt ctcaaactcc tgggcccaag 15060

ccatctaccc gccttaacct cccaaagtgc tggattacag gtgtgagcca ctgcacctgg 15120

cccaaccaat aggctctttc taaagaaact agaaaactaa agtatctcaa aggttatgct 15180

aaatttcgga cttgggaaat acctgctctc agaaatgttc aaatactacc ttttgtatct 15240

gactaaactt ccagtcatac aatgcataag aaaactacat tttttttcat tttggtttct 15300

tttgtgatag gtttagcatt cagcttgtag gctgatgtct ttccttaaat tttaggtcat 15360

tatttctgga tgctaagtcc attcagtcca ccatctccag ctcgcagaat tactgaagtt 15420

tggggtattc cttcccccat tgatactgtt tttactaggt gcaactgtga aggaaaaact 15480

ttcttcttta aggtaacaca aatatctttg tgagagaaat ttaataataa tttgtaactg 15540

cattatttat tacaacatat attgccttaa cctccaatat gtgtcctttt aaatactctg 15600

tcatcaaaat ttaatcataa ttcaacagtt tgataacagt aatggtcaaa attcaaccgt 15660

ttaaccaagt taacacgttc tctctatact taaatctaga aaggagttcc aaatcttttt 15720

tcccaaagct gataacataa ttttctcttt taaattatca ggattctcag tactggcgtt 15780

ttaccaatga tataaaagat gcagggtacc ccaaaccaat tttcaaagga tttggaggac 15840

taactggaca aatagtggca gcgctttcaa cagctaaata taagaactgg cctgaatctg 15900

tgtatttttt caagagaggt atgtgttaca taattgacaa gattacaaaa cttttaaaga 15960

attacactca gcattttcat ttattattga ctttactgtc aaaagatatc tttaatggct 16020

gaaatcaaat attttcagtg aaaaaaatca atattgaggg tagtattctt attgccctca 16080

atttttattt tcatttcttc acaggcaagt cacaattcat ttgagttttc aggtcactga 16140

tagaatgtca tttactttcg cttcacaaat gtgcatgtca tccttgcaca agggccatgc 16200

taatctctgt atcattccag ttttagtata tgtgctgcca aactgagcac ttgtcacttt 16260

caattgaaat taaatatata actatgtaat ttgctgcatc tattcattca acaagtattt 16320

cagtttaata attattttta taataccctt gactaatagc cattattaat atcaatatat 16380

tatatgaaaa atgagaacaa aactgtttcc tatacattgt aaaagttgtt ttccaccttt 16440

tctgagggta ttaaaattaa ttttcatttt ccatgtgata ttctaataca taacaggtgg 16500

cagcattcag cagtatattt ataaacagga acctgtacag aagtgccctg gaagaaggcc 16560

tgctctaaat tatccagtgt atggagaaac gacacaggtt aggagacgtc gctttgaacg 16620

tgctatagga ccttctcaaa cacacaccat cagaattcaa tattcacctg ccagactggc 16680

ttatcaagac aaaggtaaca tttttattgt gttaaaaaaa tccttaaaca taagtagatg 16740

taatacagtt tcttatacag taaggcttat gaaatatgga tactgatcaa acagccctaa 16800

taatttaagc ttactgatga aaaactcatc cacttgcctt agtgaataac caaagaccaa 16860

tactttcttt ttccttgtgg gtaagtaaag cagtttgttc aggtttgtta gttataacca 16920

atactattta tcaagtactt cttaccaaga acattatata ccagtctata accctcaata 16980

gttctacatc agagggctaa aactgagatt aaaactcaga tgtctggctc tttccaagat 17040

agtacattgc cttttaatct ggtatctttt attaaacatg ccacttacag gtgtccttca 17100

taatgaagtt aaagtgagta tactgtggag aggacttcca aatgtggtta cctcagctat 17160

atcactgccc aacatcagaa aacctgacgg ctatgattac tatgcctttt ctaaaggtaa 17220

ggtattaact aacagtttcc caaggaggtg atatcatttg tgaaaacatg aagataaagt 17280

aaaaatgctg gctgcaatga tgactgaatg tgagaagtta cactacggta cttattctaa 17340

ttgctgtgga aaagagttaa gactttgctt ttaatttcaa ttaaaatgat ggcactgcaa 17400

ttcaattctg ctctaacctt catgagatta cgataaaaca tccagttact agagtaaact 17460

tgctactgac aatagtattt tacttctatc atttgtggca tttaacagat accttgtttg 17520

gcacaaggtt ataatgattt gcatcactgc agagtctgta agatcagaaa gaagttcctg 17580

agaaactaat tttattaatt aaattatatt actcaggatg cttttctttg aaatgcccag 17640

aaatctaagt tattctgcct gtaatttcat aatatgaatg acattggcat gtatttttta 17700

aaaggaataa ccccaagtaa attatttttc aaacttggtt actctttaat gtttacttca 17760

taaaggagag ctgaacctga ttttacaaaa agatggtgaa atgtcaatct tttgaaacat 17820

caaaaaagct gaaaagtcta ggcaattgtt tttctttttg ttataaagtt caaattaatt 17880

agtaaaaaca tctctattaa aatgataaac attgtctttg agcataatag tcaacataag 17940

ttatttttta gtttgggcat tgttttcttt ttaactaaaa aaatgttttc tcttccattt 18000

agatcaatac tataacattg atgtgcctag tagaacagca agagcaatta ctactcgttc 18060

tgggcagacc ttatccaaag tctggtacaa ctgtccttag actgatgagc aaaggaggag 18120

tcaactaatg aagaaatgaa taataaattt tgacactgaa aaacatttta ttaataaaga 18180

atattgacat gagtatacca gtttatatat aaaaatgttt ttaaacttga caatcattac 18240

actaaaacag atttgataat cttattcaca gttgttattg tttacagacc atttaattaa 18300

tatttcctct gtttattcct cctctccctc ccattgcatg gctcacacct gtaaaagaaa 18360

aaagaatcaa attgaatata tcttttaaga attcaaaact agtgtattca cttaccctag 18420

ttcattataa aaaatatcta ggcattgtgg atataaaact gttgggtatt ctacaacttc 18480

aatggaaatt attacaagca gattaatccc tctttttgtg acacaagtac aatctaaaag 18540

ttatattgga aaacatggaa atattaaaat tttacacttt tactagctaa aacataatca 18600

caaagcttta tcgtgttgta taaaaaaatt aacaatataa tggcaatagg tagagataca 18660

acaaatgaat ataacactat aacacttcat attttccaaa tcttaatttg gatttaagga 18720

agaaatcaat aaatataaaa tataagcaca tatttattat atatctaagg tatacaaatc 18780

tgtctacatg aagtttacag attggtaaat atcacctgct caacatgtaa ttatttaata 18840

aaactttgga acattaaaaa aataaattgg aggcttaagg attagaaaac ttgttccatt 18900

tattactatt ccacattcta tcatgtaatc cagttatgtc agaaattcac ctctctgtct 18960

gttaaactga cgaccacgga caccttgtct caatgttgtc tgaagtctta ctcgtctgaa 19020

aggctttggt tgactactac tagtatctaa gaaaaacatt aagataaaag aaaagcaagt 19080

gaaaaaatga gaaagcattt ataatgatac aaactaatta ctaaatgaat gaatgaataa 19140

gatgtagact tgttcatttg atttcaattc tttttttcaa acaatgagga acatttaaca 19200

aaccgaaaaa gggtaaatgg ttaaacaaca aaaaaagcag cactttggga ggctaaggtg 19260

ggaggatcac ttgagctcag gagttcgaga ccagcctggg caacacagcg agaccctgtt 19320

tctacaaaat tataaaacaa ttagctgggc atggtggaac atgcctacag tcccagttac 19380

tcaggaggct gaggctagag gatcacttga gcccgggtga ggttgaggct gcagtgagcc 19440

atgatcaagc taacagtact ccagggagac cggtgagatc ctgccttaa 19489

<210> 8
<211> 13649
<212> DNA
<213> Artificial sequence

<220>
<223> Mutated epx gene sequence; mutated base: chr17:58193733 (G->C);
      mutated codon: caG->caC;

<400> 8
gcaccttgga gggagtgagt agggctaggg gataaggaga ggggccaaat cattcatcac 60

agatcattca ttcactcaac aaacacctgc taagcacata ctaaatgctg ggctttgagc 120

tgggtatggg gggtgtgtat aacagtaagg cagattcagt ttctgccctg ttgcaggagg 180

agaggcaccc ttccaatcat ctggcccaac tctcattcta gggatgtgaa aactgaggcc 240

tggagaggga aagtggcttg cccaaggcca tgcagccagg cggtggcgac tgaggtgcac 300

cgctcctgtc agccaacaaa tatccattga gcgacacctg tgtcccaggt gctgctctgg 360

gccctgggag aagtgcatca gtgggcttgg tagtagaggg tagggatgga gtgaagggta 420

ggcaggaaga atgtccccag gctggtagga ggtggggtgg ggggtttcag tctcaaaact 480

cccatgaaaa ccagagagaa gtttcagaac tccacccaag aggctgggtt tctagggccc 540

agagctgccc tcccccaccc tagaatgggc tataaaagtc ccttcccagc tacgtccaga 600

gaagagctgg aggaagtgag aggtcggctg ggggtcctca aagtgagagg ggagcagagg 660

atcctcccgt gcaggctgtg gatgtcactc acttcccagc tggtgaagcc tcgctgcaga 720

gatgcatctg ctcccagccc tggcaggggt cctggccaca ctcgtcctcg cccagccctg 780

tgagggcact gacccaggta atagtcccct agacaggcaa ggaggaggga ggggaaatgg 840

aaggggaagc acttgggtct tggagggggt cttgtggctt gctgaaccct gagtccccat 900

ctctttgaac agcctcccct ggggcagtgg agacctcggt cctgcgagac tgcatagcag 960

aggccaagtt gctggtggat gctgcctaca attggaccca gaagaggtgg acttgggtct 1020

gggggctgca tgggcctggg aggatcagtg aggggcatgg gccccagcca agttcatctc 1080

actcatcagc cacctctgga ccccatgaac atttcctgtt ggtagagcct cccttccatc 1140

catccttctg tccgcttgcc ctgtcctgac tgtgccccag gactgggtct ctgctgggtg 1200

ggtctgcacc ctctctccag ccctcactcc tcctctcctg ggcagcatca agcagcggct 1260

tcgcagcggt tcagccagcc ccatggacct cctgtcctac ttcaaacaac cggtagcagc 1320

caccaggaca gttgttcggg ccgcagatta tatgcatgtg gctttggggc tgcttgaaga 1380

gaagttacaa ccccagcggt ccggaccctt caatgtcact ggtactctga tccccactga 1440

gcccgctggg cctaccctgg cctggagtag aaggaatcca ggagagggag gcagggtgca 1500

caggtttggg gtgctgggag gagagagggt aaagggatgg gaggtacaga gcaggccagc 1560

tcaggtctgc ccatttgcct tcccacagat gtgctaacag aaccacacct gcggctgctg 1620

tcccaggcca gtggctgtgc tctccgggac caggccgagc gctgcagcga caagtaccgc 1680

accatcactg gacggtgcaa caacaagtgc gtgcggggcg gcaggagggg ctgcccctgc 1740

ctgggggacc tctcccttcc tgcacccacc ctctccctcc atgctgagcc atctccaggc 1800

cctgccccct gctaacctat cccacccatg gctgcaggag gagacccttg ctaggggcct 1860

ccaaccaggc tctggctcgc tggctgcccg ccgagtatga ggatgggctg tcgctcccct 1920

tcggctggac ccccagcagg aggcgcaatg gcttccttct ccctcttgtg agttggggct 1980

gagggtttgg gaggttgctt gatctcttaa atgcggggag taaaacacag cccagagtca 2040

cgcaggcagg gcttgaaccc aggcccttcc actgaccagt gcagtgactt gaggcaaata 2100

acacagcttt ctgaggctta gtttcctcat ctgtaaaatg gccctaaaac ctacctcgta 2160

gagcctgtgt ggatattgaa gtccttggta taaatagcta tagaggacat gatctattca 2220

tgctaaccat tacggtgttg tacggttttt tctgagaaaa ttagtgtcaa agtcttgcta 2280

aatataaaca gctctatacc ttaatctggc tttatctatt gatctatttc acatttatca 2340

actcatcagt tttctattga tatatcgata gagcagtcta tcatctaccc atctatcaac 2400

atctattaat taattttctt ctatccaccc ttccatctgc tgaccccgta ctccctacgt 2460

gtattatcca tcacctggca accagcccat gcacctgtga atccgtgcta tcatctggtt 2520

agctatcagt gtacaaattg atctatctat ttgatctccc ttcccctctg ggagagctgg 2580

tgaggtctga gccagtcaac ctagcccctc tccttctctt taccaccgga atcctcagga 2640

gcccagccag aaaccatcct tctaggaatg agagcaggag gtggctacgc ctccagggac 2700

aaaaggggca tggagggcag aagaggagag gctgtcaatt ccagcagggg agctgctgct 2760

ccctgagtcc tgggttggct ctaatacctt gtggggtcag ggagcccatg tcccgtgctg 2820

atgttatttc cccaccaggt ccgggctgtc tccaaccaga ttgtgcgctt ccccaatgag 2880

agactgacct ccgaccgtgg ccgagccctc atgttcatgc agtggggcca gttcattgac 2940

catgacctgg acttctcccc ggagtccccg gccagagtgg ccttcactgc aggcgttgac 3000

tgtgagagga cctgcgccca gctgcccccc tgctttccca tcaaggtacc taccctcagc 3060

caatctccca tgcccttgtg tggcctcccc caaaggcaag gtgctggggg tggggatctg 3120

gaagactgga gcaccatcct taaggagctg cctgtggagc tagggtatga gacagagaca 3180

caagaaacac agctgagcag agaccccgcg ccgtgtgtgt ttgagaggtg ggggtagggc 3240

aatctgccag gaggctcagg tcaggcttca tggagtggga agcccttgac acatgccttg 3300

acccatgggt ctgaatccaa caggggaagc ctctgggccc ctgcttttgg caacctaagg 3360

gcctcttagc tcttgccctt ctctccttcc cagagtagtc caagggtcct gcggctcttg 3420

ccagcttctt gggcttgggc tgtaagtagg gtcctaacct tcaggctaga agccaaggac 3480

agtgtggggc actaggggag gaaagataga atccagggag cagagtctct gctaggaaca 3540

tagggttgac acacgtgcac acacacactc tctcacacac acacacgtgc acacacacgc 3600

tccctctcac acacacatgt acacacacag tcacccttaa taggtggggc cacattcatg 3660

agggatgaca tgtgagggcc tctggatggc ctctccctgt tgcaacctta tctattcctc 3720

aatccctggc ttaggggcac ctcctctggg gagccttctc agatcttcct attacacttt 3780

taacatatgt cctcccctcc cctctcctct ctctccttcc ttccttcttc cttcctcttt 3840

ctttctttct tctttctttc ctttccttcc ttccttccct tccttccttc atttccttcc 3900

ttcctttctt tctttctttt tctttctttt tctttcttcc gtcctttctt tctttatctt 3960

tccttctttc tttcttcttt attattatta ttattgtttt gagacagaat ctcactctgt 4020

tgtccagact ggagtacaga ggggcaatct cagctcactg caacctctgc ctcctgggtt 4080

caagcagttc tcatgcctca gcctcccgag tagctgggat tacaggcatg tgccaacaag 4140

cctggctaat ttttgtattt ttagtagaga ttgggtttca ccatgttgac aaggctgatc 4200

tcaaacttct ggcctcatgt aatccacctg cctcggcctc ccaaagtgct aggattatag 4260

gcgtgagcca ccacacctgg cccacatatg gcttttctat caagcacagc agatgccact 4320

tcaacttagt ttcttaactc tatgtgtgtg tgtctttttc cactgaactg ccagcttcct 4380

gcaacctttc ctgtgttttt ttaaccttca taatctccaa agcagcaagc aagaaagggg 4440

cttagaaaag gtctgtcgaa ttgaacagaa ttaaagaagc tcaggcctaa gagtcaagag 4500

aactaagttc taatcccacc tttcctgcta actggttgtc acttcccctc tctgaggctg 4560

ttttccttgc tgtaaaatga ggatcgagat tgtttcaaaa gcctcattca ttcctaacct 4620

tttgagactc tatgaaacaa aatgttaagg acactcagac tgacggggtg agcagcatga 4680

gcctgggtga gtcaaggagg gcttcctgga ggaagggagt tttaagcaag gttttgagga 4740

ggtggaagat gacggagagg agtgagtctg ctattgaggg ggccccatgt cactgtctcc 4800

tcttccatct cagatcccac ccaatgaccc ccgcatcaag aaccagcgtg actgcatccc 4860

tttcttccgc tcggcaccct catgccccca aaacaagaac agagtccgca accagatcaa 4920

cgcgctcacc tcctttgtgg acgccagcat ggtgtatggc agtgaggtct ccctctcgct 4980

gcggctccgc aaccggacca actacctggg gctgctggcc atcaaccagc gctttcaaga 5040

caacggccgg gccctgctgc ccttcgacaa cctgcacgat gacccctgtc tcctcaccaa 5100

ccgctcggcg cgcatcccct gcttcctggc aggtcagaca gggaggaagg tggtgtcttc 5160

ccaggaaaca gccatccctg gggtcccaac tgggaagcaa tggtgggatg tggtgaaggt 5220

acatggtttg ggacctcagt attaggcaca ccataagcat ggatctgtgc acagccatca 5280

tagaatcaga acgttgagtc cctttgcaag ctccctgtgt gtggccagag ataactgctt 5340

aaccgtctcc agtgacaagg ctctcaccgc ctctatggca ccccagctca gccacaggca 5400

gctccgactg ttagaaagtc tccttatttt gatcacatat tcacctattc cccttaatca 5460

tgcttctgga acctcagaaa agacactgtc cctcttacac gcaggaatac ctcaaagtag 5520

ctgttaactc catctccatt ttttttctct tctctgggat aaatttccgt agttcctccc 5580

tgtccccagc tgttgctaga cagttaatac cctcttctgg aagcataatt tgaactttcc 5640

tcagggtctt ttgtcccatg tacatggtta caataataat ggtagtcatt tctcattaag 5700

atatttatct tattattatt attattttaa gatggagtct cactctgtcg cccaggctgg 5760

agtgcagtgg cgtgatctca gctcactgcc acctctgcct cctgggttca agtgattccc 5820

ctgcctcagc ctcccgagta gctggaatta caggtgcctg ccaccacgtt tggctaattt 5880

ttgtatattt agtggagata gagtttcacc atgttggcca ggctggtctc gaactcctga 5940

cctcaggtga ttcacctgcc ttggcctccc aaagtgttgg gattacaggt gtgagccacc 6000

acgcccaacc ttatcaagat atttaaatgt gccatctcta aaccttgcaa cagccctgaa 6060

aggagggaat tattattgtt ctcattttac gataagaaac tgagactcag ggaaagtgaa 6120

tcatgcgccc aagagcacag agttagtttc aaatgacaga gtccacttga gaacacagtc 6180

agtgtagtat caaggtctct gcccagtcca ctgtattcca ctgttttcct cacagagagt 6240

cggccccaac ccctcttttg aaggattgta catttgtgtg tcctggaaag agcagcttgt 6300

gccttcggga gaggtcaact gaactaagac cttgtgcaaa agggtttagt taaaaccaag 6360

caaatgatgg aattaacttt ctttccctgc ctgtagtagg tctgttcgta aggacagtct 6420

ctgcccaacc ctccctctct tcctctttcc ctctggttca cgggggagag cagtattata 6480

ccaagcccaa agataggaag aggtacacca gcccttggca gagaacacgc aggcagatgc 6540

agctcatgga caggtgggct ctcagtccca aagtcaaggt gcccttgccc atagctggtc 6600

caggctatgg atctggttgt cttgtagagt agaatcctta ggcaaagatg cccctgcctg 6660

agaccagcag ctgccctgtg ctttcccacc tcacccaagc ctgctaggaa cccccggtcc 6720

ttccgctcct ggcctgtggt gttagggagg agggtggcga tgaggagcct cagcacagct 6780

gaagagatgg aggtccagtg agggccagga gtttggccca ccccgtctct cccatcccca 6840

gccctgggtc taccctggta gaaagacatt tctctgggaa aggctgcagt aaatctgagc 6900

ttggggtttt caaggtgaca cccgatcaac ggaaaccccc aaactggcag ccatgcacac 6960

cctctttatg cgagagcaca accggctggc caccgagctg agacgcctga atccccggtg 7020

gaatggagac aaactgtaca atgaggctcg gaagatcatg ggggccatgg tccaggtaag 7080

gagctctgca tcccagcatc ccccagatga caagcttggc atgagaagca gtccttaaca 7140

catccttgcg gatgtgccta aaaccagctg ggtctgggca actggcggag cacctggacc 7200

tgtcctctgg ccccgattct gcctctaact cccgtgtgac cctgtctgtg tcactcaccc 7260

tctctgggct ttgtatctcc acccaccaat agtaaattaa tgttgtcaca tttgacgtga 7320

tgacaataaa gaatatgtct gagccaccct ttgaaaaggc aagggtatgg gtgagtagcc 7380

tctggggaat gttcctcctg tcttcccttc cagatcatca cctaccgaga ctttctgccc 7440

ctggttctgg gcaaggcccg ggccaggaga accctggggc actacagggg gtactgctcc 7500

aatgtggacc cacgggtggc caatgtcttc accctggcct tccgctttgg ccacacaatg 7560

ctccagccct tcatgttccg cttggacagt cagtaccggg cctccgcacc caactcgcat 7620

gtcccactta gctctgcctt ctttgccagc tggcggatcg tgtatgaagg tgaccaggtt 7680

ttccaggggg caaatggggg tgagggtggg gagcatgccc tcccctaggt gggccaagct 7740

tactgccagg aagccaggct gctgcagagg ccactgctaa tatctcccca ggacagtgga 7800

aacaaggcag gtgccagcaa gaccctcagt cacgggcctc ccatcctgtg tggaatgaga 7860

ggatttttta aaggggtgga gactaatgtc agattgatgg ggagctcacc ttccattcct 7920

taagaagtac ctcccagctc cagctgcttc atgtctctcc agaactctgt ttcctgacaa 7980

acgttactaa catacccgac tggcttgtcc agctctgggc tagcttggca tcatgtgata 8040

acccaagtag cttcccagag gctggtccaa tctgtgctgc tcacattccc tgccaccagg 8100

gggcatcgac cccatcctcc ggggcctcat ggccacccct gccaagctga accgtcagga 8160

tgccatgtta gtggatgagc tccgggaccg gctgtttcgg caagtgagga ggattgggct 8220

ggacctggca gctctcaaca tgcaacgaag ccgggaccac ggccttccag gtgagggggc 8280

tgtccacctc ttctcccagc tttgctcggg ccaggctgct caaggggttc tgggaagacc 8340

ctggtacctc ctttctgact gggactgtct acaggatgtg caggagtgca gaggcatgca 8400

aggccaaggt cgatgtccca aagcactcct ggaacaccgc ttgctgtcct gccatggctc 8460

ccccatccac tgtaggggct ccagctttag gttcaccaca ggaccagcac ttaggctcag 8520

gagctgaaaa agggaggctc gaactttcca agtagaaaaa aagcaatagg cttaaaaggc 8580

agggaagaac tgctccattc ccaacacgct ggcaagttcc aaggaggaag gctaaaattc 8640

cagagctaca gaaacgctcc ttgctctaag tggagaaagg caaagagcct tagaggcagg 8700

gaataggtgg aataaaacta aggctgcagt ggctgtttgg tgagggcaca aatgacttta 8760

tcccaggaaa gaagaaaata taggcacttt gttagtcact ttcatgtata ttaatttatt 8820

ttgttctccc aatcaccttg agaagcaaat gattacagcc attttataga tgagaaacta 8880

tgttcagaga cagctagtaa tttgcccaag atcacataaa taagaagtag taaaatctga 8940

atttaaattt acaatttctc ctttcttccc cctgttcaac cccagtaggg gaaggttttg 9000

gccactgcct tgctcctctc tccttcccac ctcctacctc cctgtctaga aagaacctga 9060

aggcccatct gcccatgtat gaagcagtgg tggcacctcc tactgcccac cccacttcac 9120

acagcgcctg gtagagcaca gcaggcactc cggaaacctg agcttctttc ctttcttaaa 9180

cttgggcgct cctggcagct aaggagctgt gggcaccccc tgccggagag ctgagatcgc 9240

tagccaccca agtttctatt aggttttgaa gggaaggcaa gggttaagga aagacacaaa 9300

gagagagggc ggctgaacag ccaatgcagg tatttatttc cagcataaaa cctacagagg 9360

tgggggacca gcttattgcc agaacctgcc gccgcctaca gctggggtac tgggtatggg 9420

tatgggtatg ggcaggaggg gtctgggtcg tatggcttgc tacccagcag gatattgata 9480

agatgtacct atgatcaggt ccttttttca gtgggatgtg gtaagatgtt ccttggacct 9540

ttgcccagca ggatatgata gggatgttcc ttcagttggg cctttgccca ttagggtatg 9600

ttatgttttt catggcccga cctcctgtgg aacgtttcac tttgaccaag gtctgggaaa 9660

tggtgggggg cttagaaaat ggtgcagttt ggactaacat tcttgccttc tactttggta 9720

taaaaggaag agctggagag ctgagatcgc tagccaccca agtttccaca ttgcgagctt 9780

ctgctccccg caatacctgt gccctccaga aatattatcc tgacactagc tcttgcaatc 9840

tcctctaatc ccttcccaag tccaacatca aactgggaac tggacaacct ggtttgagct 9900

ccacttaaca gtaaagtgac cctgaagcct atggttgctc aactttagta aacataccag 9960

atatagttag catgccataa aagcacctca aaaaggtgct gtgagggtcc tgagatattc 10020

acccaatagt gctcagtaat ggtaaaatac tgtgtggttt tcatgggttc cgctggggga 10080

tggtgtgtgt gagctgtagt tcaatggatg acgttgggct ggtgtccctt cgggacatgg 10140

caaagctatc tcctaccttg attgtcacat cttatgatgt ggcttggccc tgcgtgggat 10200

cctgatggct gacaaattgg tatggcttgt ctttggaagt ttctgaaaac tttttaaaaa 10260

cttctttatt gagatatacc ataaaattca cccatatgaa atagacaatt caatgatttt 10320

agtgtattta gagttatgca cccatcacca tgatctaata tcacaccatt ttcactcccc 10380

aaagaaacct tgtaagaaca gtcaatcccc atccccaccc cagccccaat cacctctaat 10440

atactttgtg tctctatatt gacctatgca ggacctctca tatcaacaga atcatagtgt 10500

gccgtccttt tctgagtaga tgaattttat cattgctctc catttgtttg cttgttcatt 10560

catttattca tttgctcact cattcattct acaaacctga tggagagtct tttatattct 10620

gggtactgtc ttacttgtgt ctaatgaatg cagtcgagcc cagtaatatc actaagtgtc 10680

ggatttgtgg ttatgagttt tgggttaaaa cagcttttta ggttaggaca cactcctcag 10740

tctgatgcac actgagcaca gttgtccact cacattttca atcagaggac agaaacctag 10800

tctttggaag ctctagggac tcttctgcca gcaggatctt ctggttctgc ccaatattga 10860

ctggccacag cttcccccca gaggactggt ggagaaaaac agaagctaat gggagatcag 10920

caagactgaa gctgcttctc cccgttcccc tgcagggtac aatgcttgga ggcgcttctg 10980

tgggctctcc cagccccgga atttggcaca gcttagccgg gtgctgaaaa accaggactt 11040

ggcaaggaag ttcctgaatt tgtatggaac acctgacaac attgacatct ggattggggc 11100

catcgctgag cctcttttgc cgggggctcg agtggggcct cttctggctt gtctgttcga 11160

gaaccagttc agaagagccc gagacggaga caggtaagtg accctatcat aaaagacatc 11220

agcaccagag gcagagcaga aaaacactag catttcaaga ctaaacattg aagaacactg 11280

ctctttttag tatcatttct tccaagttca caggatctga aatcaggagg ctcctctctg 11340

aaaagctggg tcaaaggaag agagacactg acccaggcaa ggcccatatt gcctgagctg 11400

gagtcatctt aaacccagag gttgcctggc agcctcacta ttctgggttg gtggatccca 11460

gaaaacatgg gcagaaaggg ctaaatctgg tttccctcca atacatttgt gatttaagac 11520

ctgacggccg ggcgcggtgg ctcacgcctg taatcccagc actttgggag gccgaggcgg 11580

gcggatcacg aggtcaggag atcgagacca tcccggctaa aacggtgaaa ccccgtctct 11640

actaaaaata caaaaaatta gccgggcgta gtggcgggcg cctgtagtcc cggctacttg 11700

ggaggctgag gcaggagaat ggcgtgaacc cgggaggcgg agcttgcagt gagccgagat 11760

cctgccactg cactccagcc tgggcgacag agcgagactc cgtctcaaaa aaaaaaaaaa 11820

aaaaaaaaaa aaaaaaaaaa aaaaagacct gtctccttat ttggagttgg tattgcccga 11880

gttcaaatcc agccctggcc acttaaatta ctgtgcactt aatactgggc aatttatttg 11940

gccactgaat ttgtttcatt tctgagattc caatcttgca ggaattttgt gagaattgaa 12000

tggaataata tatgtaaagt acctggcaca caacaggtgc tcattataag gtaattcctc 12060

cccagccttc acccacatct ctcgactgcc tggtaggttc tggtggcaga aacgaggtgt 12120

tttcaccaaa agacagcgca aggccctgag cagaatttcc ttgtctcgaa ttatatgtga 12180

caataccggt atcaccacgg tttcaaggga catcttcaga gccaacatct accctcgggg 12240

ctttgtgaac tgcagccgta tccccaggtt gaacctatca gcctggcgag ggacatgagg 12300

cttctgcagg taaggggagg ccacctccag caccctgggc tgggttaagc cctcacatcc 12360

ttccctggat ggatggctga gtcctcttag gtctctaagc agagaaaaca gaacttgtca 12420

ctaggtactc tttccaagtg gcttcccaat gtgctagttt ctgggctgac agtcaattcc 12480

aggccctagg actttggggg gaaattagga gcatccaact atcctcagct tggtcactaa 12540

tacctgaaaa cagctccatt tctaaacatc cagtaaattc ctgacttatc cagttctgtt 12600

acttccacag gagtctatcc caagtctcca acttttggag acaaggggaa ggggaggacc 12660

atgaggctgc cttgtctccc tggagcaagt gcaggctgct gacgcttctg ctggctacag 12720

ctcagagctg ggttccccag ccaggagtga aggctggggg ctcctatcag caatggacct 12780

tcccgccttg ggagcctctt aggtattagg ctatgaatca gcgccacgtg caaaggcttg 12840

ggagccaagc catgtggtct tgcaccccag gcaagaaaag tcagctggag ggtttacagc 12900

actttctact gtttcccagc cctccctccc ctccctcacc atgactaaga gaccactcgg 12960

tcctagcctc cagacacccc acaatactcc tctgagcctg aggccaggca gcatgctctg 13020

cttctaccaa taaagcactg ctaagggcag gggctgctgt ttctgtgttg ggctagggaa 13080

gacgtggcct cagttccatg ctttgctcca gatgtttaag agaagtgctg ttaacacgca 13140

agctggcaca accatctaag cctctcctgg gggctgaatt aggagagccc aaagaaacac 13200

tgtggagtag ggaaaggcag aaaaggggaa gggcagacat ggtaggggcc ccgacattaa 13260

gaacacccta gccataccag caacctgagc agacaaacag ccaacagaac atcagtcaaa 13320

ataaaagggg tttatgtaac aaaaaacaaa aaaacaaaca aacaaaaaaa cacagtaaaa 13380

gataccaccc agctagcaga aaggactcag cactgccttc agccttcact tactcagaaa 13440

taactcatat ctcaacctca gcaagcccca agcagtagaa tgaggcttcc ctggaaagag 13500

gctgagactc acaggctggg gatttaagac cctctcaccg tccaccttcc ttccttcttt 13560

ggtcttggaa gatgaaaggc tccattttta aagggtggag aacagcagat cccctgctac 13620

tgtgagacaa gccagaaaag tgagtcaag 13649

<210> 9
<211> 28057
<212> DNA
<213> Artificial sequence

<220>
<223> Mutated PON1 gene squence; mutated base: chr7: 95316772 (A->T);
Mutated codon: Atg->Ttg;

<400> 9
gaagacgttt tgtcctgttc tgtagcatct agaacagcgc ctggcacata gtaggtactc 60

aataaatgcc agctgcatga ggaaatgaat gagctgtgtg ggggatgtac ttgagtgaac 120

tctaaagtca gagtggtgtt gagagaaaaa tgcttgaaat ccagatgttg gaaggtgaca 180

cagagtagta gcctggtgag aacagttaga tcttaggggt tcctactaca gccctccctt 240

ccgcaccttt ttggctgtca ccatgatcaa gctactgaat ctctctgaga cgcaaggacc 300

gggatggcac aaagtgagtg ctcaccaaag cttgactgtc ctttcccatg gcaatttact 360

tcagcttgtt tgatttcccc tccccgactg gactaggcac ctattctctg tcttctctct 420

ttacagttgg aaggagcaaa atgggacttt tggctgaaag tgctgagctc ctgcggtggg 480

ggctgaccgc aagccacgcc ttctgtgcac ctggtcggcc cagctagctg ccgacccggc 540

ggggaggggc ggggcgggcc aatcggcgct gccccagcag ggctgcggct gcaggcaggc 600

agagcctcct agcccgtcgg tgtctgcgcc catcgatccc tttgtctatc cccgaccatg 660

gcgaagctga ttgcgctcac cctcttgggg atgggactgg cactcttcag gaaccaccag 720

tcttcttacc agtaagttgg gttgtggtga gggctgcagc cttcgtcctc ctcactccct 780

gccagctcca cgagccccca gaaagttggg tccaggctgt taacagaaca tttacaagca 840

ggtgctagat gcacactctg cgggggtttc ataaatgttt gtccagccgt catgtgtcac 900

ttgaaaacaa actcttgctc atggcctgag acatgggata aggaggcaat tgccaggggt 960

gtgagtgccc aggttgtcag catacacgaa ttaatttttc atgaagattt gtgtgccctg 1020

gctacagatt cacttctcaa cctggaaaga aacaaactaa cctggctggg ggttctcctg 1080

atattttgtg tattagagaa aggagcccct gtcgcttctg cgccttgact gcctggccat 1140

ggcaaacctt ccaggcttga agattctcaa attgaggcta ctgagggcca cagcaaaact 1200

gcgtgtggac ccgagcaggt ggagctaacg tgtgctgtga tggttcgctt cagtgggggc 1260

aggcggggaa gagggaaccc tggagccagc atctgatgtc ctggggttta gctctgcccc 1320

atgagtatgc gatttgggca aatcacttta catctctaac cttagctgct tcagtaaact 1380

ggaaatagta atacacactg cctgcgacag agtagctatt caataaatgt tagttccctg 1440

cttcccttta aagtatttaa taaagggtta agtgctatac aaggggggtg gggatatttt 1500

aagaatgtat caaaagtttc ataactttta acaggagttc aaagtctaca gatcatccta 1560

gtctctaagg cttagtcaac aatgccttat ttactttaaa gtaaaaccta ataaaaatta 1620

aatataaaaa aattatatga gcttatggac taattagaag cttacattca gaatgggttt 1680

ttactattta ttgtggatgt ttttctaatt tttttaagtt acagagtgca gaaaaaaagt 1740

aagaagcagt tggtattctt ggcttttaaa tcatgtaaaa attacagttt gtgagttttt 1800

attaatcaca gtgctggtgg ctgccaaagg agattatagg tatagataaa aatatataga 1860

taaaggatgg gttgctgagc ctcctggagt gacccagaca gaggaaggaa gggaaatttt 1920

acccactgtt ggaaacccct aaaacctagc tactcaactg ggtctgggga ccagatgcct 1980

gagcatcacc taggggctcc ctgtacaggc agaatctcaa gccctacccc acactattga 2040

atcagaacca gcgtcatgga acaaactccc tgggggatta gagatcatga atctgcccca 2100

gggagagaag gcaagagatg atatcatgag gcctgcatca ggagcaagcc aaatccccag 2160

gatagatgca tccttttttc ttcagacttg gagtgtatgt gtgtgcggct cacaggaagc 2220

tatatatgcc tgggctggag ggaacttaag gaagcacaca agggatgcct caagagattt 2280

ctgagtttcc caggtgggag cagtacagag aaaagaactg agagagttcc cctagaccac 2340

gagtctccca cccacgagtc tcccaccttt gtagtcggct gtatgtgccg cacaagtcca 2400

gggaatgcct tggatgatgg tagaaacagc tcccctcccc taccccaact tgggctatgg 2460

aatgttgcag catctgtatg gcaacttcct tgtccctaca ttgctaattg caatacgtga 2520

gtccactgag tttcccttct cagaaggaaa ccacaaagct aacgttgggc ctctcgatgc 2580

atttctgcca ttccatgctt actgtaggca acacacagtt gtgggaagag agtagttgtg 2640

gattcctgat gacctgggtt agatatcatt tctgcaactt tcaagttatt ttaccacttt 2700

attccagaaa agttccagcc agtttctcca cccgaaaatt aggggataat aatacatatt 2760

acagcatata tatatatata tacacacaca cacacacaca catacatata tttatattta 2820

tataaaacat ataaataaaa ctaagtatat atatatatat aaaaatcagg catatagcag 2880

atgtcctgta aacgttagct tttatagctg tcatatgtga tctcaaaaat atagagtgat 2940

acttgaatat gtattataat catacttcat tgaaagtctg agataaggac aaataatcct 3000

ccaaccctcc agtctcccct atgaatgcaa ccaacaccat cttctcagtc tgatgtactt 3060

gcctagtatc tccacaaaac acttcagtgg acatctgcag agtaccttct ctttgcaaac 3120

gttgtgtttc tgctggaggt tataggaatg aataggttgc agtattcact caccaatttt 3180

aaagtctact gggagcaaaa gaacaaaaac tgacagctgt gaaactgaca actatgacaa 3240

ctatggtaag aaagttgaca gatgtatatg tgaaggttcc tgagggtaaa ctaaggagtt 3300

attaatccca atgcagaaga ctggagaagc tacaaagcat catgagtagg ggtctttcca 3360

gtgggtaatg ggctaaagag cattctaggt gaagggaaaa gtgtgagcaa ataaaaagag 3420

gtatgaaaga aatacaggga agagtcctta gtatcatgac acttgatttc aaatcccaac 3480

tctaccctct taaataagca accacatgca atttactcag cctctcagtg cctccatttc 3540

cttacctaca aaacagggta aatattcatc tttttctcaa aggtggctaa aaagattaaa 3600

tgaggaagta tgtgtgagag cccttattgg agcttggcac atagtaggcc atgaaaagca 3660

atttgaatga atgaggcgtg tgtagtagca gaaaatagac tgattagagt gaatttggcc 3720

ccaagaagag tttgagaatc gatacggatt tcttaatgcc taggcaaaga tctctgcttt 3780

tttctgcgag cagccagcat gtattttcac cgaatacttc tgccttggtt tggatgttcg 3840

tcctctccaa acctcgtatt tgatctctaa tgttggaggt ggggcccaat gggaggtgtt 3900

tgggtcatga agatggatcc ctcatgaata gattaatgac ctccttggga gtgagtgaat 3960

tctcactcta ttcatttcga gagagctggt tgattgaaaa gagcctggta cctctcccct 4020

ctcttacatt ctctctcatc ccatgatctc tgtgagtact ggcttctctt catcttctgc 4080

aatgagtgga agcagccaga gcccctcacc agatgcagat acccaatcct gaactttcta 4140

accatccaga atcataagcc aaataaacct tttttcttta tcagttgctt agcctcagat 4200

gttcctttag agtgacacaa agcagactca gacaagttca cagcaaaggc tgtatggttt 4260

ctgcgcagag ttcagagtct agtgaatcag agatatatct cagacattga cccttatctc 4320

ttaggcccag aacatttgga cttaagcctt tgacaagata tttcctaata aattgaaaat 4380

gaggtagaaa aaatatgagt tggatgataa taacacaggt gggtcaattt tcaggtgcat 4440

cttccagctt ttaagcagtg ttctctgtag attttgcctg gtcagagagc taaatgtgag 4500

ctttgtgttc atacttgaat tccaatccct gctcagccac tttataactc tgtggcctta 4560

gaaagccact taatcttggt caactataac ttagggaaaa taacacctca cggaggtgtt 4620

tgaggattaa acgcgggcat atacggtacc tgaactatag tagtaatgat tatcctgttg 4680

agatatgaca tgtttgggcc acaaattgga acccaaatca acaagtgctg acttaatgcc 4740

cccttgccca ccctgggctc cctttaactg tgattattct atgttttcca ggctagagat 4800

catcttctat gagagagaag cctgaattag aataggagct gaccagttct gcctttctcc 4860

atcttttgtg aacccatatg tttagtgacc cttgtgagtc aggcagcatg agtattcttc 4920

ccattggttt tgagtctaag aaaagtgaat ttgactatcc ttggtgtttt tcacaagtct 4980

tggcccattc agtgatttta tgtccttgaa tctctccttc catgccagtc tttgtgttgt 5040

cctcggttat gaccatcacc cttaacatct tgtacctgcc ctttggttcc ttgagggcat 5100

gctacaaata ttcactacac tttccctaga ccagagtttt ttcaaatggt gggtaacaaa 5160

gtcaatttag taaataatta ccaacattta aaaataaata aaacagaatt gaacagaaaa 5220

gttatgaatg tatgacacag attaggatga gtattgtttt gtgaagcttg attcagatgt 5280

gtatgtgtat gtgtgtgcat gtgctgggtg ttcgcaccat ataaaatact tttcctactg 5340

tgagtctctg tctgaaaagt ttgtaagcca ctgctttatc acttcccctt ttatttcctt 5400

attgtcaata tttgcaatta taatgtcaga ttttcatttt gaaaatacct ttccctttct 5460

taggttatgg gacagattcc aactttcctt tgaagcctgc tttctaagtt cacatctatc 5520

aatcaggaaa ccacagtctc ctcacagtcc aagccttcat catctattca ttcatctctg 5580

tatttctagt acctagactg gtggctcttt actctggtta tacgttggga caaactgtgg 5640

cacttttaca aagcagatta ttaagaggca ctccttgcct cttacgtcag atttttttag 5700

acagcgaaaa tgatagccag tggcctgatg gtaggaatgt actcattttt ttctgaatga 5760

ataaatgaaa gatcaatcac ccccaaattc ctatcatttt gtaccacgtc ggtaagaatt 5820

ttatctagcg tgtctgttcc gcctgtagcc catccgcctc ctgtgagttc aagtcatcag 5880

gaggacaagt aaatttctta gcctatctga agccaaataa gatttctagg gatatttgga 5940

taattgtcac tacttgctat gacaatactg tatcactagt tctctaaaaa aaaaaaaaaa 6000

agtttattgt ttaaatctct tggttgtgct aacgtcgtct gtttctttca aatgcagcat 6060

cagtagcttc gctctgctca ggcagccggc tggcacttcc ttctcacatc tgttcatttt 6120

tcctttgtcc tttactcaga cattttctat ggtgtttatg cactcatatc taataagtcg 6180

ctcctgtgta tgtgtgtgag tgcacctgtg tgtgtgaaat gcctttttac atgcaggcct 6240

tgtgtatgat tttctgaagt tacaaatcct ttcaacgtac ctgtattttt caagcactac 6300

atcacaaagc agatgcccag tccaggctgc ggagtcagat tgggcagatg agtaggaggc 6360

tgcccatctg tcagcaactc taaaacatca atggaataaa ctggaaccat gttgccattt 6420

acccggactt tccacctagg agtccttaca cactgatgaa attgctcctg ttcctgccaa 6480

agtacacaga gccctagaga ggatattatg tatctcttat gtgtacctat ccacctctgc 6540

taaataaaaa ctaaatattg tacccagagt tccccacttg tcttgccctg gccttgcctg 6600

gaaaaattgc aggaaaaact ctggagttga aactcaggca aagtacagtg aaggagaact 6660

tttgtggact gcctaccttt gcaacttatt ataatttctg aatagttttg tgtgtgtttt 6720

atttctttct cccccacaga acacgactta atgctctccg agaggtacaa cccgtagaac 6780

ttcctaactg taatttagtt aaaggaatcg gtatgtatgt gagaggaaga agggtctcat 6840

ttgaacttcc ggtatttttt tgctctttgt gtgattaatg tgcctgttca attctgtcca 6900

aaaataaaat tttgaatcag gagagtcagt ggtagttaat gataaatacc atgttgtttg 6960

acttttgact tcaccaacat actgccaagg tgttgggagt tttggttgtt tgtttttgtt 7020

tgatcaatag agcaaaaaaa aaaaaaagaa aaaaagaaaa aagaaaaata gaataatgtt 7080

tgattccatg gcaatctaac ctgaggagat ggcagaagag gggagggaga gccagataaa 7140

gcattgaata aaggtaactt ttttttagaa aggctctgac aggactttgc aattaccaca 7200

aggcttctcc ttatgcatct ctcttttgat attggacagg aaaatttttt ccagaagctc 7260

tcagcagatt tccccttaga tcttatttgt cagagctgga ggacaggctg gtgccctagt 7320

tgcaaaggat gctgggaaag taattatcaa gcctccaatg tgggaggcag gccctgtcag 7380

caaagataaa acggtaggag aatagcagct gggaggcaac caacaaggag agtccccggg 7440

ctcaactctc cagctggttc caaccaacca gctttgttat ttgaagacaa ttggtttgag 7500

atcatgcttt tgtgtgcttt gggtgtttct ttttctttct tttttttttt tttttttttg 7560

ttcttttaga attattcatc tcagttgatt aaggctcagg aggctagatc agatgactta 7620

gtaccactaa atttcactag atatcttatg gtgcgattgt tttctagaaa aatatgatat 7680

tgatatcaaa tatcactggg tttttgaaaa gcaaactttc cactaagtgt agaaatacct 7740

atcaaatagt tcatttttct ctagataaat tgaaatgtta aaaatttaca tgtacttaaa 7800

gtataactaa cttgaagaca gtaatattta accgatgttt ataattagta gtaaaaattc 7860

atattcttga tcacctaaaa tgtatcccca ttttaactca actatttgga atttgcttat 7920

atcttcaata tggctttcca agggtttaat tgatttggaa gtattaagta tcaacatata 7980

tttgtaattt accttgtatc tttagaaaac aagttctttt caaataatca tggcttttgt 8040

acgttttgtg aatttccatg aactttaaga ggattcagtc tttgaggaaa aagctctagt 8100

ccatcaattt aaaacaaata tactaagtgt aaattttaat taaacttgtt aatgaattaa 8160

tatattttta atttccatat aatcgcattc atcaatttga ataaatgaaa gaatgaatta 8220

ttctgaacct attaaagaag agtgatgtat agccccagtt tcaagtgagg tgtgataaag 8280

aaatggatcc acatcctgca ataatatgaa acaacctgta ctttctgttc tcttttctgg 8340

cagaaactgg ctctgaagac ttggagatac tgcctaatgg actggctttc attagctctg 8400

tgagtgtttt ctttcacttt tctgtgttct agaacgtttt ctaggactgg cagtttaagt 8460

ctttcactta ggctttctgt atacccatgc ccactttcaa ataaaatgaa cagtcatggt 8520

ttaaaagaca aattgtgtat gcatttacac ttgtgctata attatgatca tgcgtgttct 8580

atgcatattt gtgttcttcg gtctacattg aaccatatta cgttagcttc tattgcagca 8640

atttttttgt gagttcagtg tattcaagaa ttgttttcct ctcaattgtc tattccctaa 8700

gcacttaaaa ttgccatatt caaatatttg ctaaattaaa tatacattta atgtatacta 8760

cttcacaggt catttcaact ttcacatttt taaacaaatt tcagaataag ccctttaatt 8820

taaaattgtt gataggtccc aattcccaat tcccaagctt tgaagacaga atccaccata 8880

tttgctgaaa ctgtcctgca tatagatcgc tatggtagct gatatagtgc aaaacaaaag 8940

aagtccacca tctggcctag ggatcataac ataacagagt tgataggaaa cgacagacaa 9000

tcatccagcc atttaaaaac ctttgctacc ccttgtaatt tacttagtcc agttttctct 9060

tacaaagatg atagactagt catcctgcaa agcaatgtct tgcaaacttt cttgagcaac 9120

ataaggtgta tttcaagcaa aatcttatat gggaacacaa tatataaaac aacagcttta 9180

ttgttttgat tgaaggctaa gaaaggagta gttcctctgg cccaccaaaa ctctgtcttt 9240

gagatgcttc caaggaccta ccagggaaca gtttaaaagc cataagcaat attctctctg 9300

tattccttgc ttatatcatt gaagactatt attaattgcc tttttggtct gctcttccat 9360

ttttgatctt tctcatgggc tggaagctgt agtgtcgtgc acatatgact ttgctgcagg 9420

ggatgaaagg gtttgtgtgg ggtggcctag tggagcatgt ggcttccaga agacttctcc 9480

tggggaccca ttacagaagc taactaagag gcttcctatg tggccatctg gctagccctg 9540

tggtttggag cagtcaacat gaacccatgg gccatgcagc gcctctatta gcatttgaag 9600

tactggtatt gtgcttgatt tttctcctcc atggtttata agggattaaa gtatcctgga 9660

ataaagagct tcaaccccaa cagtcctgga aaaatacttc tgatggacct gaatgaagaa 9720

gatccaacag tgttggaatt ggggatcact ggaagtaaat ttgatgtatc ttcatttaac 9780

cctcatggga ttagcacatt cacagatgaa ggtaacaata tttactattt attaaaacca 9840

aaaccaaact taaaacaaaa acagaaaaac atgccaataa atgaattatt ttttctcatt 9900

caaacaaagc atagtcatga ataatgttct tactctgggt tttaaactta ccctctatgt 9960

taattgaact ttatagtaaa accacttaaa tacttgagaa tgctaaaata tttaagattt 10020

gtaaagcatc taattttgta aaatactaac attcacatta tccctataat taccataata 10080

ataatatcca gccctgatca ttatagcttt tccattttaa acgattttca agactcgggc 10140

atttacagga acagattttt ctaacccaag gcatctgtaa gctataggtc cagtgacaga 10200

aagtcaccat ggtcagcttt ctgccggtcc cattatcttc aatgcctcct gtccgctggt 10260

tcctctgttc tgtgttcaaa atgctcctct gcatgttggt ccctttcaac ataacttggt 10320

gtcatccttt atgtctttac tccctttctc cctttccatt tgctgtcact tccttgtctc 10380

tgtattaatt ttcagcccca gttgcacaat ggtagcacct gaagagttta aaaagactcg 10440

atgcccagcc cccagcttga cagattccaa ttccgttagt ctgagggagg cctggatcac 10500

agtagttgtt aaaagttttt cagttgataa aaatgtcagg ctagggttaa gaaccacgtt 10560

tatacccatt acctccattt catcatcaga cgatccctca ttcagttcct cttctggact 10620

ttccccactg tgggtggcta aaattgctac cttgagaatc atcagtgatg ttcctactaa 10680

caaatctcct ctcttccctt cctccctctc cttccttccc tcttcccttc cttccttcct 10740

tcacctcctt ttgttttttg ttttgttttg tttttgagat ggagtcttgc tctctcccag 10800

gctggagtgc agtggcgcaa tcttggctca ctgaaaactt cacctcccgg gttcaagcaa 10860

ttctcctgcc tcagcctcct gagtagcggg gaccacaggt gcatgccgcc atgcctggct 10920

gatttttcgt attttagtag agacgggggt ttcaccatgt tgcccaggct ggtctcgaac 10980

tcctgagctc aggcaatcct cccatctcgg cctcccaaag tgctgggatt acagttgtga 11040

gccaccgcgc ccagcctact tcacctgctt ttatgctgtt taccattttc tcattcacaa 11100

aacttgctct tccctgatac tgcagtgcac ctagctgttc cagttctcat cctactacag 11160

gttttctccc caattcattg ttttgtcact cattgctact tccctgagca ctaccccaga 11220

ggtttgatct tttacttcct ctatattttc tctattttag aactttcaaa ccagtgtcac 11280

actgcctgct ttctaggagg acatccccaa ataggggtat tattattctt cttcttttca 11340

ttagcccttc acttttaatg ccgcaggtgc ttactggaca cttctatttg gattcctttc 11400

ctatagacac tttaaactca aaatgttgaa agtgattttc catcctttta tctttccttc 11460

tcttccccat gtgagacaca cacacacaca cacacacaca cacacacaca cacatataca 11520

tatatatccc tttcaatgga ctttccactc atccacccat tcatcaccat tatgttttat 11580

tttaaaaata tttttaagta caaagaaata tagtgtttga tgtgtggata ctttttaaaa 11640

taaaaatgag gtaatattac acaacccctt ctataaattt cttctgcatt aaacactaca 11700

tcatggactc ctttatatat agcacatttg cagttgcttt gccaaggcag ttcgggattc 11760

agttgtgtgg gagcactgta atatagctca tctgctaaca aatatttagg ttgttcccac 11820

ctttatgctc attgcaaaca atgccaaaat aaactttctc gcacatgtat tttctgagac 11880

tatatcctta ggatacattt ctagaagtag aatccactgc acaaaatgtg aattcaggtt 11940

ttaatcctca cttgaatttg atttccaaca ccctccttcc tcagctgtat tcagggcctt 12000

tggattcttc ctgtcaaatg cctctcatga cctttcctgc cttttattgc cagtggcatc 12060

ctcttggtcc agtcctttct ctccacattg ctggattact gctgtagcct cagctcatgt 12120

tactccttgg ttcaaatacc ccagtgagca aagcttggat cttcatctta agccctaaca 12180

agactgattc tggctccctg tggtttcagc cctctcactc tcccactctc tcttgccttc 12240

actctgctcc cagcacaacc tcacaattaa gtgaatatgt ccaggaagtt ctgccccggg 12300

gcctttgcac actcccagca caacctcact attaagtgaa tatgtcccgg aagttctgcc 12360

ccggggcctt tgcacactcc cagcacaacc tcactattaa gtgaatatgt ccaggaagtt 12420

ctgccccagg gcctttgcac actttctccc ctcaacctgg aatgctcttt ccctagatat 12480

ccacattatt cgttctctta tttcacttag gtttttgctc taacatcccc tcatcagaga 12540

gaacatgcct gactcccatg aagataatac ttcgttactc tctattcttt tatcctgttg 12600

tatttttcct ttgtagcatt taacgccaac taaaataaat taaacacttg tttgctgaat 12660

ggggtaatga aaatcatttt agcagctttc tctgttcaaa tcttctcaat tcaacctgcc 12720

aaattttgca tcttaacatg gtgaaacccc atctctacta aaaacacaaa aattagccag 12780

gcatggtggt gggcacctgt aatcccagct acttgggagg ctgaggcagg agaatcgctt 12840

gaacctggga ggcggaggtt gcagtgagcc gagatggcgc cattgcactc cagcctgggc 12900

aacaggagtg aaactccgtc tcaaaaacaa aacaaaacaa aacaaaaacg ccaatttgat 12960

catgtcagag gtttcaaaag acctactctt tcttaaggca aagaccataa tctaactttt 13020

acttgccagc tctgatcaca cattcttgac cgcttagact ggtcaccttt gtgttcttaa 13080

agtcccactt atccaaggaa gctgcttctg tttgtttgct actatttctc agatgttctt 13140

ctatggaatg tatcatctca cactgtgtaa attacacctg atcacacctt ctcatattgg 13200

gagttcccat ggtttttatc cctttcatcg caactgtgca tttcgtggag accatttgaa 13260

ctggaaaact taaaggattc tttggagttt tagtgtcagt gatactcatt cttgagaatt 13320

tttttttctc ctgtggagtt ttgagttttc agagactgtc actggttctt ccttgagcca 13380

cttttgtttg gaacaggagc aatcaccaat gcttctctct acaaagagat gaaaatattc 13440

catctgcctg acatcctaac tactagctgg aggtgggttg aaattggttt ttggagagag 13500

ggctgacagt gagagcttag ttaatgtttc attttgtttt ttaatctcct ctcagataat 13560

gccatgtacc tcctggtggt gaaccatcca gatgccaagt ccacagtgga gttgtttaaa 13620

tttcaagaag aagaaaaatc gcttttgcat ctaaaaacca tcagacataa acttctgcct 13680

aagtactgag atatatcaca tttctatttt cctttagctg tagcggatag ttaatccact 13740

tttagttttt ccttttggcc agagtaggta tggctgttca actctcattt ctaatgatca 13800

caaactcctc cacatgctct tctgaagaac cttctgagga ctcaagcccc tcagagacgg 13860

cccattcttt tcctctcttg tttctcgagc tgattggatg ctggctcctg gcctcaggat 13920

ctccattcaa gttattacca ggaaaaaaat cctcttcttc agaaccagac ttagtatttc 13980

aaacccaaac cggcttcata gataaatttg acatcatgag cttatttatt tatattaaaa 14040

agtagactct tagatgtttc catgaattag aaacacagac ccaagaggag accctgaatc 14100

aagtgcatct aagacctcct tatactgcct ccgctgctgt ttgattgcac aaaatttatg 14160

caatagcaga tctgtggtaa cactcaattc cccctaggca ccttgttcat gtaacatatg 14220

gtgaaataaa acactcaaga atctccaatc tcagtcaact tcctcacatg ccctgtgaag 14280

atttggcttg taaggccagt gatggattta ggagaacaat ccgactctat cttctgaaac 14340

atttacctcc aactgattac gattttctag aatcccaacc tatttttata taatttatgt 14400

acagcatttg actacctcca tatctgtaaa tctagatttg tactctttgc aggtacctag 14460

ctaagggcct ggtatgtagt gtgcactctg tgttaccagg accccttcct cttctttctt 14520

atctggtggt ttgaggatga ggaaaatgag attggagaga aattgagtta ctggatgaag 14580

gccaatcaac tcactagtgg ccaagtagga accccagctc aggtcatctt ctgccccatc 14640

aagatgaata aaagttcaga gaacagcttt tggtggaaga gtttattcgt gcatttattt 14700

aacaaatata ttttggagag agaataaggt cactgagtgg agaatgtgag gaacaccaag 14760

atttaaagta gagaacagta tgtagaatgg agaagctggg caaatgataa cactaataaa 14820

tgagctttct ctgtaagtca ggtgctacac taactaattc aaacagatta ttttattgat 14880

gttttacaaa taccttctga attaggtgtt attattatat ccattttgga tttttaaaaa 14940

gttgaggcct ggactaattg ttaagtttcc ccagatgaca tcatcagtaa atggtgcagc 15000

tgtgattcta atcctggtaa catgagttca gagctggtgc tttaaacctc tacaaaatag 15060

tgaaaaaaat tatgtgtttt aacatttaat cttggaaatc tcagattggt ctacgttacc 15120

ggattggagt atttttttct tgagccagtt gcaaaaatat tggtcttagg cagcattgga 15180

gactgaaaat aaccacggtg ccgtaattaa ctagagttct gctctctcaa tccaaggatt 15240

agctgtgtgt attttcttct ttgtatttta tggtttttta taacattaat attccaataa 15300

ctaaatctat tataatttaa acttgttatt attcatatat cacaactgtt attattggtc 15360

atttactgta tgccaggcat ggtgtgaagg cctttgcatt cattatttca ttgtattctt 15420

actctagcct ttcaaggtat acagagaaaa cagaggttca gaatagtggc tcaaaattta 15480

gtgacaatgc tgtggcccca cattataagg atagcaagca gtgtcaggat gcaaatgaag 15540

tccatcagat ttcaaagcta ttactctatt accctttaaa aatttgttat ttatttactt 15600

attttactac acctgggggt agtaaatttt cagcaataaa aagctattgc tctaaaccat 15660

tccactctgc ccctttctgt gaaaaacaga caaatgatag tccagcatac taatttaaga 15720

aatttacttt taaaaaagag agagtaaaat cagattgtag atttatgtct ggagcccatc 15780

cagactaatc ctaaattccg agaccttttt ttttttttca tgttgtaata gtggctagca 15840

ttgattgagg gtttattgaa tgtcatgctc cctgcaaagc acacaacagg cattgtctca 15900

ttgtaattcc cactgcaact ccacaaatta agatcctgac ccttagtttt ctactaatga 15960

gctgtatgat tttgagaata tcatttaacc tctctttaca gatgagaaaa ctgaggctct 16020

gcatgcctgt ctaacttgcc tgagagctca gagctgcgtc agagctgatg cgctggccat 16080

gacgcctctg caatagagca gagataattt tctcccagat catgccctgg ggaacttcca 16140

tcacctcaat cagcaaagaa tgactcactt atgtgttccc tattgtatcc attaaagcag 16200

aaaaatagac tcatatatct tacattttca gtaacagaga atcctaatca gaaaggaaaa 16260

aaaaaaagcc atagcattct atttctgagc attcttgttc atggacattt cattcagttt 16320

agaatcacga aatcttaagg ctggaagatc aaccagtcta tcatcctgct ttcacagata 16380

agaaaactga ggtcctgaca tgtgaaaaga ttgatttttc ttagatcaca cagctaatag 16440

tagcagagct taaactaaaa tgcaagtatc caggctgctg gttcagcatt ttttcaattt 16500

cttatgggag ttagttctat gtgagcccag atatgtgagc acatttttgc cctgacaatg 16560

atttctggct taatttaatt tctactgaat ttagtgtatt tacacacaga cacacacaga 16620

tacacacaca cacacacaca cacacacaca cacacacgac gtaacactat tttaagatac 16680

atataatagg atttttatgt tacagtgcta taatcacctc ctcagatttc ttagaccaat 16740

gattgtctaa ggattgtatc ggcaggacta atttcatatt aattgtgttc cattatagct 16800

agcacgaagg ctccatccca catcttgatt ttaggaatag acagtgagga atgccagtta 16860

ataatcctgt aatgttcaat accttcacct tatatattat gtgtgtatgt tttaattgca 16920

gtttgaatga tattgttgct gtgggacctg agcactttta tggcacaaat gatcactatt 16980

ttcttgaccc ctacttacaa tcctgggaga tgtatttggg tttagcgtgg tcgtatgttg 17040

tctactatag tccaagtgaa gttcgagtgg tggcagaagg atttgatttt gctaatggaa 17100

tcaacatttc acccgatggc aagtatgtga actctctgaa atgtagtgga tttactcaga 17160

ttctcaggag atatcttgga atatattctt tttttaagta atatgataca ttttaacatg 17220

ttaccctagt gagataaaat taggaaaaat gtaaaatgtc ttaatttttc agggggtgaa 17280

atttatcaaa tcaaaaattt cattgtgaag gcatactggt tttatttcct ttttggaaag 17340

aaagtacatt ttaagagggg gaactgtgat gaagatttga tatgtctgaa tagctttgtt 17400

ttgagtaaag atgtgagtgt ataatgagtt gaatagtatg caggtataga tacgtcagta 17460

gtgcaggttt atgacaagat gaggaatttg taccaaaatg taaatcaagg caatacttcc 17520

ttcatcaaga aagtattact gtgaaaaaca agtcagttta actaagcagt gtgcttagtg 17580

atgtatttga tttataatgt tatctcatgg tgagctataa caaacaattt gcaaaccaac 17640

attggtctgg agactgcatt ttgggtagca ttggtatata catgaaatat ctatatccat 17700

ccacatatat gccctttctc atcttgtgtc atcatcaagg attttagagt ccgtaacata 17760

tgcttagaga aagcaacagg ggccaggtgt ggtggttcat gcctgtaatc ctagcacttc 17820

gggaggccaa ggtgggtgga tcacctgagg tcatagacca gcctggccat catggcgaaa 17880

ccccatctct actaaaaata caaaaattag ccaggcgtgg tggcgcactc ctgtaatccc 17940

agctaccagg gaggccaggc aggagaattg cttgaacctg ggaggtggag gttgcagtga 18000

gctgagattg caccactgca ctccagcgtg ggtgacagag caagactctg ttttttttta 18060

aaaaaaaaaa aaagaaagaa agaaagaaaa gaaaaagcaa gcaagcaagc aagtgagagc 18120

aacagggaca gggacagctt ttaattattc atatcataga ccagttttca aaaaatagaa 18180

gataattgag cagtaatttc ttttttttgt tggggggatg cacagtgctt agactaaccc 18240

cagatctttg tggggtatga ctaaagccac agggtcagga ccacactcag ggcagaagag 18300

gaggaagact tctcttgatc caagccaagc agcttttctt ctctcatatt ttctctggat 18360

taaaacaagg ggctgttgtc caagggcttt cctgcaactt tcccgagaga gaactcacta 18420

gcccctgaaa tgcagaacct gggccttgac actaaattgt caaaccaaaa gagaaacgct 18480

gtaatgcatt ttactcccag atactaaaag ttagtgttag ggctaagttc ctcccatact 18540

gataaggaca actatgtccc ctccttcaga aacaactttt cagagattaa acccctctgt 18600

cactgtaaac tcactgagtt ctttcagaat ttggtgtgtt tgaggtgggt tccattttcc 18660

attccttaag caaagttctt ccctatgaat aggtattaca acttcatctt aatctctcaa 18720

gttgtgttac ttctagtact ttgatgtagc tctttcttct ttgaaggtat gtctatatag 18780

ctgagttgct ggctcataag attcatgtgt atgaaaagca tgctaattgg actttaactc 18840

cattgaaggt aagacgtatt aacactgtaa gtctgcagag tgataattag attctaattg 18900

atttgtgaaa ttaaatgtga agaaaaaagg actgcttaat tggggtgggt gagaaccaat 18960

ttttcactcc tttattaaga gtcattagct tggttatgtg tttgttgttc atttttactc 19020

aactgcagag tggcaaacag caaaccccac ctcttacccg tgaaccttgc attctttctg 19080

ctctagcaaa ttttaagctt ggcattgtgt tcccagatcc tccttccatc cctcccttcc 19140

tgcctttctt ctgatctccc tctcctccac cttccttccc tctcctccct tttctttctc 19200

ttttttcctc tatgttgatg gaacagccaa gaaatagcct caaattgtac tttatgcagg 19260

gctgctctcc acaatgttta tgaagtccac gtgctcacga tggctgctag gtgacctggc 19320

tgcccctcac ctctctgcct ttacctccaa ccattgtgtc cctgccccac gtctcctgcc 19380

actgtctcag aacggggcac cattccctct gctaggagga gaaaccttcc tccagatctc 19440

catgaccccc tccttcatct cctttcattt ttactaaaac gtcacctcga tcttcaaaac 19500

ctggaactgc tccctcccgc tacgtatttt ccctcgcctt tcctgcttta tttttccatt 19560

cagcacgtat tgctaacata ctatatattt tattcatacc ctttattacc tgattcccca 19620

actagggtgt aagctccaca aggacaggct gtttttctat ttgcttctgg tacaattcac 19680

attgactagt tggcacttaa caaatacttg ttaaattact gcggtggggc tgggcatgct 19740

tcagggattc acctgctgcc cccaccaatt ctggtcatct gaaggttgga gtcaaaatgg 19800

gtgaaaacac aaaggagagg aaaaagagac caagagcaaa ctagagccct aagaaagcag 19860

ccctctacct ccgaaaaaca gcaagacgtt gctttcctgc cataaacgcc cactgcagga 19920

agcactgtca gcgccaattg tatgactgtt cctgtcagca catctgagag actgtcatga 19980

ggggcctcac tcagagaaaa ttaccttaaa gcaacatcaa atgtaacatc gtgtttgaca 20040

ttaaaaatca ctggcagggc tagtttggta ttagctcaag taaaatggaa tttcacacgc 20100

tcatactggc taagggaata aaaaacaagc tgcggtttta acaacaggta tttctgtaaa 20160

acaaaagtta aagaatttta tgagaataga acccaatgta acagacattc accacagaat 20220

gtcaaggctc tgctctcacc agggtgagac tgagggcaag gccagtttca gcaacagacc 20280

aggcctgtct cccatcatga aaaccccaaa aaggttctcc atcaattgtc acattattct 20340

ttcttttgaa agaccgtatt aattaatatc agttaattaa tgcccgagag tgccaagtaa 20400

tttgataaac tagctgaggc tattcttaaa tttgtgatga ggtgtggatg ggtttatgtc 20460

caattggata ttgttcagcc ttaaaaagga atgaagttct tggccgggtg cggtggctca 20520

cacctgtaat cccagcactg tgggaggctg aggtgggtgg atcacgaggt caagagttca 20580

agaccagcct ggccaatatg gtgaaacccc gtctctacta aaggtacaaa aaattagcca 20640

gacatggtgg cacatgccta taatcccagc tacctgggag actgaggcag gagaatcact 20700

tgaacctggg aggcggaggt tgcagtgagc tgagatcaca ccattgtact ccagtctggg 20760

caacaagagc aaaacgccat ctaaaaaaaa aaaaaaaaaa aaaaaggaat gaagttctga 20820

tacctgctac aacatggata agccttgaaa aacatgatgc taaatgacat aagccagcaa 20880

caaaaggata aatattgtac gatttactaa taggaagtac ctagaacagg caaattcatg 20940

gagacagaaa atagaataga atggtgattg ctaggaactt gggggagaga agaataggga 21000

gctattgttt aatgggtaca ggatttctat ttggggctga ttaaaagttc tggagaggga 21060

tggtgatgat ggttgcacaa cattgtgaat atacttaatg ccactgagtt ttacacttaa 21120

aaatggttaa aatgtcaaat ttttatatat tttaccacga tagaaataat tctctcctct 21180

ctccaaaaaa agattccagg acttagtcaa tgaattagtc acacttcaat gaacctcaat 21240

gcgcaaaaac ctggaatatt tgttaacaga gaatactctg tttttcccaa agagaataat 21300

ttattatgtt ttcttgacca aggaatgcga atttttcgag ttgcctttgt gggagtgtga 21360

gatgtgatac gcaggggtct tgcatgagtt agcatggaga ggaattattt atttgcaagg 21420

caaagagttc attgctggac tcagaacagg ctccctgcta gactgtcagc acttccatgg 21480

catgagctgt gtctgctggg tacctggtgc tctccaggtg ttgcgttcct tgcagagctc 21540

atgtaggagc tcagtaattg tgtcttgaat gaatgggacc ccaggtttct gtgtccttct 21600

cccactccca ctctcatctc aggtttcatc tgccgtggaa ttcaccaatt ggctgtactg 21660

cttccaaaga taagagcaaa gccctgaaac aaacattttc ctcattcaaa gacattatgg 21720

tatcagagtg gtgtgctgac actgggatgg acagggtttt agggatggag aaagcaagca 21780

tggagccgcc tgtcttgcct gtctctgggg taagaaagac tgatggctct gcatggtggc 21840

gagactgagg ctctgccatg atgcttgagc cctctaatcc ctccttgggc atgtgaggaa 21900

cagctctcat tttgagtagg aggcacaggg aaacgttggt tttctgcagc tgtctgctta 21960

tgccctggct ttttcaatga gtcatgacca gttatttaca ggttctctgg gcactgaggt 22020

atgctggcct ctgaggtaca tggtagatat tatttctcca actgagaatt agaggtaaac 22080

aaatagatgg gatttctccc cagtagaagg cagccaagat gatgaatggt ggaattaaga 22140

atcattggta ttatttatga tccttgtaca aagcctcgac agcaatctgt gcagtcatca 22200

aatgcttgca acttatattt agagaataaa cagcagaaga acggggagtt gaacaacatc 22260

aggaaactaa agaacaaact tttcctcaac tacaaggcaa aaatgagacc ctgggtgatc 22320

atttcttctc tggctgcaca gcctatgatt agccattaaa gagtccacat ttcattgata 22380

ttcttctgag aaagaatcta catttcctgg ttgttttctg aatgcttata aaaatatcat 22440

taccacatgc cattttagaa ggagatgttt tatgatgcac cgaaaacttc tttttttttt 22500

cagttttagg ttcatgcaca gatgttggta caaatgattt tgtctaactt ttaacagtct 22560

ttcttggctt tctgtatctt caccctaccc aattctgccc tggaaactga caaaatacta 22620

gcatagatga aaggtcagag aaattttgtg agtcttattg aaatggggca gaattgtatc 22680

cttggaagca gcagcgtggt gaccaaggac aagaggcaac ttttctctga tatattttgc 22740

tccatcacaa ttgtgtttta ctttatgtct tgttcttttc ttgtttttaa tcttttaagt 22800

cccttgactt taataccctc gtggataaca tatctgtgga tcctgagaca ggagaccttt 22860

gggttggatg ccatcccaat ggcatgaaaa tcttcttcta tgactcagag aatcctcctg 22920

catcagaggt aagttttaaa aatgaaccag ataagtgact ttattcccag ctactgtttt 22980

ttcagttgac aacataattc tcaattcttg gttttttttt tttttttttt ttttttgtga 23040

cagagtatcg ctctgtcgcc caggctggag tgcagtggcg caatcttggc tcactgcaag 23100

ctccgcctcc cgggttcaca ccattctcct gcctcagcct cccaagtagt tgggaccaca 23160

ggcgcatgcc accatgcctg gctaattttt tgtatttttt ttttttttta gtagagacag 23220

ggtttcatcc tgttggccag gatggtcttg atctgctgac cttgtgatcc acccgcctcg 23280

gcctcccaaa gtgccgggat tacaggcgtg agccaccgtg cccggccaat tctcaattct 23340

atgtgcaaat aaaagggggg acatggatag atgatccaaa tgatcagaaa tgaaggcttt 23400

tggaggtgct ttagtcagag tctaggcaag aaacacatgg cacacttaaa ttggataatt 23460

tgaggggagt ttaatcaacg gactatacac aaaatgtgtg gacacagtgt agggtaatca 23520

acagagttat aatgcagtac cccagggcta gcaagggagg gaggaaatga ataccagaag 23580

ccagaacagg tagacggtgg tgggtggaga gggttgcctg acaggaactt ggcctctggt 23640

taagggatcc tcaaagaaca taatgatgcc atactgctat ggtttaatca ttcattacct 23700

gccttcctat tcaactcccc cttcctgtgt ggcggtatac gcacaggtcc atgcaatgga 23760

ccagtcctgg agcacactgg ccagcctctc tagatcttcc tgcaggtggc aagataaata 23820

gctggaatta ttctggctta gaagatctca tcttccacct tagttacagt actcaaaggt 23880

cccatttttt gtccacggtc tttattcagt gaactatttt gggtacaagt aagaatttgc 23940

aacctacttc aatataggta tttgcacatt tactgagttt tcagtatcta ctaaggatac 24000

ttattactta catcatatat atgatatcat cttgacatat ttgtctactg ctgtgtattg 24060

ttaaagctca gttttaagag tccatttcac ctgcccaaca ggaaaaaaaa aagtgttaaa 24120

gcccgtatct cttgggttaa aaaaaaaaga aacaaatgtg tatttccatt tacattatgg 24180

ctctcttacc ttttatctct aggtttgcag gacaattgtg atcatgttta atggcaaccc 24240

tttgtgcctt ttgctagttt ttagctatta gggttgctga atggtatgtt taatagaatt 24300

gaagaaatgt atgctgtgga gtgttcagtg ctcagaactg agcagaatgt tagattctaa 24360

tttgttcttg cttgttagct catttagtaa ccttgggtca tttgctatag catggacttg 24420

gtttgttgtt ctggagaaaa tacttcattt ctaatgattg atctgcaaag gagtagcaaa 24480

aagtagtaac aattccttat ccaatgcact gcccctagat gagtgattta gttttattaa 24540

caagaagctt agctaagcca aggtgtacca tcaactattt ccctgtaact actacatgtt 24600

aatgctatgg caacttccta agacatctgt ctttggtgcc agctgttttt aagaggtcat 24660

tttgaacgtt cccacagacc cttttatatc cacgcagtgg ccttcaccgt ctgccacatt 24720

tgtgatgtag atatcctttc ttcaagtcat tatatattat cataaaggct taataataga 24780

aagctagagc tgaaggggaa tttagagata atctagtccc acccatttca ttctacagat 24840

gagaaacaaa tgtgagtgga agatccaaga catgacccag gtctcccacc tcagtgtagt 24900

gcttttcata ctaagttgaa tcacacgcta ttgctatttt ttacagtcag aatgatcaga 24960

tattggctat ttcatatggt tggcttaaca tgatactaca ctgttattat tgatacaatc 25020

acatttctcc tcaattaagt gaaggaaact gaacttacaa aatcttatac atttagagac 25080

aggaggttaa gttcagaaca catgagtatg gtacacagat atggtttttt gagggccaca 25140

ttttttttct tgtctgcatt ttaaaaacca ggaattaagt gtaaacatcc agttagttcc 25200

ctggcttacc tgaaaataca tgaaaacgga ccttaattac tgcatggcaa cagtttggct 25260

attgctgagt gtattagtca aggttcccta gagggacaga actaatagga tatatatata 25320

tgtaaagggg gtttattaag tattaactta cctgatcaca agatcccaca gtaggcggtc 25380

tgcaagcctg aggagcaagg agagccagtc tgagtctcaa aactgaagaa tttggagtct 25440

gatgtttgag ggcaggaagc atcagcatgg gagaaagatg taggctggga gggtaggcca 25500

gtctctcctc ttcatgtttt tctgcctgct ttatattcgc tggcagctga ttagattgtg 25560

cccacccaga ttaagggtgg gtctgccttt tgcagcccac tgactcaaat gttaatctcc 25620

tttggcaaca cgctcacaga cacccaggat caatactttg catccttcaa tccaatcaag 25680

ttgactctca gtattaacta tcatactgag taacagcttc ctccctccac atttactgaa 25740

atccccactg ctccctaatg tcccacatcc aggatgcgct gctcatttgc atttcctgcc 25800

tcatccctgt aggtctagat actctccacc tctgctaaag atcacttgag atcagtaccc 25860

acctgttccc aaatgaagaa acaggtgtgt taagtgtaat tttggaacaa aataggattt 25920

atctcattgt ataagattat atggcttctt atggaggatg accctattat gcataaggtt 25980

gttttaagtg acttaacaaa aatattaacc caatgttatt tcttccacag gtgcttcgaa 26040

tccagaacat tctaacagaa gaacctaaag tgacacaggt ttatgcagaa aatggcacag 26100

tgttgcaagg cagtacagtt gcctctgtgt acaaagggaa actgctgatt ggcacagtgt 26160

ttcacaaagc tctttactgt gagctctaac agaccgattt gcacccatgc catagaaact 26220

gaggccatta tttcaaccgc ttgccatatt ccgaggaccc agtgttctta gctgaacaat 26280

gaatgctgac cctaaatgtg gacatcatga agcatcaaag cactgtttaa ctgggagtga 26340

tatgatgtgt agggcttttt tttgagaata cactatcaaa tcagtcttgg aatacttgaa 26400

aacctcattt accataaaaa tccttctcac taaaatggat aaatcagtta tgtcaattgt 26460

cagatattaa ataacagtgt gtgaccccaa aagtacttac cctaaaacat gtgttgcctg 26520

gaagcacatg tgtgtatcgc tgccttgcca tgtcttgttc agaagacaca ggggagcagg 26580

gttagctcac gtgtctttag aactccagta ctcacccagg gactccagtt cacaggccag 26640

aaaacatatg cattatgaag ttcccctcta ctccatgcac atagtaagtc tgactatggc 26700

agtcagactt acttactccc attttccctt cgatatatga ctttttctca gtaaatatta 26760

acctgaatta ttccaactcc ccttgtactc ttgctttttc aattctcctg ttgcaatgac 26820

acataggaaa atcttaaaat tcttgggagt gttgtcacac ctgaaattta tgagtctcta 26880

tgatcttggc acaaattgta catttgagtg tctttgactt ggttaaagga agtttgttca 26940

cttcgatgac tggatacaga atgaatccca taattgacat gggcgaagct aaaagtgtcc 27000

ccaaagacta cactgttgtt gaggtggtgg tagtgctggt gggtttttgt ttaatattta 27060

aacttcttgt tgtggaggct gaaaagaaaa aaaataatag aaaggtaaac aaacaaataa 27120

atagaaaaga tcaacaaccc ctttggctat ctactgagac atgactagga agaaaacatg 27180

actttatcat tttgttatag aaactgatat ataaaggtta cacattttca tttatttgtt 27240

tttctgattt gaaggtataa ccttcatgat gaattacttc ttcagggtgt taaggcagtg 27300

actttagaaa caaatttttt tcttgttttt gttttgtttt tgagaccgaa tctcactctg 27360

ttgcccaggc tggagtgcag tggtgcgatc ttggctcact gcaacttcta cctccgaggt 27420

tcaagagatt cttgtgcctc agcctcccgg atagctggga ctacaggcac acaccaccat 27480

gcccggctaa tttttgtatt tttagtagag acggggtttc accatgttgg ccagcctggt 27540

ctcgaacacc tgacctcagg tgatccaccc gccttggcct tccaaagtgc tgggattaca 27600

ggtgcgagcc accacaccca gccagaaacg aagtttttct aaggcacttg ttatgatgat 27660

agtctggcat ttccgggaaa atgttttgcc aataaattga gaaccaccaa cagttgttta 27720

agaatcactg acttgaagta ataaacagga acattttgtt tgggtcaaaa aaccacttta 27780

ttcacagcaa gattggagtt actcatgatt ccagtgacat aatatcccgt gccctacatg 27840

ggctcaattt ttctgagatg ctgctcacag cccaggatgc tgggttctct gaaagactgt 27900

cttgaaatta tcaatgagtg tgatgcttag ggaacacccc caacatattt cattatttag 27960

aaagaaagaa tttcctgagt agttaggctc cctaacatct tcaataaaat gaccacattc 28020

tgttctttct tgttcttgat gatagataag caaattc 28057

## Description

The present invention relates to a specific set of circulating protein and metabolite biomarkers for the diagnosis of Brugada Syndrome (BrS) in a human being and relative methods of detection. The invention further relates to mutated *Prg4, Epx* and *Pon1* genes and related proteins for the prediction and diagnosis of Brugada Syndrome.

The Brugada Syndrome (BrS) is an inherited arrhythmogenic disease defined by a coved-type ST-segment elevation in the right precordial leads on the electrocardiogram and increased risk of sudden cardiac death (SCD) in patients with structurally normal hearts [1-4]. Brugada Syndrome has been reported to be responsible for 5-40% of sudden cardiac deaths and is an important cause of death in individuals aged <40 years [5-7], even if it occurs also in infants and children [1]. The syndrome is endemic in Asiatic regions [6] and appears to be 8 to 10 times more common in men than women.

The syndrome typically manifests with cardiac arrest or syncope, occurring in the third and fourth decade of life [3, 4, 6], however, the majority of patients are asymptomatic with structurally normal heart, and they are usually diagnosed incidentally.

The risk stratification of Brugada Syndrome, particularly in asymptomatic patients, still represents a clinical challenge.

It was demonstrated that the extent of the arrhythmic substrate is the only independent predictor of inducibility of ventricular fibrillation (VF) and ventricular tachycardia (VT) and might be considered as a new marker for risk stratification and therapy [8].

Currently, subjects with spontaneous type 1 ECG pattern and aborted sudden cardiac death or syncope of arrhythmic origin are at highest risk for future arrhythmic events and are advised to receive an implantable cardioverter defibrillator (ICD), despite the risk of device-related complications and inappropriate shocks.

To date Brugada Syndrome diagnosis is based on the identification of the typical ECG-trace signal that may be spontaneous (type 1) or can be induced by a pharmacological challenge test with intravenous administration of sodium channel blockers, such as ajmaline or flecainide. To be considered as diagnostic for BrS, the pattern seen on the ECG includes coved-type ST elevation in the right precordial leads, mainly V1 and V2 placed in the high right intercostal spaces (from the second to the fourth).

The ajmaline challenge is the most accurate test to-date to diagnose Brugada Syndrome in individuals without a spontaneous type 1 ECG pattern. In patients with normal cardiac cells, ajmaline has little or no effect on the ECG. The drug has proven to be a powerful tool in unmasking the Brugada type 1 pattern [10] and to be more accurate than flecainide, which is associated with a 36% false negative rate [11]. However, ajmaline administration can provoke VT/VF during the diagnostic procedure. As a result, the Brugada diagnostic test with ajmaline challenge may be also not allowed in several countries as screening test for safety reasons. Moreover, given the risk associated with the procedure, patients themselves refuse to undergo the procedure. Additionally, the procedure requires an experienced staff, in hospitals with an Extra Corporeal Membrane Oxygenation (ECMO) team. Therefore, this procedure is not widely available, implying that patients may need to travel long distances for the procedure. From an economical perspective, the procedure is expensive and a considerable burden for Public National Healthcare System. As a result, potential patients do not undergo the diagnostic test in a timely fashion, or at all, exposing them to considerable risk of sudden cardiac death. Moreover, Brugada Syndrome has a further not negligible genetic treat: it is a heterogeneous channelopathy, inherited as an autosomal dominant trait with incomplete penetrance [9].

To date, about three hundred pathogenic variants in 25 genes have been identified in subjects with Brugada Syndrome, but most of them (25-30%) involve the *SCN5A* gene on chromosome 3p22, encoding the α-subunit of the cardiac sodium channel NaV1.5 [10]. Other genetic lesions associated with Brugada Syndrome include heterozygous variants in *CACNA1C, SCN10A, PKP2, TRPM4, KCNH2,* and at least eighteen other genes. *SCN5A* heterozygous mutations account for 11-28% of all Brugada Syndrome cases [11].

However, about 90% of patients with Brugada Syndrome do not have a mutation in the SCN5A gene.

To further complicate the genetic assessment, there are different related Brugada Syndromes caused by mutations in genes other than the SCN5A gene. For example, Brugada Syndrome-2 (Phenotype MIM #611777) is caused by mutation in the GPD1L gene. Brugada Syndrome-3 (Phenotype MIM #611875) and Brugada Syndrome-4 (Phenotype MIM #611876), the phenotypes of which include a shortened QT interval on ECG, are caused by mutation in the CACNA1C and CACNB2 genes, respectively. Brugada Syndrome-5 (Phenotype MIM #612838) is caused by mutation in the SCN1B gene. Brugada Syndrome-6 (Phenotype MIM #613119) is caused by mutation in the KCNE3 gene. Brugada Syndrome-7 (Phenotypte MIM #613120) is caused by mutation in the SCN3B gene. Brugada syndrome-8 (Phenotype MIM #613123) is caused by mutation in the HCN4 gene. While the scientific community still regards Brugada Syndrome as an ion channelopathy, further evidence suggests that the syndrome may also originate by additional factors. For example, an alpha-tropomyosin heterozygous mutation appears to link HCM and Brugada Syndrome in one study, suggesting a role for sarcomeropathies [12]. Another study, published by the present inventors, demonstrated for the first time a pathogenic variant in the *MYBPC3* gene and its potential association with Brugada Syndrome [13]. In fact, altered sarcomeric properties have been directly implicated in arrhythmogenesis and sudden death [14-16].

In the light of the complexity of this multi-factorial disorder and the existence of many genetic variants of the disease at the molecular level, genetic testing may be used just to confirm a clinical diagnosis of Brugada Syndrome but is not suitable to make diagnosis of the disease in any subject.

These limitations of genetic screening highlight the urgent need to seek biomarkers aimed both at confirmation of the Brugada Syndrome to establish a prognosis in already affected patients.

In this direction, the systematic introduction of active screening in unmasking Brugada Syndrome using sodium-channel blockers, has considerably increased the diagnosis and incidence of the disease, although its true prevalence is still unknown [17]. Nonetheless, the drug challenge used for the diagnosis can be associated with the occurrence of potentially life-threatening ventricular arrhythmias, thus limiting its widespread use in the absence of a safe hospital environment.

In view of the foregoing, there is a need for a more time and cost-efficient, sensitive and non-invasive laboratory test for diagnosing Brugada Syndrome, which could eventually replace the drug challenge, or at least limit it to those individuals that are positive to such initial laboratory screening and overcome the limits of genetic screening.

The authors of the present invention have now identified a set of circulating biomarkers that allows to overcome the different technical problems affecting the prior art, providing a reliable and sensitive method for the diagnosis of Brugada Syndrome in any patients, herein included also asymptomatic subjects, patients without previous family history of Brugada Syndrome and patients without previous family history of sudden cardiac death.

Specifically, the authors found that patients affected by Brugada Syndrome possess signs of the disease that are detectable in the peripheral blood. In particular, based on the increasing number and diversity of the mutated genes found in patients affected by Brugada Syndrome, the authors hypothesized that the disease could affect cellular and sub-cellular compartments (such as mitochondria) other than those present in the heart. To this end, considering the complexity of the pathology and the heterogeneity among the patient population, they applied a multi-omics (genomic, proteomics, metabolomics and lipidomics) characterization of peripheral plasma and peripheral blood mononuclear cells (PBMCs) on a large scale, supported by an extensive and integrated bioinformatics and Machine Learning (ML) approaches, leading to the identification of a univocal set of biomarkers, statistically significant related to Brugada Syndrome.

A significant advantage of the use of the set of biomarkers of the invention includes the diagnostic and prognostic utility in a wide set of subtypes of Brugada Syndrome, regardless of the genotype or expression of other known genetic biomarker, such as the SCN5A gene.

The set of biomarkers of the invention may also be very useful for risk certification of the patients with Brugada syndrome. Furthermore, a positive result with the set of biomarkers of the invention allows to identify patients affected by Brugada disease, independently from being symptomatic or not and independently of previous family history of Brugada syndrome or previous family history of sudden cardiac death (SCD).

Therefore, it is an object of the present invention a set of 10 biomarkers (core-set) comprising at least:
i) the subset of 7 proteins listed in the following Table 1:

**TABLE 1**

| **Protein** | **UniProtKB ID** |
|---|---|
| Fibronectin 1 (FN1) | P02751 |
| Inter-alpha-trypsin inhibitor heavy chain 3 (ITIH3) | Q06033 |
| Proteoglycan 4 (PRG4) | Q92954 |
| Adaptor related protein complex 2 subunit sigma 1 (AP2S1) | M0QYZ2 |
| Component of oligomeric Golgi complex 5 (COG5) | Q9UP83 |
| Eosinophil peroxidase (EPX) | P11678 |
| Ribosomal oxygenase 1 (RIOX1) | Q9H6W3 |

ii) the subset of the following 3 metabolites:
- fumaric acid;
- O-palmitoleoylcarnitine;
- L-Glutamic acid;
for the diagnosis of Brugada syndrome in a human being.

The core-set of 10 biomarkers according to the present invention allows a diagnosis of the disease of Brugada syndrome on PBMC/plasma of any subject with an overall average accuracy of about 74% and mean area under the curve (AUC) of about 0.81.

The subset of the invention may further comprise additional biomarkers belonging to the above listed subsets i)-ii) of protein and metabolites.

Therefore, it is another object of the present invention the set of 10 biomarkers further comprising one or more of the following additional biomarkers:
i) a protein selected from the group listed in the following Table 2:

**TABLE 2**

| **Protein** | **UniProtKB ID** |
|---|---|
| Paraoxonase 1 (PON1) | P27169 |
| Aldehyde dehydrogenase 3 family member B1 (ALDH3B1) | P43353 |
| Solute carrier family 25 member (SLC25A5) | P05141 |
| S100 calcium binding protein A11 (S100A11) | P31949 |
| Phospholipase C beta 2 (PLCB2) | Q00722 |

and the metabolite 2-hydroxydecanoate;
or a combination thereof.

Preferably, all the additional biomarkers above listed are included in the set of biomarkers according to the invention, albeit also intermediate solutions wherein only some of them are included are contemplated in the scope of the present invention.

Therefore, according to a further preferred embodiment it is another object of the present invention a set of 16 biomarkers (extended-set) comprising:
i) the subset of 12 proteins listed in the following Table 3:

**TABLE 3**

| **Protein** | **UniProtKB ID** |
|---|---|
| Fibronectin 1 (FN1) | P02751 |
| Inter-alpha-trypsin inhibitor heavy chain 3 (ITIH3) | Q06033 |
| Proteoglycan 4 (PRG4) | Q92954 |
| Adaptor related protein complex 2 subunit sigma 1 (AP2S1) | M0QYZ2 |
| Component of oligomeric Golgi complex 5 (COG5) | Q9UP83 |
| Eosinophil peroxidase (EPX) | P11678 |
| Ribosomal oxygenase 1 (RIOX1) | Q9H6W3 |
| Paraoxonase 1 (PON1) | P27169 |
| Aldehyde dehydrogenase 3 family member B1 (ALDH3B 1) | P43353 |
| Solute carrier family 25 member (SLC25A5) | P05141 |
| S100 calcium binding protein A11 (S100A11) | P31949 |
| Phospholipase C beta 2 (PLCB2) | Q00722 |

and
ii) the subset of the following 4 metabolites:
- fumaric acid;
- O-palmitoleoylcarnitine;
- L-Glutamic acid;
- 2-hydroxydecanoate;
for the diagnosis of Brugada Syndrome in a human being.

Using the 16 biomarkers (extended-set) on PBMC/plasma cell populations in different patient subgroups, the overall accuracy of the test is 75%-80% and the AUC is 0.83-0.89, as reported in Table 4 of Example 1.

According to a preferred embodiment of the invention said biological sample is selected from the group consisting of whole blood, plasma, serum, peripheral blood and PBMCs. Preferably, the biological sample is plasma and/or PBMCs. According to alternative embodiments, said biological sample can come indifferently from adult subjects or children, male or female subjects.

In a preferred embodiment of the invention said human being may be either asymptomatic (that is with structurally normal heart) or at high risk for Brugada Syndrome due to family history, previous events of heart atrial and/or ventricular fibrillation, diabetes or obesity.

It is another object of the present invention an in vitro method for detecting the presence or measuring concentration of the occurrence of one of the set of biomarkers above listed in a biological sample of a human being.

In order to provide a reliable result, the in vitro method should at least foresee the detection of the presence or measuring concentration of at least the 10 core-set of biomarkers up to the 16 extended set of biomarkers.

In a particular embodiment the invention provides an in vitro method for detecting the presence of one of the set of biomarkers above listed in a biological sample of a human being, comprising the following steps:
a) detection of at least 7 up to 12 proteins of subset i) by an assay selected from the group comprising Western Blot, Dot blot, ELISA, flow cytometry, enzymatic activity assay, targeted MS, multiplex protein analysis, ProQuantum high-sensitivity immunoassays, or a combination thereof;
b) detection of at least 3 up to 4 of the metabolites of subset ii) by an assay such as one selected from the group comprising MS, GC-MS, fluorimetric or colorimetric assays, NMR, spectroscopy with nanoprobes, Enzyme-Linked oligonucleotide assays, or a combination thereof;
wherein the positive detection of all the biomarkers compared to a normal control allows the identification of patients affected by Brugada Syndrome.

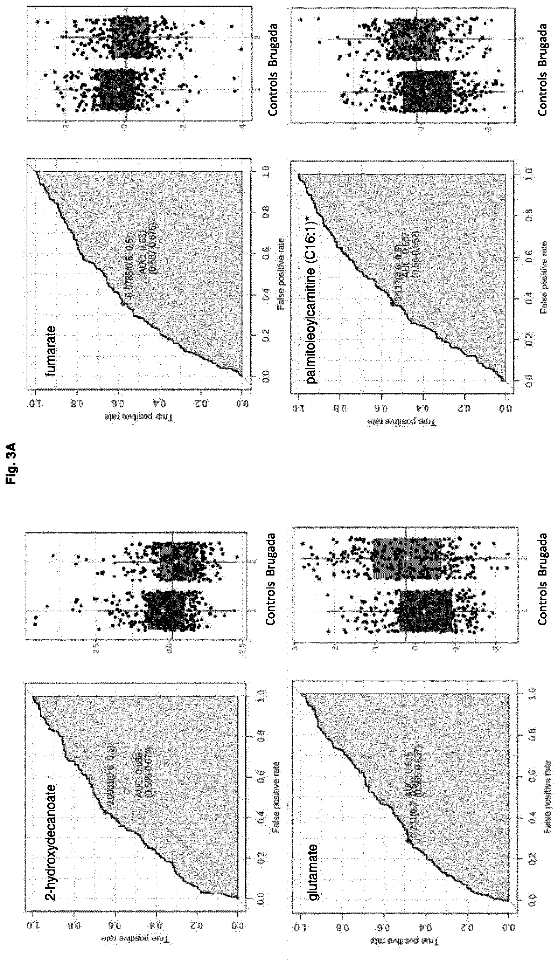

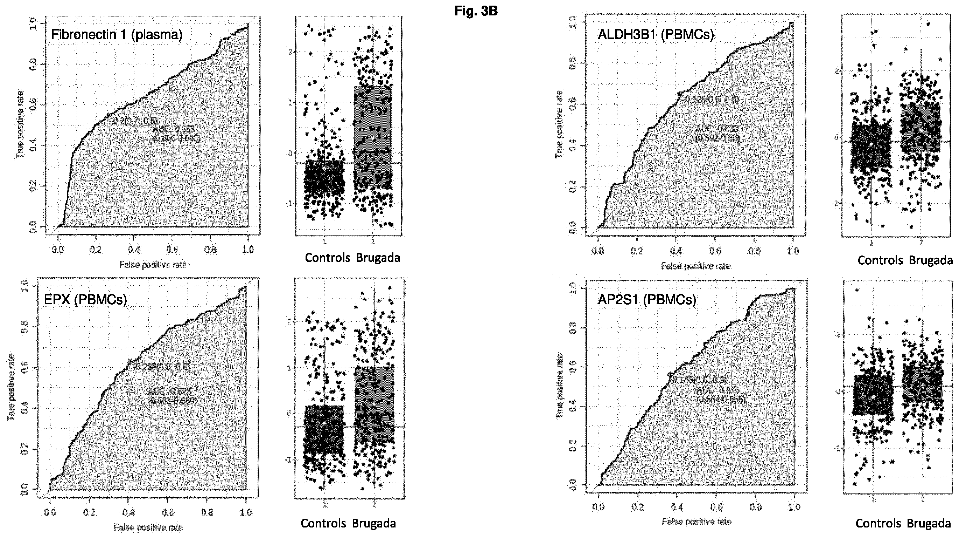

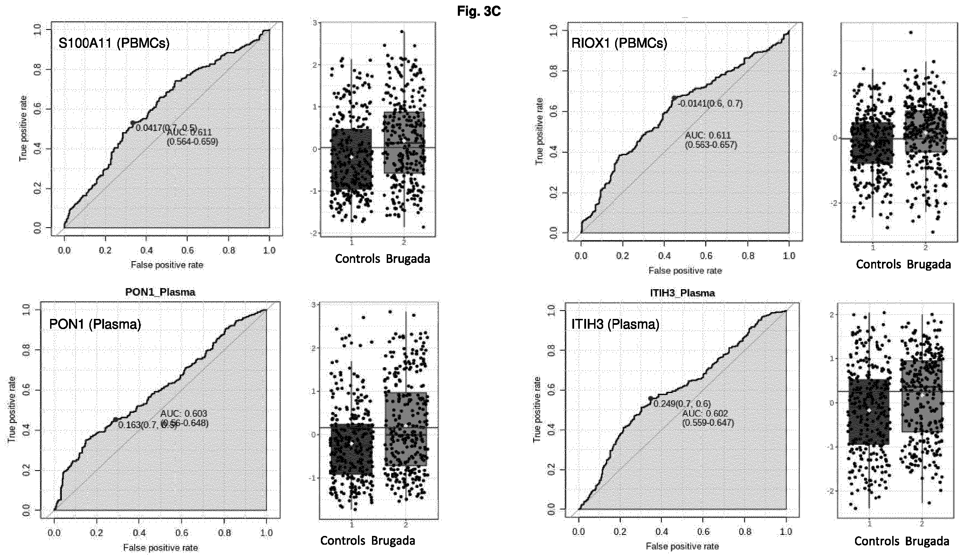

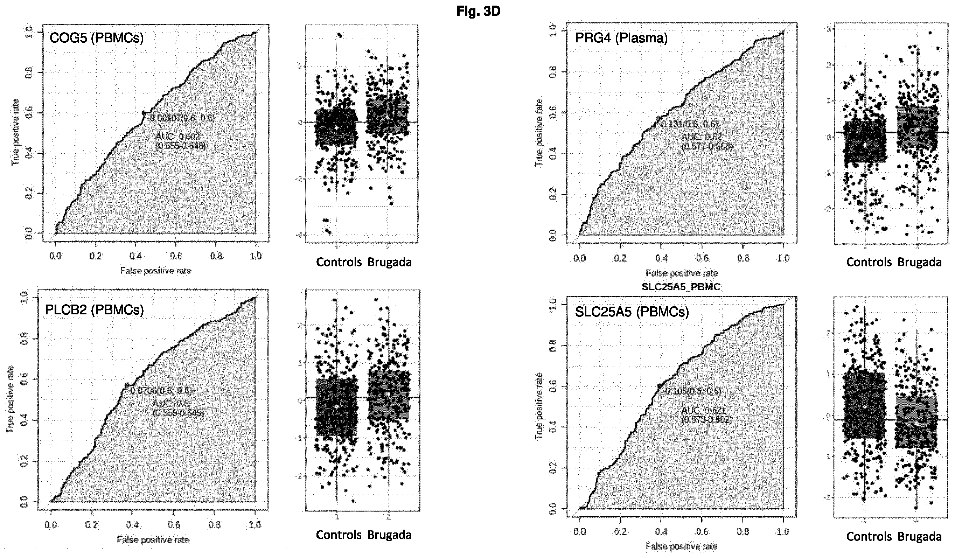

According to an alternative embodiment the invention relates to an in vitro method for measuring the concentration of one of the above listed set of biomarkers in a biological sample of a human being, comprising the following steps:
a) quantitative determination of at least 7 up to 12 proteins of subset i) by a quantitative technique such as Western Blot, ELISA, enzymatic activity assay, targeted MS, multiplex protein analysis, ProQuantum high-sensitivity immunoassays, or a combination thereof;
b) quantitative determination of at least 3 up to 4 of the metabolites of subset ii) by a technique such as HPLC, MS, NMR analysis, ELONA, or a combination thereof;
wherein an increase or decrease of the concentration of the biomarkers compared to a normal control indicates a diagnosis of Brugada Syndrome (as in Figure 3).

A combination of all biomarkers means that can predict the patient is affected by Brugada Syndrome, independently on the fact that such patient is asymptomatic or symptomatic.

According to a preferred embodiment of the invention said biological sample is selected from the group consisting of plasma, PBMCs serum, peripheral blood and whole blood, or a combination thereof. Preferably, the biological sample is plasma and/or PBMCs. According to alternative embodiments of the afore mentioned method, the starting biological sample can come indifferently from adult subjects or children, male or female subjects.

In a preferred embodiment of the invention said human being may be either asymptomatic (that is with structurally normal heart) or at high risk for Brugada Syndrome due to family history, previous events of heart atrial and/or ventricular fibrillation, diabetes or obesity.

More preferably said human being is about 40 years old or younger.

The invention is further directed to a kit for the detection of the subset i) of at least 7 proteins of Table 1 up to the subset i) of 12 proteins of Table 3, said kit comprising antibodies specific for anyone of the protein of the subset i), wherein said antibodies are labelled or attached to a solid support.

Preferably, said antibodies are labelled with fluorescents.

Said solid support is preferably a multi-well plate. Preferably, said kit is an ELISA kit. In a preferred embodiment, cell detection after incubation with antibodies of the invention is carried out by flow cytometry.

The present invention further relates to an oligonucleotide sequence of *Prg4* gene (Gene Ensemble ID ENSG00000116690; SEQ ID NO:1) characterized by the presence of a deleterious mutations C→T in position 186,304,862 of exon 6 the nucleotide sequence, said oligonucleotide sequence being RNA or DNA, for use as a genetic diagnostic marker for the diagnosis of Brugada Syndrome in a human being. Preferably said mutated oligonucleotide sequence comprises or consists of SEQ ID NO: 7.

It is another object of the present invention a mutated PRG4 protein encoded by the above mentioned mutated oligonucleotide sequence of *Prg4* gene, for use as a genetic diagnostic marker for the diagnosis of Brugada Syndrome in a human being. In a particular preferred embodiment, said mutated PRG4 protein comprises or consists of SEQ ID NO:2, wherein the amino acid change R→W in position 180 occurs.

The present invention relates to an oligonucleotide sequence of *Epx* human gene (Gene Ensemble ID ENSG00000121053; SEQ ID NO:3) characterized by the presence of a deleterious mutations G→C in position 58,193,733 of exon 4 of the gene sequence, said oligonucleotide sequence being RNA or DNA, for use as a genetic diagnostic marker for the diagnosis of Brugada Syndrome in a human being. Preferably said mutated oligonucleotide sequence comprises or consists of SEQ ID NO:8.

It is another object of the present invention a mutated EPX protein encoded by the above mentioned mutated oligonucleotide sequence of *Epx* human gene, for use as a genetic diagnostic marker for the diagnosis of Brugada Syndrome in a human being. In a particular preferred embodiment, said mutated EPX protein comprises or consists of SEQ ID NO:4, wherein the mutation Q→H occurs in position 122. Another object of the present invention relates to an oligonucleotide sequence of *Pon1* human gene (ENSG00000005421; SEQ ID NO:5) characterized by the presence of a deleterious mutations A→T in position 95,316,772 of exon 3 of the gene sequence, said oligonucleotide sequence being RNA or DNA, for use as a genetic diagnostic marker for the diagnosis of Brugada Syndrome in a human being. Preferably said mutated oligonucleotide sequence comprises or consists of SEQ ID NO: 9.

It is a further object of the present invention a mutated PON1 protein encoded by the above mentioned mutated oligonucleotide sequence of *Pon1* human gene, for use as a genetic diagnostic marker for the diagnosis of Brugada Syndrome in a human being. In a particular preferred embodiment, said mutated PON1 protein comprises or consists of SEQ ID NO:6, wherein the amino acid change L→M in position 55 occurs.

The invention further relates to the use of one or more of the mutated oligonucleotide sequences or mutated proteins as above described, for the diagnosis of Brugada Syndrome in a human being by detection in a biological sample, wherein said biological sample is selected from the group consisting of plasma, PBMCs, whole blood, serum and peripheral blood, or a combination thereof. Furthermore, the invention provides an in vitro method for detecting the presence of one or more of the oligonucleotide sequences of *Prg4, Epx* and *Pon1* genes above detailed by genetic analysis through PCR or DNA sequencing.

Finally, one further object of the invention is a kit comprising primer or probes complementary to one or more of the oligonucleotide sequences of *Prg4, Epx* and *Pon1* genes above detailed for the diagnosis of Brugada Syndrome in a human being.

Therefore, given the role of several biomarker proteins dysregulated in Brugada Syndrome patients in metabolic pathways and disease (e.g., PRG4, PON1), alcohol metabolism (e.g., ALDH3B1) and the direct identification of dysregulated metabolites of the mitochondrial TCA cycle (e.g., fumarate), a causal link between these metabolic processes dysregulated and Brugada Syndrome is the central discovery of the present invention. The further identification of several biomarker proteins to be mutated in Brugada Syndrome patients further suggests a causal link with the disease.

The present invention will now be described, for non-limiting illustrative purposes, according to a preferred embodiment thereof, with particular reference to the attached figures, wherein:

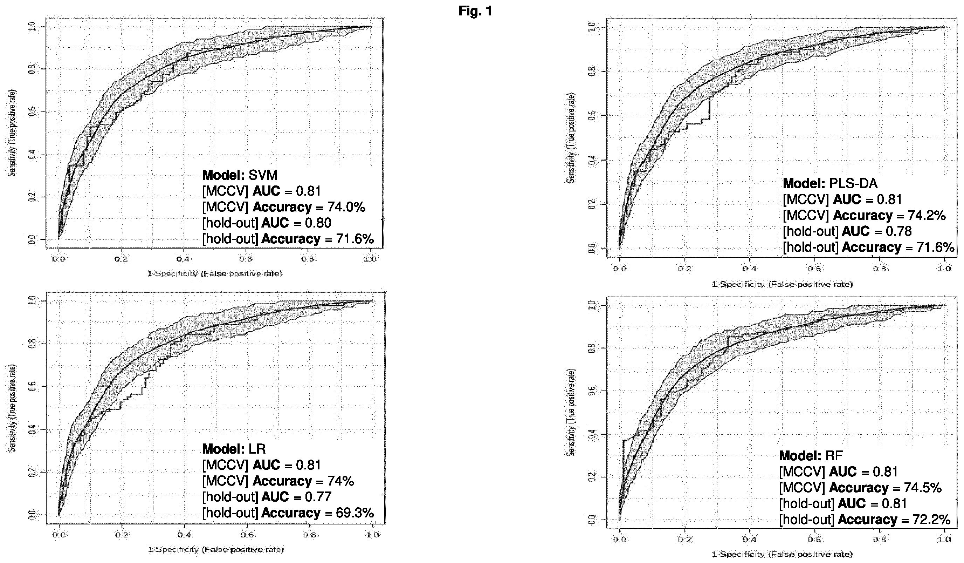

- Figure 1 shows accuracy and AUC estimates of the core-set of 10 biomarkers of the invention by 4 independent models (SVM, PLS-DA, RF and LR). For each model, Monte Carlo cross-validation (MCCV) with 100 runs was applied, using, in each run, different random sets of 67% (2/3) of patients for training and 33% (1/3) for testing. The AUC curve obtained by the MCCV method is indicated by blue colour, where the 95% confidence interval (95% CI) of the AUC is estimated by 100 MCCV samples. In addition, the AUC curves and AUC estimates using the hold-out method were reported, where 409 subjects (70%) were used as training-dataset and 176 subjects (30%) were used as test-dataset. The AUC curve obtained by the hold-out method is indicated by magenta colour.

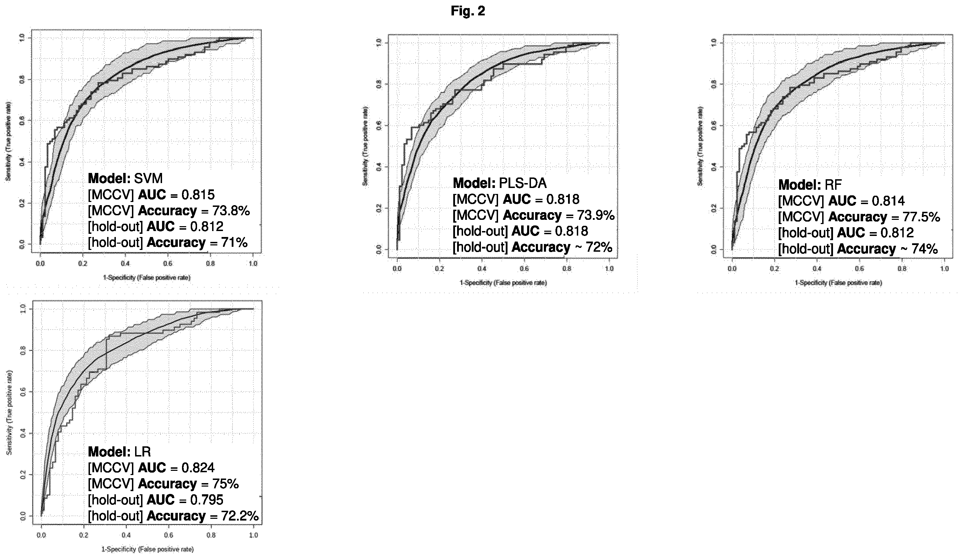

- Figure 2 shows the accuracy and AUC estimates of the extended-set of biomarkers of the invention by 4 independent models (SVM, PLS-DA, RF and LR). For each model, Monte Carlo cross-validation (MCCV) with 100 runs was applied, using, in each run, different random sets of 67% (2/3) of patients for training and 33% (1/3) for testing. The AUC curve obtained by the MCCV method is indicated by blue colour, where the 95% confidence interval (95% CI) of the AUC is estimated by 100 MCCV samples. In addition, the AUC curves and AUC estimates using the hold-out method were reported, where 465 subjects (79%) were used as training-dataset and 120 subjects (21%) were used as test-dataset. The AUC curve obtained by the hold-out method is indicated by magenta colour.
- Figures 3A-D show the AUC and box plots in PBMCs and plasma for each metabolites (3A) and protein (3B-D) belonging to the biomarker core-set and extended-set for the diagnosis of Brugada Syndrome.
- Figure 4 shows the dysregulation of protein and metabolites of the TCA cycle in Brugada Syndrome.
- Figure 5 shows dysregulation of SLC25A5 and VDAC1 mitochondrial proteins in Brugada Syndrome. Both SLC25A5 and VDAC1 proteins are essential for ATP/ADP transport activity and energy metabolism by mitochondria.
- Figure 6 shows protein-protein interaction (PPI) network derived from STRING v11.0b (https://string-db.org/) of the 995 dysregulated proteins in blood PBMCs from Brugada Syndrome patients (FDR ≤ 5%), showing the 79 proteins significantly enriched in "Metabolic Pathways" (KEGG) (FDR = 0.00099).
- Figure 7 shows the dysregulated proteins in patients with Brugada Syndrome that are part of the ethanol metabolism pathway resulting in production of ROS by CYP2E1. plateau reached by the curve of the accuracy of the test when more than 60 features have been employed.
- Figure 8 shows a graph illustrating the number of deleterious mutations at the EPC, PON1 and PRG4 genes encoding for the biomarker proteins (extended set, listed in TABLE 2) and number of Brugada Syndrome patients (out or 186 tested) carrying at least one damaging deleterious mutation in each gene. For comparative purposes, the number of damaging deleterious mutations at SCN5A (i.e., the gene in which mutations are most commonly found in Brugada Syndrome) that have been detected in the 186 Brugada Syndrome patients tested by WGS analysis was reported.

The following examples are merely illustrative and should not be considered limiting the scope of the present invention.

### EXAMPLE 1: Accuracy of the test

Accuracy and AUC have been independently estimated for the core-set and the extended-set of biomarkers using 4 ML methods: Support Vector Machine (SVM), partial least squares discriminant analysis (PLS-DA), random forest (RF) and logistic regression (LR), which provided very similar and highly consistent estimates **(****Figure 1****).**

To assess accuracy, the Monte Carlo cross-validation (MCCV) method with 100 runs was applied, using, in each run, different random sets of 67% (2/3) of patients for training and 33% (1/3) for testing.

The core-set of 10 biomarkers according to the present invention allows a diagnosis of the disease of Brugada syndrome on PBMC/plasma of any subject with an overall average accuracy of about 74% and mean area under the curve (AUC) of about 0.81.

By increasing the number of biomarkers from 10 (core-set) to 16 (extended-set) it is possible to reach an overall accuracy of the test on PBMC/plasma population of about 74%-77% and AUC to 0.814-0.822 across methods **(****Figure 2****).**

Using the 16 biomarkers (extended-set) on PBMC/plasma cell populations in different patient subgroups, the overall accuracy of the test is 75%-80% and the AUC is 0.83-0.89, as reported in Table 4.

**TABLE 4**

| Patients subgroups | Number of patients | AUC¹ (P-value)² | Accuracy¹ |
|---|---|---|---|
| All samples | 585 | 0.842 (10⁻⁴⁶) | 75.6% |
| No previous family history of Brugada Syndrome | 231 | 0.895 (10⁻²⁴) | 78.8% |
| No previous family history of SCD | 366 | 0.883 (10⁻³⁷) | 79.5% |
| Asymptomatic patients | 366 | 0.863 (10⁻³³) | 76.5% |
| No pathogenic mutations at the SCN5A gene | 499 | 0.837 (10⁻³⁸) | 76.4% |
| "Old" patients (age >37.7 years )³ | 293 | 0.833 (10⁻²¹) | 75.4% |
| "Young" patients (age <=37.7 years)³ | 292 | 0.866 (10⁻²⁶) | 80.8% |
| ¹ AUC and accuracy are calculated by Logistic Regression model | | | |
| ² P-value of significance, where the null hypothesis is true area = 0.5 | | | |
| ³ "Old" is defined as age > median age in the patient cohort; "young" is defined as age <= median age in the patient cohort | | | |

Therefore, the set of 16 biomarkers (extended-set) have an increased accuracy and a significantly high AUC for the diagnosis of Brugada Syndrome in a human being with no previous family history of Brugada Syndrome (AUC-0.89, accuracy ∼79%) or in a human being with no previous family history of SCD (AUC-0.88, accuracy ∼79%) or in a human being with no symptoms of Brugada Syndrome (accuracy ∼76%).

The set of 16 biomarkers (extended-set) have a high accuracy (-76%) and significant AUC (-0.84) in patients with Brugada Syndrome who do not have a mutation in the SCN5A gene. The set of 16 biomarkers (extended-set) have a high accuracy for the diagnosis of Brugada Syndrome in both "young" patients (AUC-0.87, accuracy ∼81%) and "old" patients (AUC-0.83, accuracy ∼75%). A positive result related to the overall combination of biomarkers belonging to the set (being either the core set or the extended set or an intermediate set) means that the patient is affected by Brugada Syndrome, independently on the fact that such patient is asymptomatic or symptomatic. In other words, rather than a single upregulated (or downregulated) protein or metabolite, a combination of upregulated or downregulated proteins or metabolites predict the patient is affected by Brugada Syndrome, independently on the fact that such patient is asymptomatic or symptomatic.

Each biomarker metabolite or protein that defines the composite set of 16 biomarkers (extended-set) is independently predictive of the diagnosis of Brugada Syndrome, with AUC > 0.6, as depicted in **Figure 3A-D**.

The FN1 (Fibronectin 1) protein is one of the and 10 biomarkers (core-set) and of the and 16 biomarkers (extended-set) for the diagnosis of Brugada Syndrome in a human being. FN1 protein levels have been previously reported to be altered by the presence of an implantable cardioverter defibrillator (ICD) [20]. In our patient cohort (n=585), FN1 protein levels in plasma are significantly elevated (∼3 fold) in Brugada patients with ICD implant (n=91) compared Brugada patients without ICD (n=203), P=0.001 by Two-Sample Kolmogorov-Smirnov test. However, not all Brugada patients with an ICD implant have consistently high expression of FN1. Despite this, a high predictive ability of FN1 as biomarker of the diagnosis of Brugada Syndrome is retained in the subset of patients who do not have an ICD implant (n=494, accuracy -66%, AUC = 0.614, P = 10⁻⁵) with an odds ratio (OR) = 1.95 of having Brugada Syndrome associated with increased FN1 protein levels in plasma. Table 5 reports the accuracy of FN1 (fibronectin 1) biomarker in patients with and without ICD implant.

**TABLE 5**

| Patients subgroups | N | AVC¹ (P-value)² | 95% CI AVC¹ | OR [95% CI]³ | Accuracy¹ |
|---|---|---|---|---|---|
| All samples (with and without ICD implant) | 585 | 0.653 (10⁻¹⁰) | 0.606-0.693 | 1.70 [1.39-2.08] | 64.4% |
| Only patients without ICD implant | 494 | 0.613 (10⁻⁰⁵) | 0.561-0.665 | 1.95 [1.62-2.35] | 65.8% |
| **¹** AUC and accuracy for the biomarker FN1 (Fibronectin 1) are calculated by Logistic Regression model | | | | | |
| **²** P-value of significance, where the null hypothesis is true area = 0.5 | | | | | |
| **³** Odds Ratio (OR) and 95% CI of OR for the biomarker FN1 (Fibronectin 1) are calculated by Logistic Regression model | | | | | |

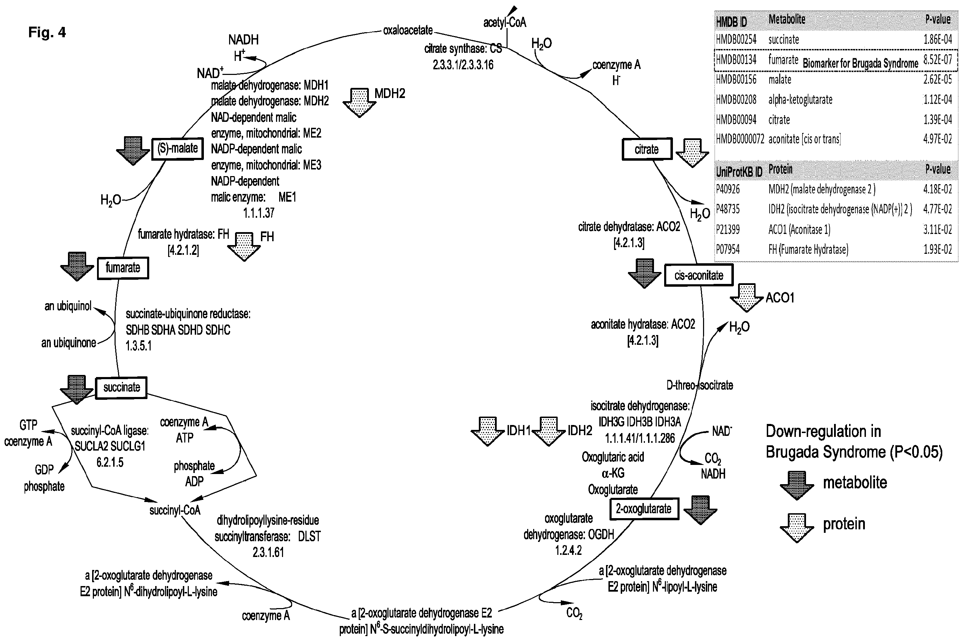

Plasma levels of fumarate (fumaric acid) is one of the and 10 biomarkers (core-set) and of the and 16 biomarkers (extended-set) for the diagnosis of Brugada Syndrome in a human being. Decreased plasma levels of fumarate are significantly associated with Brugada Syndrome (adjusted P = 8.2x10⁻⁵), suggesting a dysregulation of the TCA cycle (tricarboxylic acid cycle) in patients with Brugada Syndrome. Other proteins encoding for relevant enzymes (MDH2, malate dehydrogenase 2; IDH2, isocitrate dehydrogenase (NADP(+)) 2; ACO1, Aconitase 1; FH, Fumarate Hydratase) and metabolites (succinate, malate, alpha-ketoglutarate, aconitate, citrate) of the TCA cycle are significantly dysregulated (P<0.05) in blood from Brugada Syndrome patients, see **Figure** 4. This indicates an overall downregulation of TCA cycle in mitochondria, linking mitochondrial dysfunction to Brugada Syndrome.

The TCA cycle is a key metabolic pathway that connects carbohydrate, fat, and protein metabolism. Therefore, according to the identification of reduced levels of fumarate (a biomarker of Brugada Syndrome, Table 1, Table 5, above) and of several other metabolites (TCA cycle intermediates) and nuclear and mitochondrial proteins (enzymes of the TCA cycle in mitochondria) in Brugada Syndrome patients, a further preferred embodiment it is another object of the present invention the mitochondrial dysfunction and/or dysregulation of TCA cycle for the diagnosis of Brugada Syndrome in a human being.

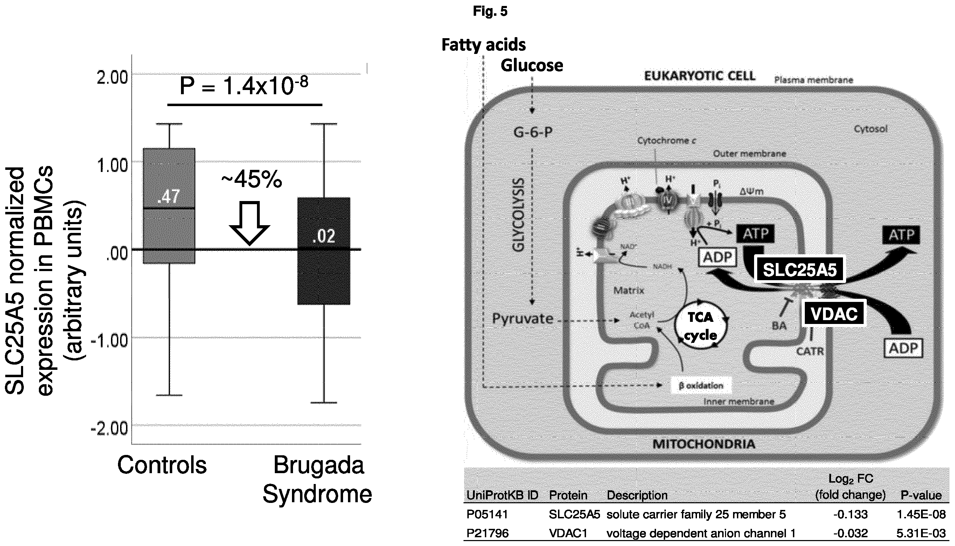

Another biomarker protein (extended-set), SLC25A5 (solute carrier family 25 member 5) also known as ANT2, is significantly downregulated (-45% on average) in PBMCs from Brugada Syndrome patients (P=1.4x10⁻⁸), and it belongs to the mitochondrial carrier family SLC25. SLC25A5 is a key mitochondrial ATP transporter that is expressed ubiquitously at a level depending on the specific respiratory activity of the tissues, including brain, lung, kidney, pancreas, heart, skeletal muscle, spleen/inner and in the mitochondrial membrane. SLC25A5 encodes for a mitochondrial carrier that is mostly localized in the inner mitochondrial membrane **(****Figure 5**); SLC25 protein family have been implicated in diverse metabolic pathways, including oxidative phosphorylation, TCA cycle, fatty acid oxidation, gluconeogenesis, lipogenesis, ketone body production and utilization, urea synthesis, amino acid degradation, regulation of nucleotide and deoxynucleotide pools in the mitochondrial matrix, heat production [21]. Together with the inner-membrane mitochondrial protein SLC25A5, the outer-membrane mitochondrial protein VDAC1 appears to be significantly downregulated in blood PBMCs from Brugada Syndrome patients; both proteins are key for ATP/ADP transport activity and energy metabolism by mitochondria **(****Figure 5**). Despite VDAC1 levels change less than those of SLC25A5 in Brugada Syndrome (Log2FC = -0.133 [SLC25A5] vs -0.033 [VDAC1]), VDAC1 location in the outer mitochondrial membrane, makes it to interact with over 100 proteins, and to orchestrate the interaction of mitochondrial and cellular activities through several signaling pathways [22].

The downregulation of both SLC25A5 and VDAC1 mitochondrial proteins in Brugada Syndrome is another object of the present invention of the mitochondrial dysfunction for the diagnosis of Brugada Syndrome in a human being.

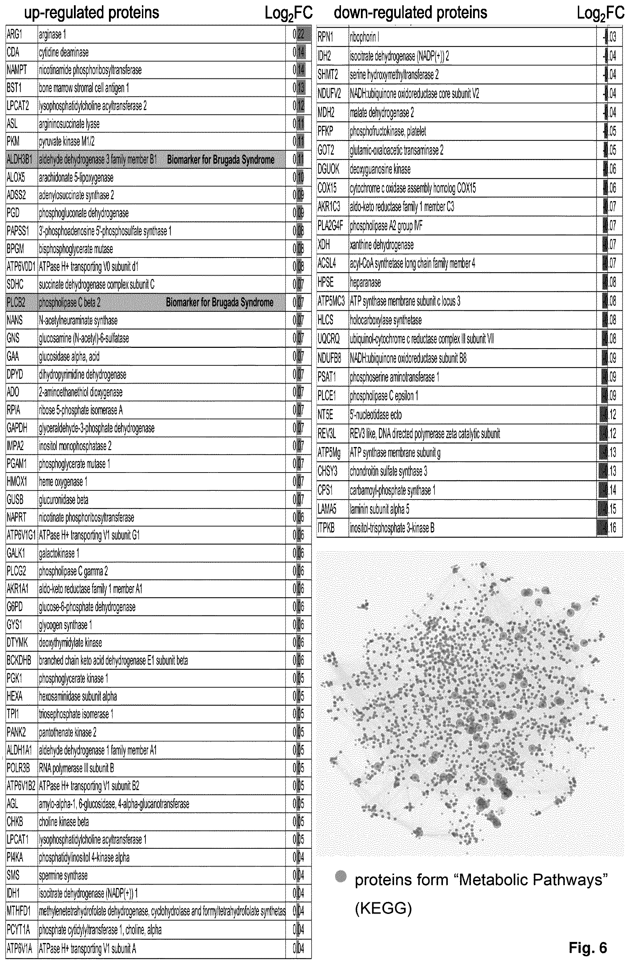

Functional analysis of the proteins that are dysregulated in blood PBMCs from Brugada Syndrome patients (n=995 proteins, false discovery rate (FDR) ≤ 5%), identified significant enrichment for 79 proteins (COX15, NANS, HMOX1, MTHFD1, NAMPT, POLR3B, GAPDH, TPI1, GOT2, LAMA5, PI4KA, NT5E, GNS,LPCAT2, DGUOK, BST1, PAPSS1, HEXA, IMPA2, PGD, ATP6V1A, ATP6V1B2, LPCAT1, RPIA, ATP5G3, ATP6V0D1, PCYT1A, AGL, RPN1, ALDH1A1, NDUFB8, ATP5L, CHSY3, GUSB, DTYMK, GAA, ASL, PANK2, GYS1, BCKDHB, PKM, MDH2, NDUFV2, IDH2, SHMT2, ACSL4, ARG1, REV3L, ADSS, SDHC, DPYD, PGAM1, PLCE1, AKR1A1, PGK1, ADO, ATP6V1G1, ALOX5, CDA, PSAT1, UQCRQ, XDH, AKR1C3, PFKP, BPGM, G6PD, PLA2G4F, HLCS, HPSE, CHKB, SMS, IDH1, NAPRT, CPS1, ITPKB, GALK1, PLCG2, PLCB2, ALDH3B1) involved in metabolism (Metabolic pathways (KEGG, https://www.genome.jp/kegg/), hsa01100), FDR = 9.9x10⁻⁴), see **Figure 6****.** This suggests a dysregulation of metabolic processes in Brugada Syndrome patients, which is in keeping with metabolic abnormalities observed at the clinical level.

Of these proteins important for metabolism, PLCB2 and ALDH3B 1 proteins are also part of the biomarker (core-set and extended-set) of Brugada Syndrome (see Tables 2- 3 above, **Figure** 3).

The PLCB2 (Phospholipase C Beta 2) protein is mainly localized to the plasma membrane and cytosol, and one of the and 16 biomarkers (extended-set) for the diagnosis of Brugada Syndrome in a human being. Compared with controls, PLCB2 protein levels are significantly elevated (Log2FC = 0.073, P = 3.3x10⁻⁶) in blood PBMCs of Brugada Syndrome patients. PLCB belongs to the PLC protein family, which are key enzymes that metabolize inositol lipids and have a pivotal role in multiple transmembrane signaling transduction pathways that modulate a series of cellular processes, including cell proliferation and mobility [23]. The PLCB family consists of 4 isozymes, PLCB1 to B4, which share their primary structure and PLCB 1 is an important molecule that regulates b cell insulin secretion and can be considered a candidate for therapeutic intervention in diabetes mellitus [24]. However, no data on the role of PLCB2 in diabetes mellitus are available, suggesting the function of PLCB2 in Brugada Syndrome is novel and object of the present invention.

The ALDH3B1 (aldehyde dehydrogenase 3 family member B1) protein is one of the 16 biomarkers (extended-set) for the diagnosis of Brugada Syndrome in a human being. Compared with controls, ALDH3B1 protein levels are significantly elevated (Log2FC = 0.105, P = 2.4x10⁻⁸) in blood PBMCs of Brugada Syndrome patients. Aldehyde dehydrogenases (ALDHs) detoxify toxic aldehydes by oxidation to the corresponding carboxylic acids. Long-chain aliphatic aldehydes are largely produced by catabolic metabolism of several lipids, such as ether glycerolipids, fatty alcohols, sphingolipids. Medium-chain aliphatic aldehydes, such as hexanal, octanal and 4-hydroxy-2-nonenal (4HNE) are produced via lipid peroxidation during oxidative stress and ALDH3B 1 can oxidise both medium- and long-chain aldehydes. C16 aldehydes such as hexadecanal (HXAL), typically generated through sphingolipid metabolism on the plasma membrane, can be oxidised to palmitic acid (PALM). 4HNE, amongst other reactive medium-chain aldehydes, can be detoxified by ALDH3B 1 via oxidation to 4-hydroxynonenoic acid (4HNA), therefore indicating a potential physiological role for ALDH3B1 against oxidative stress. Therefore, ALDH3B1 plays a major role in the detoxification of aldehydes generated by alcohol metabolism and lipid peroxidation [25]. Alcohol intoxication is a potentially under-recognised yet important precipitant of Brugada Syndrome; thus, the identification of ALDH3B1 as a biomarker for the diagnosis of Brugada Syndrome in a human being is further indicative of a link between dysregulated alcohol metabolism and Brugada Syndrome and is another object of the present invention.

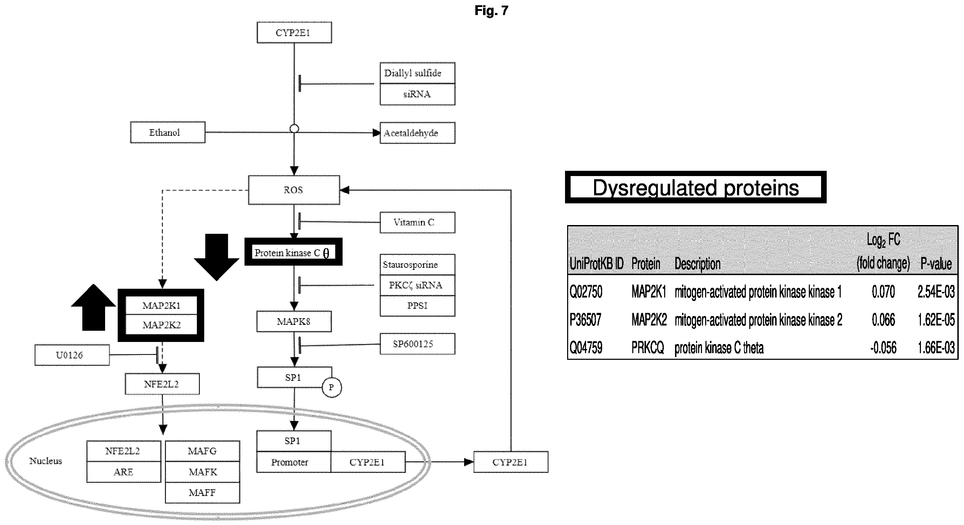

Other proteins that are dysregulated in blood PBMCs from Brugada Syndrome patients link alcohol metabolism to Brugada Syndrome. A significant enrichment for the "ethanol metabolism resulting in production of ROS by CYP2E1 (WP4269)" has been identified (see **Figure 7**) where increased oxidative stress by CYP2E1 induction is known to be a major consequence of ethanol-mediated liver toxicity [26]. Three proteins dysregulated in Brugada Syndrome that are crucial for the pathways are: MAPK1 (mitogen-activated protein kinase kinase 1), MAPK2 (mitogen-activated protein kinase kinase 2) and PRKCQ (protein kinase C theta), see **Figure 7****.**

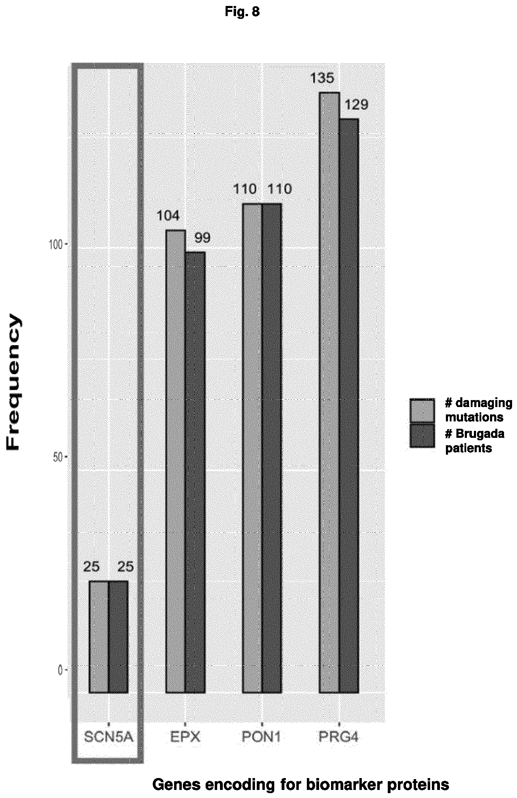

Integrated functional analysis of all proteins (n=995 proteins in PBMCs, FDR ≤ 5%; n=25 proteins in plasma, FDR ≤ 5%) and metabolites (n=275 metabolites in plasma, FDR ≤ 5%) found dysregulated in blood from Brugada Syndrome patients, identified a significant protein-metabolite interaction network (Figure 8). This network is derived by using existing knowledge of functional interactions between proteins and metabolites from the Human Metabolome Database (HMDB) (which currently covers 114,100 metabolites and 5,779 proteins) [27] and STITCH database (which currently covers 9,643,763 proteins, 430 000 chemicals and 1.6 billion interactions) [28].

The network is significantly enriched for "Alanine, aspartate and glutamate metabolism" pathway (P=3.2x10⁻⁹), comprising 7 metabolites (L-Glutamine, L-Glutamic acid; L-Aspartic acid; Uric Acid, L-Alanine, Choline, Taurolithocholic acid 3-sulfate) and 1 protein (ABCB), which are all dysregulated in in Brugada Syndrome patients.

The central "hub" node the network is GOT2 (glutamic-oxaloacetic transaminase 2) (intramitochondrial isoform) - a member of the malate-aspartate shuttle (MAS) - which plays a key role in the intracellular NAD(H) redox balance. The malate aspartate shuttle requires the concerted action of different components, including mitochondrial malate dehydrogenase MDH2, which is also dysregulated in Brugada Syndrome patients, see **Figure 8****.** Therefore, both GOT2 and MDH2 proteins identified in the same protein-metabolite interaction network, are essential for malate-aspartate metabolite exchange between mitochondria and cytosol, and for amino acid metabolism. In keeping with the dysregulation of GOT2 and MDH2 proteins in Brugada Syndrome patients, the malate metabolite is also dysregulated (decreased) in Brugada Syndrome patients compared with controls (Log2FC = - 0.12, P = 2.6x10⁻⁵), see **Figure 4**. Defects in the MAS have been described due to mutations in genes encoding mitochondrial malate dehydrogenase (MDH2) and encoding for glutamic-oxaloacetic transaminase 2 (GOT2) [29]. Thus, the identification of both GOT2 and MDH2 proteins dysregulated in Brugada Syndrome patients is further indicative of a link between dysregulated MAS in Brugada Syndrome and is another object of the present invention.

### EXAMPLE 2: Multi-omics identification of the set of biomarkers of Brugada Syndrome of the invention

### MATERIALS AND METHODS

### Ethical Statement

All procedures performed in studies involving human participants were in accordance with the ethical standards of the institutional and/or national research committee and with the Helsinki declaration and its later amendments or comparable ethical standards. The study design was examined by the Ethical Committee of the San Raffaele Hospital (Protocol BASED-version 07/01/2004).

### Patients enrolment

The population study has been constituted by two groups (300 subjects/group): the Brugada (BrG) and the Control (CG) Groups. The expected enrollment period was of 24 months.

Patients were evaluated for:
- baseline laboratory examinations, including total blood count, hepatic, thyroid and coagulation function.
- transthoracic ECG.

The inclusion/exclusion criteria for the two groups have been respectively the following.

Brugada Group: All consecutive Brugada Syndrome patients, aged >18 years, positive to the ajmaline test, or considered at high risk of SCD and undergoing epicardial ablation will be enrolled in this study.

Control Group: A population of patients with a structurally normal heart with a negative ajmaline test confirming the absence of Brugada Syndrome.

### Inclusion criteria for Control Group:

- Age >18 years
- Absence of Brugada Syndrome confirmed with ajmaline test.

### Exclusion criteria for Control Group:

- Ejection Fraction <55%
- Coronary artery disease with the need for revascularization
- Previous heart surgery
- Recent surgical/percutaneous intervention for valvular heart disease (<6 months)
- Life expectancy <1 year
- Impossibility to provide consent to the procedure and to the study

### Metabolomic analysis on plasma

Samples were prepared using the automated MicroLab STAR^{®} system from Hamilton Company. Several recovery standards were added prior to the first step in the extraction process for QC purposes. To remove protein, dissociate small molecules bound to protein or trapped in the precipitated protein matrix, and to recover chemically diverse metabolites, proteins were precipitated with methanol under vigorous shaking for 2 min (Glen Mills GenoGrinder 2000) followed by centrifugation. The resulting extract was divided into five fractions: two for analysis by two separate reverse phase (RP)/UPLC-MS/MS methods with positive ion mode electrospray ionization (ESI), one for analysis by RP/UPLC-MS/MS with negative ion mode ESI, one for analysis by HILIC/UPLC-MS/MS with negative ion mode ESI, and one sample was reserved for backup. Samples were placed briefly on a TurboVap^{®} (Zymark) to remove the organic solvent. The sample extracts were stored overnight under nitrogen before preparation for analysis.

### QA/QC

Several types of controls were analyzed in concert with the experimental samples: a pooled matrix sample generated by taking a small volume of each experimental sample (or alternatively, use of a pool of well-characterized human plasma) served as a technical replicate throughout the data set; extracted water samples served as process blanks; and a cocktail of QC standards that were carefully chosen not to interfere with the measurement of endogenous compounds were spiked into every analyzed sample, allowed instrument performance monitoring and aided chromatographic alignment.

The following Tables 6 and 7 describe these Metabolon QC samples and standards employed in the analysis.

**TABLE 6**

| Type | Description | Purpose |
|---|---|---|
| MTRX | Large pool of human plasma maintained by Metabolon that has been characterized extensively. | Assure that all aspects of the Metabolon process are operating within specifications. |
| CMTRX | Pool created by taking a small aliquot from every customer sample. | Assess the effect of a non-plasma matrix on the Metabolon process and distinguish biological variability from process variability. |
| PRCS | Aliquot of ultra-pure water | Process Blank used to assess the contribution to compound signals from the process. |
| SOLV | Aliquot of solvents used in extraction. | Solvent Blank used to segregate contamination sources in the extraction. |

**TABLE 7**

| Type | Description | Purpose |
|---|---|---|
| RS | Recovery Standard | Assess variability and verify performance of extraction and instrumentation. |
| IS | Internal Standard | Assess variability and performance of instrument. |

Instrument variability was determined by calculating the median relative standard deviation (RSD) for the standards that were added to each sample prior to injection into the mass spectrometers. Overall process variability was determined by calculating the median RSD for all endogenous metabolites (i.e., non-instrument standards) present in 100% of the pooled matrix samples. Experimental samples were randomized across the platform run with QC samples spaced evenly among the injections, as outlined in Figure 1. Figure 1 shows the preparation of client-specific technical replicates wherein a small aliquot of each client sample (colored cylinders) is pooled to create a CMTRX technical replicate sample (multi-colored cylinder), which is then injected periodically throughout the platform run. Variability among consistently detected biochemicals can be used to calculate an estimate of overall process and platform variability.

### Ultrahigh Performance Liquid Chromatography-Tandem Mass Spectroscopy (UPLC-MS/MS)

All methods utilized a Waters ACQUITY ultra-performance liquid chromatography (UPLC) and a Thermo Scientific Q-Exactive high resolution/accurate mass spectrometer interfaced with a heated electrospray ionization (HESI-II) source and Orbitrap mass analyzer operated at 35,000 mass resolution. The sample extract was dried then reconstituted in solvents compatible to each of the four methods. Each reconstitution solvent contained a series of standards at fixed concentrations to ensure injection and chromatographic consistency. One aliquot was analyzed using acidic positive ion conditions, chromatographically optimized for more hydrophilic compounds. In this method, the extract was gradient eluted from a C18 column (Waters UPLC BEH C18-2.1x100 mm, 1.7 µm) using water and methanol, containing 0.05% perfluoropentanoic acid (PFPA) and 0.1% formic acid (FA). Another aliquot was also analyzed using acidic positive ion conditions, however it was chromatographically optimized for more hydrophobic compounds. In this method, the extract was gradient eluted from the same afore mentioned C18 column using methanol, acetonitrile, water, 0.05% PFPA and 0.01% FA and was operated at an overall higher organic content. Another aliquot was analyzed using basic negative ion optimized conditions using a separate dedicated C18 column. The basic extracts were gradient eluted from the column using methanol and water, however with 6.5mM Ammonium Bicarbonate at pH 8. The fourth aliquot was analyzed via negative ionization following elution from a HILIC column (Waters UPLC BEH Amide 2.1x150 mm, 1.7 µm) using a gradient consisting of water and acetonitrile with lOmM Ammonium Formate, pH 10.8. The MS analysis alternated between MS and data-dependent MSn scans using dynamic exclusion. The scan range varied slighted between methods but covered 70-1000 m/z. Raw data files are archived and extracted as described below.

### Data Extraction and Compound Identification

Raw data was extracted, peak-identified and QC processed using Metabolon's hardware and software. Compounds were identified by comparison to library entries of purified standards or recurrent unknown entities. Metabolon maintains a library based on authenticated standards that contains the retention time/index (RI), mass to charge ratio (m/z), and chromatographic data (including MS/MS spectral data) on all molecules present in the library. Furthermore, biochemical identifications are based on three criteria: retention index within a narrow RI window of the proposed identification, accurate mass match to the library +/- 10 ppm, and the MS/MS forward and reverse scores between the experimental data and authentic standards. The MS/MS scores are based on a comparison of the ions present in the experimental spectrum to the ions present in the library spectrum. While there may be similarities between these molecules based on one of these factors, the use of all three data points can be utilized to distinguish and differentiate biochemicals. More than 3300 commercially available purified standard compounds have been acquired and registered for analysis on all platforms for determination of their analytical characteristics. Additional mass spectral entries have been created for structurally unnamed biochemicals, which have been identified by virtue of their recurrent nature (both chromatographic and mass spectral). These compounds have the potential to be identified by future acquisition of a matching purified standard or by classical structural analysis.

### Curation

A variety of curation procedures were carried out to ensure that a high-quality data set was made available for statistical analysis and data interpretation. The QC and curation processes were designed to ensure accurate and consistent identification of true chemical entities, and to remove those representing system artifacts, mis-assignments, and background noise. Library matches for each compound were checked for each sample and corrected if necessary.

### Metabolite Quantification and Data Normalization

Peaks were quantified using area-under-the-curve. For studies spanning multiple days, a data normalization step was performed to correct variation resulting from instrument inter-day tuning differences. Essentially, each compound was corrected in run-day blocks by registering the medians to equal one (1.00) and normalizing each data point proportionately (termed the "block correction". For studies that did not require more than one day of analysis, no normalization is necessary, other than for purposes of data visualization. In certain instances, biochemical data may have been normalized to an additional factor (e.g., cell counts, total protein as determined by Bradford assay, osmolality, etc.) to account for differences in metabolite levels due to differences in the amount of material present in each sample.

### Lipidomic analysis on plasma

Metabolon, Inc., Morrisville, USA performed the lipidomic analysis. Lipids were extracted from plasma in the presence of deuterated internal standards using an automated BUME extraction according to the method of Lofgren et al. [19]. The extracts were dried under nitrogen and reconstituted in ammonium acetate dichloromethane: methanol.

The extracts were transferred to vials for infusion-MS analysis, performed on a Shimadzu LC with nano PEEK tubing and the Sciex SelexIon-5500 QTRAP. The samples were analyzed via both positive and negative mode electrospray. The 5500 QTRAP was operated in MRM mode with a total of more than 1,100 MRMs. Individual lipid species were quantified by taking the ratio of the signal intensity of each target compound to that of its assigned internal standard, then multiplying by the concentration of internal standard added to the sample. Lipid class concentrations were calculated from the sum of all molecular species within a class, and fatty acid compositions were determined by calculating the proportion of each class comprised by individual fatty acids.

### Proteomics analysis on plasma and PBMCs

Omics Technologies Inc., Columbia, MD, USA performed the proteomic analyses on plasma and PBMCs samples. Plasma samples were depleted of the top 14 high abundance proteins to improve the detectability of low abundance proteins. 11-plex TMT labeling proteomics approach was utilized to reveal quantitative proteomics information across different samples in each batch.

Conventional sample preparation was conducted to generate a peptidome sample from each of the 1200 clinical specimen. Each sample was assigned to a multiplex quantitative proteomics assay, and all multiplex quantitative assays are sharing the same reference sample which is created at the beginning of the project through combining 100 randomly selected disease and control samples to best represent the complete profile of the different proteomes expected to be observed throughout this entire study. Large scale quantitative proteomics assays were performed on the state-of-the-art HPLC and LCMS instrumentations, analytical results and raw data were provided under a further agreement for raw data transfer.

### Chemicals and Reagents

TCEP (TCEP (tris-(2-carboxyethyl) phosphine) was purchased from Thermo Scientific (Waltham, Massachusetts, USA). LysC and Trypsin proteases were purchased from Promega (Fitchburg, Wisconsin, USA). C18 Cartridges for sample preparation, and chromatography columns for bRPLC and online HPLC of Triple Quadrupole mass spectrometer were purchased from Waters (Milford, Massachusetts, USA). Acetonitrile was purchased from JT Baker (Phillipsburg, NJ, USA), and formic acid was obtained from EMD Millipore (Billerica, MA, USA). MyPro-Buffer I, MyPro-Buffer II and MyPro-Buffer III were utilized by Omics Technologies Inc (Columbia, MD, USA). All other reagents were purchased from Sigma-Aldrich (St. Louis, Missouri, USA) unless otherwise indicated.

### Preparation of the Chromatography Solutions

bRPLC Solvent A contained lOmM TEABC; bRPLC Solvent B contained lOmM TEABC, 90%
(vol/vol) Acetonitrile. SRM Mass Spectrometry Solvent A was comprised of water with 0.1% (vol/vol)

Formic Acid; SRM Solvent B was Acetonitrile with 0.1% (vol/vol) Formic Acid.

### Plasma Sample Depletion

Top 14 most abundant proteins (Albumin, IgG, α2-Antitrypsin, IgA, IgM, Transferrin, Haptoglobin, α2Macroglobulin, Fibrinogen, Complement C3, α1-Acid Glycoprotein (Orosomucoid), HDL

(Apolipoproteins A-I and A-II) and LDL (mainly Apolipoprotein B)) in the plasma were depleted using the Seppro IgY14 column systems. Plasma samples were diluted 5X in IgY dilution buffer, filtered (0.22 µm), then injected into IgY LC10 columns attached to an Agilent 1200 HPLC system. The unretained fraction was collected.

### Protein preparation

In-solution depleted plasma samples or PBMC cells were further processed by Omics Technologies Inc. (Columbia, MD, USA) using a modified "Filter Assisted Sample Preparation" (FASP) method. Briefly, protein samples were suspended in 9M UREA were then reduced with 5 mM TCEP at 60°C for 15 min and reduced cysteines were blocked using 10 mM Iodoacetamide (IAA) at 25°C for 1 hour. Protein samples (100 µg proteins) were then cleaned using Amicon Filters (Millipore, MA, USA) three times with 9M urea and twice with MyPro-Buffer I (Omics Technologies Inc., MD, USA). Samples were then proteolyzed with trypsin (Promega, WI, USA) for 12 hrs at 37°C. The peptide solution then was acidified by adding 1% trifluoroacetic acid (TFA) and was incubated at room temperature for 15 min. A

Sep-Pak light C18 cartridge (Waters Corporation, MA, USA) was activated by loading 5 mL 100% (vol/vol) acetonitrile and was washed by 3.5 mL 0.1% TFA solution twice. Acidified digested peptide solution was centrifuged at 1,800 × g for 5 min, and the supernatant was loaded into the cartridge. To desalt the peptides bound to the cartridge, 1 mL, 3 mL, and 4 mL of 0.1% TFA were used sequentially. To elute the peptides from the cartridge, 2 mL of 40% (vol/vol) acetonitrile with 0.1% TFA was used, and this elution was repeated two more times (for a total of 6 mL of eluate). It was important to ensure that the cartridge had stopped dripping before each sequential wash and elution solution was applied. The eluted peptides were lyophilized overnight and reconstituted in 37 µL of MyPro-Buffer II (Omics Technologies Inc., MD, USA).

### Multiplexed TMT labeling

Digested peptides from each sample in a volume of 37 µl MyPro-Buffer II were labeled using 11-plex TMT reagents. After 2 hrs, labeling reactions were quenched and different quantitative channels belonging to each experiment were pooled and dried to remove organic solvents followed by a reconstitution in 500ul MyPro-Buffer III (Omics Technologies Inc.). Pooled sample was fractionated on a bRPLC (basic Reverse Phase Liquid Chromatography) column (XBridge BEH C18 Column, 5 µm, 2.1 x 100 mm) via XBridge BEH C18 Guard Column (Waters Corporation) using an Agilent 1260 Infinity II HPLC system. Peptides in each fraction were dried and re-suspended in 8 µl 0.1% formic acid with 3% acetonitrile for LC-MS/MS analysis.

### Liquid Chromatography Mass Spectrometry (LC-MS) Analysis

Peptidome samples were fractionated through a Thermo Scientific^{™} EASY-nLC 1000^{™} HPLC system with analytical nanoflow column system including a 2 cm trap column and a 75µm x 20 cm analytical column both packed with SuperB C18 HIFI, 5µm, 100Å (Omics Technologies Inc., MD, USA). Eluted samples were ionized through Thermo Scientific EASYSpray^{™} source analyzed on a Thermo Scientific^{™} Q Exactive HF-X Mass Spectrometer using FT HCD MS2 fragmentation mode with Orbitrap mass analyzer operated at 45,000 mass resolution. Peptides were electrosprayed through a 15µm emitter (Omics Technologies Inc., MD, USA) at a 2.0kV spray voltage. Reversed-phase solvent gradient consisted of 0.1% formic acid with increasing levels of 90% acetonitrile in 0.1% formic acid over a period of 130 minutes. The Q Exactive HF-X instrument was operated to automatically switch between full scan MS and MS/MS acquisition. Survey full scan MS spectra (m/z 350-1800) was acquired in the Orbitrap with 45,000 resolution after accumulation of ions to a target value based on predictive AGC from the previous full scan. Intense multiply-charged ions (z ≥ 2) were sequentially isolated and fragmented in the Axial Higher energy Collision-induced Dissociation (HCD) cell using normalized HCD collision energy at 30% with an AGC target 1e⁵ and a maximal injection time of 400 ms at 45,000 resolution.

### MS data analysis

Mass spectrometry raw files were processed through MyProt-ID pipeline (Omics Technologies Inc., MD, USA) developed for Peptide Identification and Quantification services. Search parameter included oxidation on methionine, deamidation on residues N and Q as different variable modifications. Mass tolerances on precursor and fragment masses were set to 15 ppm and 0.03 Da, respectively. Peptide validator node was used for peptide validation with stringent cut-off of 0.01 and relaxed cut-off of 0.05 (False Discover Rate).

### Western blot of biomarker proteins

For protein expression analysis, PBMCs and plasma samples were subjected to two different pre-processing procedures to extract or enrich the proteins, respectively. PBMCs. were lysed with RIPA buffer (1% Nonidet P-40 in 50 mmTris-HCl, pH 7.5, 150 mm NaCl, 0.1% sodium deoxycholate, 1% protease inhibitor cocktails), incubated in ice for 30 min, and then centrifuged at 13 000×g for 15 minutes at 4°C. The supernatant was collected, and the total amount of proteins was determined with BCA assay (Pierce), following the manufacturer's instructions. Plasma samples (10 ul) were depleted from the abundant proteins using the Multiple Affinity Removal Spin Cartridge Human 14 (Agilent), following manufacturer's instructions. Proteins from PBMCs and plasma (40 ug) were resolved on a 10% SDS-PAGE gel and subsequently transferred onto nitrocellulose membranes by electroblotting. The total amount of transferred proteins, used for the normalization of detected proteins, was determined with the REVERT Total Protein Stain kit (LICOR Biotechnology), following manufacturer's instructions. Membranes were incubated with blocking buffer (TBS: 10 mm Tris-HCl, pH 7.4,150 mm NaCl, 0.1% (v/v) Tween 20 containing 5% (w/v) bovine serum albumin) for 1 hour at room temperature, and then the primary antibodies were added and incubated overnight at 4°C in the blocking buffer. The following primary antibodies were used: anti-EPX (Thermo Fisher, cat. PA5-42062), anti-ALDH3B1 (Origene, cat. TA811463), anti-SLC25A5 (Thermo Fisher, cat. PA5-90592), anti-AP2S1 (Abnova, cat. H00001175-M01) for PBMCs, and anti-FNl, anti-PRG4, anti-ITIH3 The membranes were washed three times with TBS-Tween 20 for 10 min and then incubated for 2 h with the appropriate secondary antibody. The secondary antibodies used were: anti-mouse HRP conjugated(Amersham), anti-rabbit HRP conjugated (Amersham), IRDye 800CW anti-mouse (Licor), IRDye 800CW anti-rabbit (Licor), IRDye 680CW anti-mouse (Licor), and IRDye 680CW anti-rabbit (Licor). After three washes with TBS-Tween 20, proteins were detected with an ECL detection kit (Cyanagen) or with infrared acquisition at the proper wavelength with the LI-COR Odyssey Infrared Imaging System (LI-COR Biotechnology).

### Statistical analysis and biomarker identification

Proteomics analysis (in plasma of n=591 individuals and in PBMCs n=587 individuals), lipidomics analysis (in plasma of n=600 individuals) and metabolomics analysis (in plasma of n=600 individuals) yielded a common set of 585 individuals where proteomics, lipidomics and metabolomics measurements were available.

To identify a multi-omics biomarker signature for the diagnosis of Brugada Syndrome in a human being, a multi-steps procedure has been adopted as follows.
Step 1: within each omics dataset, the features (i.e., proteins, metabolites, lipids) that are significantly different (i.e., differentially expressed (DE) features) between Brugada Syndrome patients and controls have been identified.
Step 2: all DE features (from Step 1) have been combined in a single multi-omics dataset, including proteins (PBMCs and plasma), metabolites (plasma) and lipids (plasma).
Step 3: using the single multi-omics dataset (from Step 2), 6 methods to independently predict features (i.e., proteins, metabolites, lipids) for the diagnosis of Brugada Syndrome have been employed and rank each feature on the basis of its relative "importance" and "frequency" for each method.
Step 4: using the features prediction and ranking (from Step 3), features that are not consistently predicted by each method have been removed, and then the "importance" and "frequency" ranks of the consistently-detected features across all methods have been combined, to derive a single rank ("importance" and "frequency") for each feature.

This multi-step procedure (above) has been carried out separately in
1) the whole patient cohort (585 individuals comprising 294 Brugada Syndrome patients and 291 controls)
2) the patient cohort of males only (371 individuals comprising 202 Brugada Syndrome patients and 169 controls)
3) the patient cohort of females only (214 individuals comprising 92 Brugada Syndrome patients and 122 controls).

The predictions of the biomarker features in the whole patient cohort, males and females were overlapping, yet not identical. Thus, the biomarker features found in the whole patient cohort and in the male and female cohorts have been cross-matched to identify a common set of features, referred to as *"core-set",* for the diagnosis of Brugada Syndrome. The core-set included features for the diagnosis of Brugada Syndrome in males and females as well as in the whole cohort. A larger, extended-set of features for the diagnosis of Brugada Syndrome, referred to as *"extended-set"* was identified, as those features that were identified in the whole patient cohort. Technical details of each step are as follows.

Step 1. *Proteomics data analysis.* The whole PBMC and plasma proteomics PSM (peptide-spectrum match score) data matrixes (~ 17k proteins each) were culled for removing species with more than 50 % missing values. They were processed with the DEqMS tool in R [30], and the missing values (following a missing-at-random [MAR] pattern) were imputed using a k-nearest neighbour function, and the resulting matrices were normalized using an Equal Median Normalization function. The normalized matrixes were used to identify the DE proteins, using a Spectral Count eBayes function to correct bias of variance estimate based on minimum number of PSMs per protein used for quantification.

Metabolomics data analysis. The metabolomics matrix was processed using the MetaboDiff tool in R [31]. The number of missing values were rather low across samples (less than 15% and followed a MAR pattern) and were imputed using a k-nearest neighbour function. The imputed matrices were normalized using a Variance Stabilizing Normalization (VSN) function, and the differentially expressed Proteins were identified relying on Student's t-Test statistics, while the false discovery rate (FDR) correction of the p-values for multiple testing was done using the Benjamini-Hochberg (BH) procedure.

Lipidomcs data analysis. The lipidomics matrix was processed using the lipidr tool in R [32]. The low number of missing values following a MAR pattern was inferred with a KNN function, and a VSN normalization method was used to deal with the high percentage of low abundance lipids. The differential abundance of lipids between Brugada patients and controls was identified using the limma package [33], based on t-statistics and relying on the for BH procedure for p-value correction for multiple testing.

For each DE analysis of the proteomics, metabolomics and lipidomics dataset, the effect of gender, age, batch and protocol was accounted for and corrected using linear modelling in limma package [33]. The number of DE features (FDR≤5%) identified in each dataset are as follows: 995 DE proteins in PBMCs; 25 DE proteins in plasma; 89 DE metabolites in plasma (44 Xenobiotics and unannotated metabolites were removed); 230 DE lipids in plasma.

Step 2: the DE proteins, lipids and metabolites from Step 1 are combined in a single dataset. This defined a set of 1,339 proteins/metabolites/lipids DE in Brugada Syndrome and which are measured in 585 patients. This data matrix 1,339 features (proteins/metabolites/lipids) x 585 (individuals) is used as input for the biomarker prediction in the next step.

Step 3: After the data matrix is autoscaled, the following 6 methods have been used: (1) receiver operating characteristic curve (ROC curve) to determine the AUROC (Area Under the Receiver Operating Characteristics) or AUC (Area Under The Curve) for the prediction of Brugada Syndrome; (2) P-value for differential protein/metabolite/lipid abundance between Brugada Syndrome patients and controls; (3) support vector machine (SVM) with linear kernel [34]; (4) Random Forest (RF) [35]; (5) partial least squares discriminant analysis (PLS-DA) [36], (6) sparse partial least squares discriminant analysis (sPLS-DA). For each feature, univariate AUC and P-value provide a quantification of the feature importance whereas the multivariate Machine Learning (ML) methods, SVM, RF and PLS-DA, provide a quantification of the feature "importance" and of the feature "frequency" (i.e., 100 runs of Monte-Carlo cross validation (MCCV) using balanced subsampling where in each MCCV two thirds (2/3) of the samples are used to evaluate each feature importance and frequency). Multivariate sPLS-DA is employed to provide simultaneous feature selection and dimension reduction [37], therefore reducing the number of informative features for the diagnosis of Brugada Syndrome and limiting overfitting. For PLS-DA and sPLS-DA the optimal number of significant components has been determined using R2 and Q2 [38].

Step 4: Biomarker predictions using AUC, P-value, SVM, RF, PLS-DA and sPLS-DA provided a set of 330 features commonly detected by all methods. Each feature is assigned a rank for its relative "importance" (AUC, P-value, SVM, RF, PLS-DA) and "frequency" (SVM, RF, PLS-DA), and we also require the feature to be found in the "sparse" feature selection model by sPLS-DA (for details on how sparse PLS regression achieves both high predictive power and accuracy for finding the relevant features please refer to [37]. An additional requirement is that each feature has some predictive power by its own, and therefore an additional filtering, requiring AUC>0.6 has been adopted. Using the common features predicted by all methods, imposing AUC>0.6 and detected in the "sparse" feature selection model by sPLA-DA, 16 features for the diagnosis of Brugada Syndrome in the whole patient cohort (585 individuals) were identified. Similarly, analysis of the female patient cohort (214 individuals) identified 29 features and analysis of the male patient cohort (371 individuals) identified 28 features. The set of features in common between the whole patient cohort and both female and male patient cohorts consists of 10 features (*core-set*), while the set features identified in whole patient cohort consists of 16 features (*extended-set*) which includes all features in the *core-set,* as reported in the following Table 8:

**TABLE 8**

| **Signature¹** | **Cell type** | **Feature Name** | **Description** | **Overall rank²** | **AUC** | **-Log₁₀P** |
|---|---|---|---|---|---|---|
| *core #1* | Plasma | FN1 | fibronectin 1 | **2.78** | 0.651 | 13.3 |
| *core #2* | PBMC | AP2S 1 | adaptor related protein complex 2 subunit sigma 1 | **7.45** | 0.616 | 6.2 |
| *core #3* | Plasma | ITIH3 | inter-alpha-trypsin inhibitor heavy chain 3 | **8.62** | 0.602 | 4.5 |
| *core #4* | PBMC | EPX | eosinophil peroxidase | **8.66** | 0.624 | 6.5 |
| *core #5* | Plasma | fumarate | fumaric acid | **9.40** | 0.632 | 6.2 |
| *extended #1* | PBMC | ALDH3 B1 | aldehyde dehydrogenas e 3 family member B1 | **9.97** | 0.633 | 6.3 |
| *core #6* | Plasma | PRG4 | proteoglycan 4 | **10.33** | 0.620 | 6.2 |
| *extended #2* | PBMC | SLC25A 5 | solute carrier family 25 member 5 | **10.49** | 0.620 | 6.8 |
| *extended #3* | Plasma | PON1 | paraoxonase 1 | **10.77** | 0.604 | 6.2 |
| *core #7* | PBMC | COG5 | component of oligomeric Golgi complex 5 | **12.25** | 0.601 | 5.2 |
| *extended #4* | Plasma | 2-hydroxyd ecanoate | alpha-hydroxycapri c acid | **12.72** | 0.635 | 6.7 |
| *core #8* | PBMC | RIOX1 | ribosomal oxygenase 1 | **13.42** | 0.612 | 4.5 |
| *core #9* | Plasma | glutamat e | L-Glutamic acid | **15.00** | 0.613 | 6.6 |
| *core #10* | Plasma | Palmitole oylcarniti ne (C16:1)* | O-palmitoleoylc arnitine | **15.60** | 0.607 | 5.0 |
| *extended #5* | PBMC | S100A11 | S100 calcium binding protein A11 | **16.14** | 0.612 | 5.6 |
| *extended #6* | PBMC | PLCB2 | phospholipas e C beta 2 | **17.08** | 0.601 | 4.2 |

| | | | | | | |
|---|---|---|---|---|---|---|
| ¹ *core*: features in the "core-set" (n=10) identified in males and females as well as in the whole patient cohort; *extended*: features in the "extended-set" (n=6) that have been identified in the whole patient cohort. ² Features are ranked according to the overall rank obtained from the analysis of the whole patient cohort (n=585 individuals), which combines (adds up) the feature's rank calculated based on the feature relative "frequency" and "importance" across AUC, P-value, SVM, RF and PLS-DA methods. | | | | | | |

### EXAMPLE 3: Identification of deleterious mutations in protein biomarkers of the

### MATERIALS AND METHODS

Genetic analysis in a subgroup of Brugada Syndrome patients (n=186) by whole-genome sequencing (WGS) by both PolyPhen [39] and/or SIFT [40] has been carried out.

SIFT predicts whether an amino acid substitution is likely to affect protein function based on sequence homology and the physical-chemical similarity between the alternate amino acids. The data provided for each amino acid substitution is a score and a qualitative prediction (either 'tolerated' or 'deleterious'). The score is the normalized probability that the amino acid change is tolerated so scores nearer zero are more likely to be deleterious. The qualitative prediction is derived from this score such that substitutions with a score < 0.05 are called 'deleterious' and all others are called 'tolerated.

PolyPhen-2 predicts the effect of an amino acid substitution on the structure and function of a protein using sequence homology, Pfam annotations, 3D structures from PDB where available, and several other databases and tools (including DSSP, ncoils etc.). As with SIFT, for each amino acid substitution where it was possible to calculate a prediction, both a qualitative prediction (one of 'probably damaging', 'possibly damaging', 'benign' or 'unknown') and a score were provided. The PolyPhen score represents the probability that a substitution is damaging, so values close to one are more confidently predicted to be deleterious. The qualitative prediction is based on the False Positive Rate of the classifier model used to make the predictions.

### RESULTS

Many Brugada Syndrome patients carry a significantly high number of deleterious mutations even when compared with the SCN5A gene in which mutations are most commonly found in Brugada Syndrome [10, 11].

Specifically, the genes encoding for the biomarker proteins: EPX (eosinophil peroxidase), PON1 (paraoxonase 1) and PRG4 (Proteoglycan 4) carry the same mutation in 91 patients, 110 patients and 123 patients with mutations, respectively, as shown in **Figure 8****.**

The most highly mutated gene is Proteoglycan 4 (PRG4), a member of the proteoglycan family, which plays a diverse range of physiological and pathological mechanisms including metabolic pathologies [41]. Proteoglycan 4 is ubiquitously expressed and secreted into the circulation and has emerged from human association studies, on both transcriptional and protein levels, as a possible factor contributing to weight gain, dyslipidaemia and insulin resistance and PRG4 plasma levels were found to be associated with obesity and weight loss [42-43]. Mouse studies showed a causal role for PRG4 in the regulation of energy metabolism [44] and given the upregulation of PRG4 in obese individuals with type 2 diabetes, it can be suggested that PRG4 also plays a causal role in the development of metabolic disturbances in humans.

Another highly mutated gene encoding for dysregulated biomarker protein is paraoxonase 1 (PON1), a liver-derived glycoprotein that is secreted into the circulation and is associated with high-density lipoprotein (HDL) in the blood. PON1 is considered to play a crucial role as an anti-atherosclerotic factor. This enzyme exhibits antioxidant and antiatherogenic properties and, furthermore, alterations in circulating PON1 activity have been associated with a variety of diseases, including diabetes mellitus, obesity, hepatic and renal diseases, psoriasis, cancer, and rheumatoid arthritis [45]. Therefore, it has been also suggested that PON1 concentration and activity be used as a biomarker for the detection of various diseases [46]. However, the activity of PON1 is both under genetic and environmental regulation (including age, sex and lifestyle, and pharmaceutical interventions), and it has been shown to vary widely between individuals [47]. The very high number of mutations (>100) detected in PON1 in Brugada Syndrome patients suggests a causative role for this gene in the Brugada Syndrome disease, and possibly a functional role for oxidative stress control, as shown for other diseases like breast cancer in diabetic patients [48].

The following Table 9 reports the sequence variants for the most highly mutated genes (PRG4 sequence variant: 1-186304862 C->T, EPX sequence variant: 17-58193733 G->C and PON1 sequence variant: 7-95316772 A->T). All details about the deleterious mutation were detected in the Brugada Syndrome patients: chromosome (Chr.), DNA strand (Strand), genomic position, reference and mutated allele, codon and amino acid changes.

| TABLE 9 | | | | | | | | | |
|---|---|---|---|---|---|---|---|---|---|
| **Gene** | **Gene Ensemble ID** | **Variant name GRCh38** | **Chr.** | **Position (bp)** | **Ref allele** | **Mut allele** | **Codon change (ref/mut)** | **Aa change (ref/mut)** | **N. of Brugada patients with** |
| PRG4 | ENS G00000 116690 | 1-1863048 62 C-T | 1 | 186,304, 862 | C | T | Cgg/Tgg | R/W | 123 |
| EPX | ENS G00000 121053 | 17-5819373 3 G-C | 17 | 58,193, 733 | G | C | caG/caC | Q/H | 91 |
| PON1 | ENS G00000 005421 | 7-9531677 2 A-T | 7 | 95,316, 772 | A | T | Atg/Ttg | L/M | 110 |

Finally, frequency of the mutation in Brugada patients cohort as well as in reference European and African American populations has been detected and reported in the following Table 10. Population frequencies for the mutated allele have been retrieved from The Genome Aggregation Database (gnomAD) database v3.1 (https://gnomad.broadinstitute.org/).

**TABLE 10**

| **Mutation frequency** | | | |
|---|---|---|---|
| **Gene** | **Brugada patient cohort** | **European population** | **African/African American population** |
| PRG4 | 0.3918 | 0.3439 | 0.1864 |
| EPX | 0.2756 | 0.3256 | 0.2503 |
| PON1 | 0.3567 | 0.3660 | 0.1749 |

### REFERENCES

[1] Antzelevitch, C., et al. Circulation, 2005. 111(5): p. 659-70.
[2] Brugada, P. and J. Brugada. J Am Coll Cardiol, 1992. 20(6): p. 1391-6.
[3] Nademanee, K., et al. J Am Coll Cardiol, 2015. 66(18): p. 1976-86.
[4] Priori, S.G., et al. Eur Heart J, 2015. 36(41): p. 2793-867.
[5] Mellor, G., et al. Circ Arrhythm Electrophysiol, 2014. 7(6): p. 1078-83.
[6] Nademanee, K., et al. Circulation, 1997. 96(8): p. 2595-600.
[7] Tan, H.L., et al. Circulation, 2005. 112(2): p. 207-13.
[8] Pappone, C., et al. J Am Coll Cardiol, 2018. 71(15): p. 1631-1646.
[9] Chen, Q., et al. Nature, 1998. 392(6673): p. 293-6.
[10] Kapplinger, J.D., et al. Heart Rhythm, 2010. 7(1): p. 33-46.
[11] Antzelevitch, C. and B. Patocskai. Curr Probl Cardiol, 2016. 41(1): p. 7-57.
[12] Mango, R., et al. Circ J, 2016. 80(4): p. 938-49.
[13] Pappone, C., M. Monasky, and G. Ciconte. Eur Heart J Suppl, 2019. 21(Suppl B): p. B61-B66.
[14] Yar, S., M.M. Monasky, and R.J. Solaro. Pflugers Arch, 2014. 466(6): p. 1189-97.
[15] Monasky, M.M., et al. Front Physiol, 2017. 8: p. 1056.
[16] Monasky, M.M., et al. Front Physiol, 2018. 9: p. 1088.
[17] Letsas, K.P., et al. Pacing Clin Electrophysiol, 2017. 40(12): p. 1332-1345.
[18] Hofman, N., et al. Circulation, 2013. 128(14): p. 1513-21.
[19] Lofgren et al. J Lipid Res 2012; 53(8): p. 1690-700.
[20] Casillas-Ituarte, N.N., et al. J Biol Chem, 2012. 287(9): p. 6693-701.
[21] Schumann, T., et al. Pharmacol Rev, 2020. 72(1): p. 343-379.
[22] Shoshan-Barmatz, V., A. Shteinfer-Kuzmine, and A. Verma. Biomolecules, 2020. 10(11).
[23] Rhee, S.G. Annu Rev Biochem, 2001. 70: p. 281-312.
[24] Hwang, H.J., et al. FASEB J, 2019. 33(10): p. 10668-10679.
[25] Marchitti, S.A., et al. Free Radic Biol Med, 2010. 49(9): p. 1432-43.
[26] Jin, M., et al. Cell Death Dis, 2013. 4: p. e554.
[27] Wishart, D.S., et al. Nucleic Acids Res, 2018. 46(D1): p. D608-D617.
[28] Szklarczyk, D., et al. Nucleic Acids Res, 2016. 44(D1): p. D380-4.
[29] van Karnebeek, C.D.M., et al. Am J Hum Genet, 2019. 105(3): p. 534-548.
[30] Zhu, Y., et al. Mol Cell Proteomics, 2020. 19(6): p. 1047-1057.
[31] Mock, A., et al. Bioinformatics, 2018. 34(19): p. 3417-3418.
[32] Mohamed, A., J. Molendijk, and M.M. Hill. J Proteome Res, 2020. 19(7): p. 2890-2897.
[33] Ritchie, M.E., et al. Nucleic Acids Res, 2015. 43(7): p. e47.
[34] Barla, A., et al. Brief Bioinform, 2008. 9(2): p. 119-28.
[35] Breiman, L. Machine Learning, 2001. 45(1): p. 5-32.
[36] Xia, J. and D.S. Wishart. Curr Protoc Bioinformatics, 2016. 55: p. 14 10 1-14 10 91.
[37] Chun, H. and S. Keles. J R Stat Soc Series B Stat Methodol, 2010. 72(1): p. 3-25.
[38] Triba, M.N., et al. Mol Biosyst, 2015. 11(1): p. 13-9.
[39]Adzhubei, I.A., et al. Nat Methods, 2010. 7(4): p. 248-9.
[40] Sim, N.L., et al. Nucleic Acids Res, 2012. 40(Web Server issue): p. W452-7.
[41] Bishop, J.R., M. Schuksz, and J.D. Esko. Nature, 2007. 446(7139): p. 1030-7.
[42] Geyer, P.E., et al. Mol Syst Biol, 2016. 12(12): p. 901.
[43] Oller Moreno, S., et al. Proteomics Clin Appl, 2018. 12(1).
[44] Nahon, J.E., et al. Biochim Biophys Acta Mol Basis Dis, 2019. 1865(2): p. 494-501.
[45] Costa, L.G., G. Giordano, and C.E. Furlong. Biochem Pharmacol, 2011. 81(3): p. 337-44.
[46] Camps, J., J. Marsillach, and J. Joven. Clin Chim Acta, 2007. 386(1-2): p. 114-5.
[47] Zhao, Y., et al. Mol Genet Metab, 2012. 105(1): p. 141-8.
[48] Eraldemir, F.C., et al. J Med Biochem, 2019. 38(3): p. 368-375.

## Claims

**1.** A set of biomarkers comprising at least:
i) the subset of the following 7 proteins:
| **Protein** | **UniProtKB ID** |
|---|---|
| Fibronectin 1 (FN1) | P02751 |
| Inter-alpha-trypsin inhibitor heavy chain 3 (ITIH3) | Q06033 |
| Proteoglycan 4 (PRG4) | Q92954 |
| Adaptor related protein complex 2 subunit sigma 1 (AP2S1) | M0QYZ2 |
| Component of oligomeric Golgi complex 5 (COG5) | Q9UP83 |
| Eosinophil peroxidase (EPX) | P11678 |
| Ribosomal oxygenase 1 (RIOX1) | Q9H6W3 |
and
ii) the subset of the following 3 metabolites:
- fumaric acid;
- O-palmitoleoylcarnitine;
- L-Glutamic acid;
for the diagnosis of Brugada Syndrome in a human being.

**2.** A set of biomarkers according to claim 1, further comprising one or more of the following additional biomarkers:
i) a protein selected from the group consisting of:
| **Protein** | **UniProtKB ID** |
|---|---|
| Paraoxonase 1 (PON1) | P27169 |
| Aldehyde dehydrogenase 3 family member B1 (ALDH3B 1) | P43353 |
| Solute carrier family 25 member (SLC25A5) | P05141 |
| S100 calcium binding protein A11 (S100A11) | P31949 |
| Phospholipase C beta 2 (PLCB2) | Q00722 |
and/or the metabolite 2-hydroxydecanoate;
or a combination thereof, for the diagnosis of Brugada Syndrome in a human being.

**3.** A set of biomarkers according to claim 2, comprising:
i) the subset of the following 12 proteins:
| **Protein** | **UniProtKB ID** |
|---|---|
| Fibronectin 1 (FN1) | P02751 |
| Inter-alpha-trypsin inhibitor heavy chain 3 (ITIH3) | Q06033 |
| Proteoglycan 4 (PRG4) | Q92954 |
| Adaptor related protein complex 2 subunit sigma 1 (AP2S1) | M0QYZ2 |
| Component of oligomeric Golgi complex 5 (COG5) | Q9UP83 |
| Eosinophil peroxidase (EPX) | P11678 |
| Ribosomal oxygenase 1 (RIOX1) | Q9H6W3 |
and
ii) the subset of the following 4 metabolites:
- fumaric acid;
- O-palmitoleoylcarnitine;
- L-Glutamic acid;
- 2-hydroxydecanoate;
or a combination thereof, for the diagnosis of Brugada Syndrome in a human being.

**4.** An in vitro method for detecting the presence or measuring concentration of the occurrence of one of the set of biomarkers according to anyone of claim 1-3 in a biological sample of a human being.

**5.** An in vitro method for detecting the presence of one of the set of biomarkers according to claim 4, which comprises the following steps:
a) detecting the presence of at least 7 up to 12 proteins of subset i) by an assay selected from the group comprising Western Blot, Dot blot, ELISA, flow cytometry, enzymatic activity assay, targeted MS, multiplex protein analysis, ProQuantum high-sensitivity immunoassay, or a combination thereof;
b) detection of at least 3 up to 4 of the metabolites of subset ii) by an assay such as one selected from the group comprising MS, GC-MS, fluorimetric or colorimetric assays, NMR, spectroscopy with nanoprobes, Enzyme-Linked oligonucleotide assays, or a combination thereof;
wherein the positive detection of all the biomarkers compared to a normal control allows the identification of patients affected by Brugada Syndrome.

**6.** An in vitro method for measuring the concentration of one of the set of biomarkers according to claim 4, comprising the following steps:
a) quantitative determination of at least 7 up to 12 proteins of subset i) by a quantitative technique such as Western Blot, ELISA, enzymatic activity assay, targeted MS, multiplex protein analysis, ProQuantum high-sensitivity immunoassays, or a combination thereof;
b) quantitative determination of at least 3 up to 4 of the metabolites of subset ii) by a technique such as HPLC, MS, NMR analysis, ELONA, or a combination thereof;
wherein an increase or decrease of the concentration of the biomarkers compared to a normal control indicates a diagnosis of Brugada Syndrome.

**7.** An in vitro method according to anyone of the claims 4-6, wherein the biological sample is selected from the group consisting of plasma, PBMCs, whole blood, serum and peripheral blood, or a combination thereof.

**8.** An in vitro method according to anyone of the claims 4-7, wherein the human being is asymptomatic.

**9.** An in vitro method according to anyone of the claims 4-7, wherein the human being is at high risk for Brugada Syndrome due to family history, previous events of heart atrial and/or ventricular fibrillation, diabetes or obesity.

**10.** An in vitro method according to anyone of the claims 4-9, wherein the human being is about 40 years old or younger.

**11.** A kit comprising antibodies specific for each of the proteins of subset i) according to anyone of claim 1-3, wherein said antibodies are labelled or attached to a solid support.

**12.** Oligonucleotide sequence of *Prg4* gene (ENSG00000116690) **characterized by** the presence of a deleterious mutations C→T in position 186,304,862 of the nucleotide sequence, said oligonucleotide sequence being RNA or DNA, for use as a genetic diagnostic marker for the diagnosis of Brugada Syndrome in a human being.

**13.** A mutated PRG4 protein encoded by the oligonucleotide sequence according to claim 12, for use as a genetic diagnostic marker for the diagnosis of Brugada Syndrome in a human being.

**14.** Oligonucleotide sequence of *Epx* human gene (ENSG00000121053) **characterized by** the presence of a deleterious mutations G→C in position 58,193,733 of the gene sequence, said oligonucleotide sequence being RNA or DNA, for use as a genetic diagnostic marker for the diagnosis of Brugada Syndrome in a human being.

**15.** A mutated EPX protein encoded by the oligonucleotide sequence according to claim 14, for use as a genetic diagnostic marker for the diagnosis of Brugada Syndrome in a human being.

**16.** Oligonucleotide sequence of *Pon1* human gene (ENSG00000005421) **characterized by** the presence of a deleterious mutations A→T in position 95,316,772 of the gene sequence, said oligonucleotide sequence being RNA or DNA, for use as a genetic diagnostic marker for the diagnosis of Brugada Syndrome in a human being.

**17.** A mutated PON1 protein encoded by oligonucleotide sequence according to claim 16, for use as a genetic diagnostic marker for the diagnosis of Brugada Syndrome in a human being.

**18.** Use of one or more of the oligonucleotide sequence or mutated protein according to anyone of the claims 12-17, for the diagnosis of Brugada Syndrome in a human being by detection in a biological sample, wherein said biological sample is selected from the group consisting of plasma, PBMCs, whole blood, serum and peripheral blood, or a combination thereof.

**20.** An in vitro method for detecting the presence of one or more of the oligonucleotide sequences according to anyone of claims 12, 14, 16 by genetic analysis through PCR or DNA sequencing.

**21.** A kit comprising primer or probes complementary to one or more of the oligonucleotide sequence according to anyone of the claims 12, 14, 16, for the diagnosis of Brugada Syndrome in a human being.

## Amended claims

### Amended claims in accordance with Rule 137(2) EPC.

1. A set of biomarkers comprising at least:
i) the subset of the following 7 proteins:
| **Protein** | **UniProtKB ID** |
|---|---|
| Fibronectin 1 (FN1) | P02751 |
| Inter-alpha-trypsin inhibitor heavy chain 3 (ITIH3) | Q06033 |
| Proteoglycan 4 (PRG4) | Q92954 |
| Adaptor related protein complex 2 subunit sigma 1 (AP2S1) | M0QYZ2 |
| Component of oligomeric Golgi complex 5 (COG5) | Q9UP83 |
| Eosinophil peroxidase (EPX) | P11678 |
| Ribosomal oxygenase 1 (RIOX1) | Q9H6W3 |
and
ii) the subset of the following 3 metabolites:
- fumaric acid;
- O-palmitoleoylcarnitine;
- L-Glutamic acid; for the diagnosis of Brugada Syndrome in a human being.

2. A set of biomarkers according to claim 1, further comprising one or more of the following additional biomarkers:
i) a protein selected from the group consisting of:
| **Protein** | **UniProtKB ID** |
|---|---|
| Paraoxonase 1 (PON1) | P27169 |
| Aldehyde dehydrogenase 3 family member B1 (ALDH3B 1) | P43353 |
| Solute carrier family 25 member (SLC25A5) | P05141 |
| S 100 calcium binding protein A11 (S100A11) | P31949 |
| Phospholipase C beta 2 (PLCB2) | Q00722 |
and/or the metabolite 2-hydroxydecanoate;
or a combination thereof, for the diagnosis of Brugada Syndrome in a human being.

3. A set of biomarkers according to claim 2, comprising:
i) the subset of the following 12 proteins:
| **Protein** | **UniProtKB ID** |
|---|---|
| Fibronectin 1 (FN1) | P02751 |
| Inter-alpha-trypsin inhibitor heavy chain 3 (ITIH3) | Q06033 |
| Proteoglycan 4 (PRG4) | Q92954 |
| Adaptor related protein complex 2 subunit sigma 1 (AP2S1) | M0QYZ2 |
| Component of oligomeric Golgi complex 5 (COG5) | Q9UP83 |
| Eosinophil peroxidase (EPX) | P11678 |
| Ribosomal oxygenase 1 (RIOX1) | Q9H6W3 |
and
ii) the subset of the following 4 metabolites:
- fumaric acid;
- O-palmitoleoylcarnitine;
- L-Glutamic acid;
- 2-hydroxydecanoate;
or a combination thereof, for the diagnosis of Brugada Syndrome in a human being.

4. An in vitro method for detecting the presence or measuring concentration of the occurrence of one of the set of biomarkers according to anyone of claim 1-3 in a biological sample of a human being.

5. An in vitro method for detecting the presence of one of the set of biomarkers according to claim 4, which comprises the following steps:
a) detecting the presence of at least 7 up to 12 proteins of subset i) by an assay selected from the group comprising Western Blot, Dot blot, ELISA, flow cytometry, enzymatic activity assay, targeted MS, multiplex protein analysis, ProQuantum high-sensitivity immunoassay, or a combination thereof;
b) detection of at least 3 up to 4 of the metabolites of subset ii) by an assay such as one selected from the group comprising MS, GC-MS, fluorimetric or colorimetric assays, NMR, spectroscopy with nanoprobes, Enzyme-Linked oligonucleotide assays, or a combination thereof;
wherein the positive detection of all the biomarkers compared to a normal control allows the identification of patients affected by Brugada Syndrome.

6. An in vitro method for measuring the concentration of one of the set of biomarkers according to claim 4, comprising the following steps:
a) quantitative determination of at least 7 up to 12 proteins of subset i) by a quantitative technique such as Western Blot, ELISA, enzymatic activity assay, targeted MS, multiplex protein analysis, ProQuantum high-sensitivity immunoassays, or a combination thereof;
b) quantitative determination of at least 3 up to 4 of the metabolites of subset ii) by a technique such as HPLC, MS, NMR analysis, ELONA, or a combination thereof;
wherein an increase or decrease of the concentration of the biomarkers compared to a normal control indicates a diagnosis of Brugada Syndrome.

7. An in vitro method according to anyone of the claims 4-6, wherein the biological sample is selected from the group consisting of plasma, PBMCs, whole blood, serum and peripheral blood, or a combination thereof.

8. An in vitro method according to anyone of the claims 4-7, wherein the human being is asymptomatic.

9. An in vitro method according to anyone of the claims 4-7, wherein the human being is at high risk for Brugada Syndrome due to family history, previous events of heart atrial and/or ventricular fibrillation, diabetes or obesity.

10. An in vitro method according to anyone of the claims 4-9, wherein the human being is about 40 years old or younger.

11. A kit comprising antibodies specific for each of the proteins of subset i) according to anyone of claim 1-3, wherein said antibodies are labelled or attached to a solid support.

12. Oligonucleotide sequence of *Prg4* gene (ENSG00000116690) **characterized by** the presence of a deleterious mutations C→T in position 186,304,862 of the nucleotide sequence, said oligonucleotide sequence being RNA or DNA, for use as a genetic diagnostic marker for the diagnosis of Brugada Syndrome in a human being.

13. A mutated PRG4 protein encoded by the oligonucleotide sequence according to claim 12, for use as a genetic diagnostic marker for the diagnosis of Brugada Syndrome in a human being.

14. Oligonucleotide sequence of *Epx* human gene (ENSG00000121053) **characterized by** the presence of a deleterious mutations G→C in position 58,193,733 of the gene sequence, said oligonucleotide sequence being RNA or DNA, for use as a genetic diagnostic marker for the diagnosis of Brugada Syndrome in a human being.

15. A mutated EPX protein encoded by the oligonucleotide sequence according to claim 14, for use as a genetic diagnostic marker for the diagnosis of Brugada Syndrome in a human being.

16. Oligonucleotide sequence of *Pon1* human gene (ENSG00000005421) **characterized by** the presence of a deleterious mutations A→T in position 95,316,772 of the gene sequence, said oligonucleotide sequence being RNA or DNA, for use as a genetic diagnostic marker for the diagnosis of Brugada Syndrome in a human being.

17. A mutated PON1 protein encoded by oligonucleotide sequence according to claim 16, for use as a genetic diagnostic marker for the diagnosis of Brugada Syndrome in a human being.

18. Use of one or more of the oligonucleotide sequence or mutated protein according to anyone of the claims 12-17, for the diagnosis of Brugada Syndrome in a human being by detection in a biological sample, wherein said biological sample is selected from the group consisting of plasma, PBMCs, whole blood, serum and peripheral blood, or a combination thereof.

19. An in vitro method for detecting the presence of one or more of the oligonucleotide sequences according to anyone of claims 12, 14, 16 by genetic analysis through PCR or DNA sequencing.

20. A kit comprising primer or probes complementary to one or more of the oligonucleotide sequence according to anyone of the claims 12, 14, 16, for the diagnosis of Brugada Syndrome in a human being.
